(19) Europäisches Patentamt
European Patent Office
Office européen des brevets

(11) Veröffentlichungsnummer: **0 433 749 A2**

(12) **EUROPÄISCHE PATENTANMELDUNG**

(21) Anmeldenummer: **90123171.2**

(22) Anmeldetag: **04.12.90**

(51) Int. Cl.[5]: **C07D 231/22**, A01N 43/56, C07D 401/04, C07D 405/04, C07D 409/04, C07D 409/06, A01N 43/40, A01N 43/08, A01N 43/10

(30) Priorität: **14.12.89 DE 3941240**

(43) Veröffentlichungstag der Anmeldung: **26.06.91 Patentblatt 91/26**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Wroblowsky, Heinz-Jürgen, Dr.**
**Gladbacher Strasse 34**
**W-4018 Langenfeld(DE)**
Erfinder: **Babczinski, Peter, Dr.**
**In der Lohrenbeck 11**
**W-5600 Wuppertal 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Pyrazolin-5-on-Derivate.**

(57) Neue Pyrazolin-5-on-Derivate der allgemeinen Formel (I)

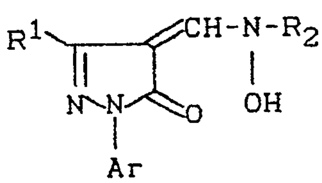

(I)

Verfahren zu deren Herstellung und ihre herbizide Verwendung.

EP 0 433 749 A2

# PYRAZOLIN-5-ON-DERIVATE

Die Erfindung betrifft neue Pyrazolin-5-on-Derivate, ein Verfahren zu ihrer Herstellung und ihre Verwendung als Herbizide.

Es ist bereits bekannt, daß bestimmte Pyrazolin-5-on-Derivate herbizide Eigenschaften besitzen (vgl. DE-OS 25 13 750).

Weiterhin ist die herbizide und fungizide Wirksamkeit bestimmter Pyrazolin-5-on-Derivate bekannt (vgl. EP-A 274 642). Aus EP-A 274 642 bekannte Verbindungen wurden mittels Disclaimer ausgeschlossen.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue Pyrazolin-5-on-Derivate der allgemeinen Formel (I)

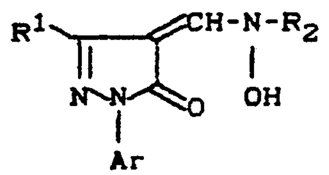

(I)

in welcher

$R^1$ für Alkyl, Cycloalkyl, Halogenalkyl, für jeweils gegebenenfalls substituiertes Alkenyl oder Alkinyl, wobei als Substituent gegebenenfalls substituiertes Phenyl ausgewählt ist, für Halogenalkenyl, Alkoxy, Alkoxyalkyl, Alkylthioalkyl, Alkylsulfonylalkyl, Alkylsulfinylalkyl, Alkoxycarbonylalkyl, jeweils gegebenenfalls substituiertes Aryl, Aralkyl, Aryloxyalkyl oder Arylthioalkyl, für einen gegebenenfalls substituierten Heterocyclus, für Heterocyclylalkyl oder für die Gruppierungen -NH-CO-$R^3$ oder -CO-O-$R^4$ steht, worin

$R^3$ und $R^4$ jeweils unabhängig voneinander für Alkyl oder Aryl stehen,

$R^2$ für Wasserstoff, Alkyl, Cycloalkyl, Halogenalkyl, Alkenyl, Alkinyl, Halogenalkenyl, Alkoxyalkyl, Alkylthioalkyl, Alkylsulfonylalkyl, Alkylsulfinylalkyl oder Alkoxycarbonylalkyl steht,

Ar für gegebenenfalls substituiertes Aryl oder für einen gegebenenfalls substituierten und/oder gegebenenfalls anellierten Heterocyclus steht,

ausgenommen die Verbindungen 1-(3-Chlorphenyl)-3-methyl-thiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on,

gefunden.

Die Verbindungen der Formel (I) können als geometrische Isomere (E/Z-Isomere) oder Isomerengemische unterschiedlicher Zusammensetzung vorliegen. Die Erfindung betrifft sowohl die reinen Isomeren als auch die Isomerengemische.

Außerdem können die erfindungsgemäßen Verbindungen der Formel (I), für den Fall, daß $R^2$ für Wasserstoff steht, im tautomeren Gleichgewicht vorliegen:

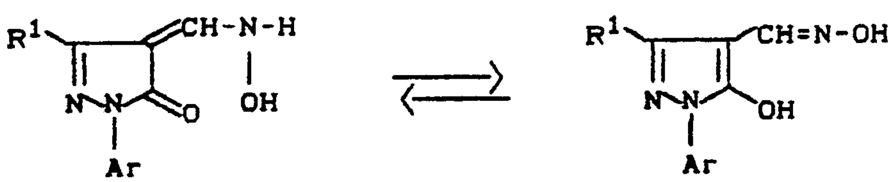

Im nachfolgenden wird der Einfachheit halber stets von Verbindungen der Formel (I) gesprochen, obwohl sowohl die reinen Verbindungen als auch ihre Gemische mit un terschiedlichen Anteilen der tautomeren Verbindungen gemeint sind.

Weiterhin wurde gefunden, daß man die neuen Pyrazolin-5-on-Derivate der allgemeinen Formel (I),

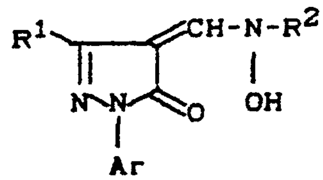

(I)

in welcher

$R^1$ für Alkyl, Cycloalkyl, Halogenalkyl, für jeweils gegebenenfalls substituiertes Alkenyl oder Alkinyl, wobei als Substituent gegebenenfalls substituiertes Phenyl ausgewählt ist, für Halogenalkenyl, Alkoxy, Alkoxyalkyl, Alkylthioalkyl, Alkylsulfonylalkyl, Alkylsulfinylalkyl, Alkoxycarbonylalkyl, für jeweils gegebenenfalls substituiertes Aryl, Aralkyl, Aryloxyalkyl oder Arylthioalkyl, für gegebenenfalls substituiertes Heterocyclyl, gegebenenfalls substituiertes Heterocyclylalkyl oder für die Gruppierungen -NH-CO-$R^3$ oder -CO-O-$R^4$ steht, worin

$R^3$ und $R^4$ jeweils unabhängig voneinander für Alkyl oder Aryl stehen,

$R^2$ für Wasserstoff, Alkyl, Cycloalkyl, Halogenalkyl Alkenyl, Alkinyl, Halogenalkenyl, Alkoxyalkyl, Alkylthioalkyl, Alkylsulfonylalkyl, Alkylsulfinylalkyl, Alkoxycarbonylalkyl steht und

Ar für gegebenenfalls substituiertes Aryl oder für gegebenenfalls substituiertes und/oder gegebenenfalls anelliertes Heterocyclyl steht,

ausgenommen die Verbindungen 1-(3-Chlorphenyl)-3-methylthiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on,

erhält, wenn man Dimethylaminomethylidenpyrazolin-5-on-Derivate der Formel (II)

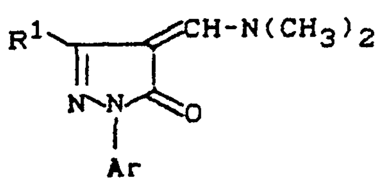

(II)

in welcher

$R^1$ und Ar die oben angegebenen Bedeutungen haben,

mit Hydroxylamin-hydrochlorid-Derivaten der Formel (III)

$$R^2\text{-NHOH} \quad x \quad HX \qquad (III)$$

in welcher

$R^2$ die oben angegebene Bedeutung hat und

HX für das Äquivalent einer Mineralsäure, wie z.B. Chlorwasserstoffsäure oder einer Carbonsäure, wie z.B. Oxalsäure steht,

gegebenenfalls in Gegenwart eines Verdünnungsmittels umsetzt.

Schließlich wurde gefunden, daß die neuen Pyrazolin-5-on-Derivate der allgemeinen Formel (I) ausgezeichnete herbizide Eigenschaften besitzen.

Bevorzugt sind Verbindungen der Formel (I), bei welchen

$R^1$ für geradkettiges oder verzweigtes Alkyl mit 1 bis 8 Kohlenstoffatomen, Cycloalkyl mit 3 bis 7 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes und jeweils gegebenenfalls einfach bis mehrfach substituiertes Alkenyl oder Alkinyl mit jeweils 2 bis 6 Kohlenstoffatomen steht, wobei als Substituenten unsubstituiertes oder einfach bis mehrfach, gleich oder verschieden substituiertes Phenyl infrage kommen und wobei als Phenylsubstituenten die unter Ar aufgeführten Arylsubstituenten genannt seien; $R^1$ weiterhin für geradkettiges oder verzweigtes Halogenalkenyl mit 2 bis 4 Kohlenstoffatomen und 1 bis 7 gleichen oder verschiedenen Halogenatomen,

geradkettiges oder verzweigtes Alkoxy mit 1 bis 4 Kohlenstoffatomen, geradkettiges oder verzweigtes Alkoxyalkyl mit jeweils 1 bis 8 Kohlenstoffatomen im Alkoxy- und Alkylteil, jeweils geradkettiges oder verzweigtes Alkylthioalkyl, Alkylsulfonylalkyl oder Alkylsulfinylalkyl mit jeweils 1 bis 4 Kohlenstoffatomen in den einzelnen Alkylteilen, geradkettiges oder verzweigtes Alkoxycarbonylalkyl mit jeweils 1 bis 4 Kohlenstoffatomen im Alkoxy- und Alkylteil, für jeweils gegebenenfalls einfach bis mehrfach, gleich oder verschieden substituiertes Phenyl oder Naphthyl, für jeweils gegebenenfalls einfach bis mehrfach, gleich oder verschieden im Arylteil substituiertes Phenylalkyl oder Naphthylalkyl mit jeweils 1 bis 8 Kohlenstoffatomen im geradkettigen oder verzweigten Alkylteil, für jeweils gegebenenfalls 1- bis 2-fach im Phenylteil substituiertes Phenoxyalkyl oder Phenylthioalkyl mit jeweils 1 bis 4 Kohlenstoffatomen im geradkettigen oder verzweigten Alkylteil steht, wobei als Phenyl- und Naphthylsubstituenten jeweils die unter Ar aufgeführten Arylsubstituenten genannt seien; $R^1$ weiterhin für jeweils gegebenenfalls einfach bis mehrfach, gleich oder verschieden im Heterocyclylteil substituiertes Furyl, Thienyl, Pyrrolyl, Pyridinyl, Furylalkyl, Thienylalkyl oder Pyrrolylalkyl mit jeweils gegebenenfalls 1 bis 8 Kohlenstoffatomen im geradkettigen oder verzweigten Alkylteil steht, wobei als Heterocyclylsubstituenten die unter Ar aufgeführten Arylsubstituenten infrage kommen; $R^1$ weiterhin für die Gruppierungen -NHCOR$^3$ oder -COOR$^4$ steht, worin

$R^3$ und $R^4$ jeweils unabhängig voneinander für geradkettiges oder verzweigtes Alkyl mit 1 bis 4 Kohlenstoffatomen oder Phenyl stehen,

$R^2$ für Wasserstoff, geradkettiges oder verzweigtes Alkyl mit 1 bis 8 Kohlenstoffatomen, Cycloalkyl mit 3 bis 7 Kohlenstoffatomen, geradkettiges oder verzweigtes Halogenalkyl mit 1 bis 8 Kohlenstoffatomen und 1 bis 17 gleichen oder verschiedenen Halogenatomen, für jeweils geradkettiges oder verzweigtes Alkenyl oder Alkinyl mit jeweils 2 bis 6 Kohlenstoffatomen, geradkettiges oder verzweigtes Halogenalkenyl mit 2 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen im Alkoxy- und Alkylteil, für jeweils geradkettiges oder verzweigtes Alkylthioalkyl, Alkylsulfonylalkyl oder Alkylsulfinylalkyl mit jeweils 1 bis 4 Kohlenstoffatomen in den einzelnen Alkyteilen, für geradkettiges oder verzweigtes Alkoxycarbonylalkyl mit jeweils 1 bis 4 Kohlenstoffatomen im Alkoxy-und Alkylteil, steht und

Ar für jeweils gegebenenfalls einfach bis mehrfach, gleich oder verschieden substituiertes Phenyl oder Naphthyl steht, wobei als Substituenten infrage kommen: Halogen, Nitro, Cyano, Carboxy, jeweils geradkettiges oder verzweigtes Alkyl, Alkoxy oder Alkylthio mit jeweils 1 bis 6 Kohlenstoffatomen, geradkettiges oder verzweigtes Cyanalkyl mit 1 bis 6 Kohlenstoffatomen, geradkettiges oder verzweigtes Alkoxycarbonyl mit 1 bis 6 Kohlenstoffatomen im Alkoxyteil, geradkettiges oder verzweigtes Alkinyloxy mit 2 bis 6 Kohlenstoffatomen, jeweils geradkettiges oder verzweigtes Halogenalkyl, Halogenalkoxy oder Halogenalkylthio mit jeweils 1 bis 6 Kohlenstoffatomen und jeweils 1 bis 13 gleichen oder verschiedenen Halogenatomen, geradkettiges oder verzweigtes Alkylsulfonyl mit 1 bis 6 Kohlenstoffatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in den jeweiligen geradkettigen oder verzweigten Alkylteilen, jeweils gegebenenfalls einfach bis mehrfach, gleich oder verschieden substituiertes Phenyl, Naphthyl, Phenoxy oder Naphthoxy, jeweils gegebenenfalls einfach bis mehrfach, gleich oder verschieden substituiertes Phenylalkyl oder Naphthylalkyl mit jeweils 1 bis 6 Kohlenstoffatomen im geradkettigen oder verzweigten Alkylteil; Ar weiterhin für jeweils gegebenenfalls einfach bis mehrfach, gleich oder verschieden substituiertes und jeweils gegebenenfalls durch einen Benzolring anelliertes Furyl, Thienyl, Pyrrolyl oder Pyridyl, wobei als Substituenten jeweils die oben genannten Arylsubstituenten infrage kommen,

ausgenommen die oben genannten Verbindungen.

Besonders bevorzugt sind Verbindungen der Formel (I), bei welchen

$R^1$ für geradkettiges oder verzweigtes Alkyl mit 1 bis 6 Kohlenstoffatomen, Cycloalkyl mit 3 bis 6 Kohlenstoffatomen, Halogenalkyl mit 1 oder 2 Kohlenstoffatomen und 1 bis 5 Fluor- und/oder Chloratomen, je weils geradkettiges oder verzweigtes, jeweils gegebenenfalls 1- oder 2-fach substituiertes Alkenyl oder Alkinyl mit jeweils 2 bis 4 Kohlenstoffatomen steht, wobei als Substituenten unsubstituiertes oder 1- oder 2-fach gleich oder verschieden substituiertes Phenyl infrage kommen und wobei als Phenylsubstituenten die unter Ar aufgeführten Arylsubstituenten genannt

seien; $R^1$ weiterhin für Halogenalkenyl mit 2 oder 3 Kohlenstoffatomen und 1 bis 5 Fluor- und/oder Chloratomen, Alkoxy mit 1 oder 2 Kohlenstoffatomen, geradkettiges oder verzweigtes Alkoxyalkyl mit jeweils 1 bis 6 Kohlenstoffatomen im Alkoxy- und Alkylteil, für Alkylthioalkyl, Alkylsulfonylalkyl oder Alkylsulfinylalkyl mit jeweils 1 oder 2 Kohlenstoffatomen in den einzelnen Alkylteilen, Alkoxycarbonylalkyl mit jeweils 1 oder 2 Kohlenstoffatomen im Alkoxy- und Alkylteil, für jeweils gegebenenfalls 1- bis 5-fach, gleich oder verschieden substituiertes Phenyl oder Naphthyl, jeweils gegebenenfalls 1- bis 5-fach, gleich oder verschieden im Arylteil substituiertes Phenylalkyl oder Naphthylalkyl mit jeweils 1 bis 6 Kohlenstoffatomen im geradkettigen oder verzweigten Alkylteil steht, wobei als Phenyl- und Naphthylsubstituenten jeweils die unter Ar aufgeführten Arylsubstituenten genannt seien; $R^1$ weiterhin für Phenoxyalkyl oder Phenylthioalkyl mit jeweils 1 oder 2 Kohlenstoffatomen im Alkylteil, für jeweils 1- bis 3-fach gleich oder verschieden substituiertes Furyl, Thienyl, Pyrrolyl oder Pyridinyl, für jeweils 1- bis 3-fach gleich oder verschieden im Heterocyclylteil substituiertes Furylalkyl, Thienylalkyl oder Pyrro lylalkyl mit jeweils 1 bis 6 Kohlenstoffatomen im geradkettigen oder verzweigten Alkylteil steht, wobei als Heterocyclylsubstituenten jeweils die unter Ar aufgeführten Arylsubstituenten infrage kommen; weiterhin $R^1$ für die Gruppierungen $-NHCOR^3$ oder $-COOR^4$ steht, worin

$R^3$ und $R^4$ jeweils unabhängig voneinander für Methyl, Ethyl oder Phenyl steht,

$R^2$ für Wasserstoff, geradkettiges oder verzweigtes Alkyl mit 1 bis 6 Kohlenstoffatomen, Cycloalkyl mit 3 bis 6 Kohlenstoffatomen, geradkettiges oder verzweigtes Halogenalkyl mit 1 bis 6 Kohlenstoffatomen und 1 bis 13 Fluor- und/oder Chloratomen, jeweils geradkettiges oder verzweigtes Alkenyl oder Alkinyl mit 2 bis 4 Kohlenstoffatomen, Halogenalkenyl mit 2 oder 3 Kohlenstoffatomen und 1 bis 5 Fluor- und/oder Chloratomen, Alkoxyalkyl mit jeweils 1 oder 2 Kohlenstoffatomen im Alkoxy- und Alkylteil, für Alkylthioalkyl, Alkylsulfonylalkyl oder Alkylsulfinylalkyl mit jeweils 1 oder 2 Kohlenstoffatomen in den einzelnen Alkylteilen, Alkoxycarbonylalkyl mit jeweils 1 oder 2 Kohlenstoffatomen im Alkoxy- und Alkylteil steht und

Ar für jeweils gegebenenfalls 1- bis 5-fach, vorzugsweise 1- bis 3-fach, insbesondere 1-fach, gleich oder verschieden substituiertes Phenyl oder Napththyl steht, wobei als Substituenten infrage kommen: Fluor, Chlor, Brom, Nitro, Cyano, Carboxy, jeweils geradkettiges oder verzweigtes Alkyl, Alkoxy oder Alkylthio mit jeweils 1 bis 4 Kohlenstoffatomen, geradkettiges oder verzweigtes Cyanalkyl mit 1 bis 4 Kohlenstoffatomen, geradkettiges oder verzweigtes Alkoxycarbonyl mit 1 bis 4 Kohlenstoffatomen im Alkoxyteil, geradkettiges oder verzweigtes Alkinyloxy mit 2 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, geradkettiges oder verzweigtes Alkylsulfonyl mit 1 bis 4 Kohlenstoffatomen, geradkettiges oder verzweigtes Halogenalkylsulfonyl mit 1 bis 4 Kohlenstoffatomen und 1 bis 9 gleichen oder verschiedenen Halogenatomen, Dialkylamino mit 1 bis 4 Kohlenstoffatomen in den jeweiligen geradkettigen oder verzweigten Alkylteilen, jeweils gegebenenfalls 1- oder 2-fach, gleich oder verschieden substituiertes Phenyl, Naphthyl, Phenoxy oder Naphthoxy, jeweils gegebenenfalls 1- oder 2-fach, gleich oder verschieden substituiertes Phenylalkyl oder Naphthylalkyl mit jeweils 1 bis 4 Kohlenstoffatomen im geradkettigen oder verzweigten Alkylteil; Ar weiterhin für jeweils gegebenenfalls 1- oder 2-fach gleich oder verschieden substituiertes Furyl, Thienyl, Pyrrolyl oder Pyridyl, wobei als Substituenten jeweils die oben genannten Arylsubstituenten infrage kommen,

ausgenommen die oben genannten Verbindungen.

Ganz besonders bevorzugt sind Verbindungen der Formel (I), bei welchen

$R^1$ für Methyl, Ethyl, n-Propyl, i-Propyl, n-Butyl, i-Butyl, s-Butyl, t-Butyl, 2-Phenylvinyl, 2-(3-Trifluormethylphenyl)-vinyl, Methoxymethyl, Methoxyethyl, Ethoxymethyl, Ethoxyethyl, für gegebenenfalls 1- bis 3-fach, gleich oder verschieden substituiertes Phenyl oder Naphthyl, jeweils gegebenenfalls 1- bis 3-fach im Phenylteil substituiertes Phenylmethyl oder Phenylethyl steht, wobei als Phenyl- und Naphthylsubstituenten jeweils die unter Ar aufgeführten Arylsubstituenten, insbesondere Fluor, Chlor, Brom, Nitro, Cyano, Methyl, Ethyl, Methoxy, Ethoxy, Trifluormethyl, Trifluormethoxy, Trifluormethylthio, genannt seien; $R^1$ weiterhin für Furyl, Thienyl, Pyridyl, Furylmethyl, Thienylmethyl oder Pyridylmethyl steht,

$R^2$ für Wasserstoff, Methyl, Ethyl, n-Propyl, i-Propyl, n-Butyl, i-Butyl, s-Butyl, t-Butyl, Trifluormethyl, Trichlormethyl, 2,2,2-Trifluorethyl, 2,2,2-Trichlorethyl, Vinyl, Allyl, 1-Propenyl, Ethinyl, 1-Propinyl, 2-Propinyl, 2-Fluorvinyl, Methoxymethyl oder Methylthiomethyl steht und

Ar für jeweils gegebenenfalls 1- bis 3-fach, insbesondere 1-fach, gleich oder verschieden substituiertes Phenyl steht, wobei als Substituenten infrage kommen: Fluor, Chlor, Brom, Nitro, Cyano,

Carboxy, Methyl, Ethyl, Methoxy, Ethoxy, Methylthio, Ethylthio, Cyanmethyl, 1-Cyanethyl, 2-Cyanethyl, Methoxycarbonyl, Ethinyloxy, Fluormethyl, Difluormethyl, Trifluormethyl, Chlormethyl, Dichlormethyl, Trichlormethyl, 2-Fluorethyl, 2,2-Difluorethyl, 2,2,2-Trifluorethyl, 2-Chlorethyl, 2,2-Dichlorethyl, 2,2,2-Trichlorethyl, Fluormethoxy, Difluormethoxy, Trifluormethoxy, Chlormethoxy, Dichlormethoxy, Trichlormethoxy, Fluormethylthio, Difluormethylthio, Trifluormethylthio, Chlormethylthio, Dichlormethylthio, Trichlormethylthio, Methylsulfonyl, Ethylsulfonyl, Fluormethylsulfonyl, Difluormethylsulfonyl, Trifluormethylsulfonyl, Chlormethylsulfonyl, Dichlormethylsulfonyl, Trichlormethylsulfonyl, Dimethylamino, Diethylamino, Phenyl, Phenoxy, Phenylmethyl oder Phenylethyl steht.

$R^1$ steht insbesondere für Methyl, Ethyl, n- oder i-Propyl, Methoxymethyl, unsubstituiertes Phenyl oder jeweils einfach bis dreifach, insbesondere einfach, gleich oder verschieden durch Fluor, Chlor, Brom, Nitro, Methyl, Methoxy, Trifluormethyl, Trifluormethoxy oder Trifluormethylthio substituiertes Phenyl oder Phenylmethyl.

$R^2$ steht insbesondere für Methyl.

Ar steht insbesondere für unsubstituiertes Phenyl oder einfach oder zweifach durch Fluor, Chlor, Cyano, Nitro, Methyl, Methoxy, Trifluormethyl, Trifluormethoxy, Trifluormethylthio oder Methylsulfonyl substituiertes Phenyl.

Die Substitution des Phenylringes in Ar erfolgt insbesondere in der Para-Position. Vorzugsweise wird der Phenylring durch Fluor substituiert.

Im einzelnen seien zusätzlich zu den bei den Herstellungsbeispielen genannten Verbindungen die folgenden Pyrazolin-5-on-Derivate der Formel (I) beispielhaft genannt:

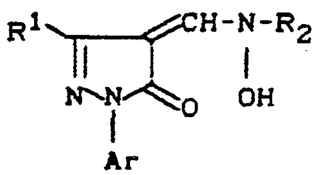

(I)

Tabelle 1

| $R^1$ | $R^2$ | Ar |
|---|---|---|
| $-C_3H_7-n$ | $CH_3$ | 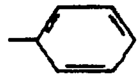 |
| $-C_3H_7-n$ | $CH_3$ | 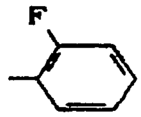 |
| $-C_3H_7-n$ | $CH_3$ | 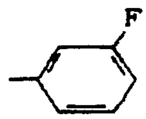 |
| $-C_3H_7-n$ | $CH_3$ | 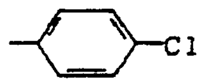 |
| $-C_3H_7-n$ | $CH_3$ | 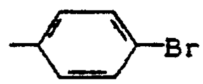 |
| $-C_3H_7-n$ | $CH_3$ | 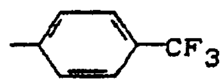 |
| $-C_3H_7-n$ | $CH_3$ | 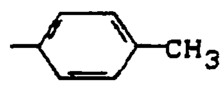 |
| $-C_3H_7-n$ | $CH_3$ | 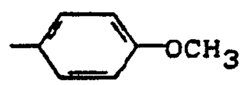 |
| $-C_3H_7-n$ | $CH_3$ | 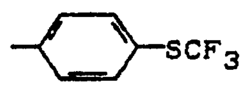 |
| $-C_3H_7-n$ | $CH_3$ | 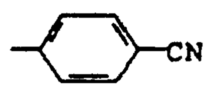 |

Tabelle 1 (Fortsetzung)

| $R^1$ | $R^2$ | Ar |
|---|---|---|
| $-C_3H_7$-n | $CH_3$ | 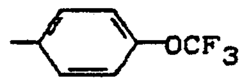 |
| $-C_3H_7$-n | $CH_3$ | 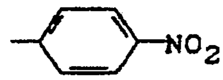 |
| $-C_3H_7$-n | $CH_3$ | 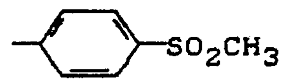 |
| $-C_3H_7$-n | $CH_3$ | 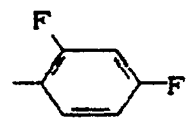 |
| $-C_3H_7$-n | $CH_3$ | 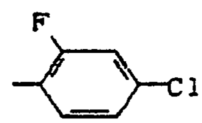 |
| $-CH_2OCH_3$ | $CH_3$ | 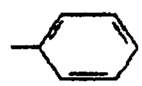 |
| $-CH_2OCH_3$ | $CH_3$ | 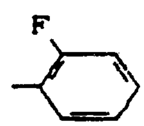 |
| $-CH_2OCH_3$ | $CH_3$ | 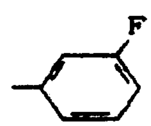 |
| $-CH_2OCH_3$ | $CH_3$ | 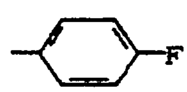 |
| $-CH_2OCH_3$ | $CH_3$ | 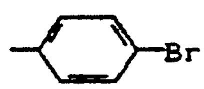 |

Tabelle 1 (Fortsetzung)

| $R^1$ | $R^2$ | Ar |
| --- | --- | --- |
| $-CH_2OCH_3$ | $CH_3$ | 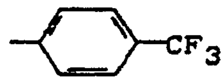 |
| $-CH_2OCH_3$ | $CH_3$ | 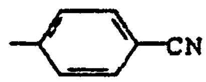 |
| $-CH_2OCH_3$ | $CH_3$ | 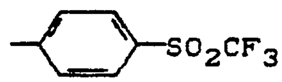 |
| $-CH_2OCH_3$ | $CH_3$ | 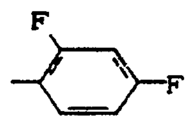 |
| $-C_4H_9$-n | $CH_3$ | 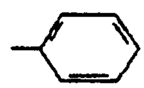 |
| $-C_4H_9$-n | $CH_3$ | 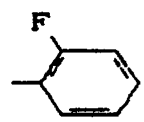 |
| $-C_4H_9$-n | $CH_3$ | 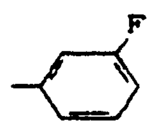 |
| $-C_4H_9$-n | $CH_3$ | 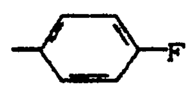 |
| $-C_4H_9$-n | $CH_3$ | 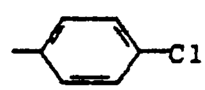 |
| $-C_4H_9$-n | $CH_3$ | 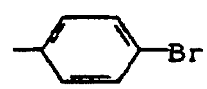 |

<u>Tabelle 1</u> (Fortsetzung)

| $R^1$ | $R^2$ | Ar |
|---|---|---|
| $-C_4H_9$-n | $CH_3$ | 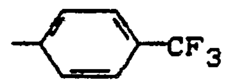 |
| $-C_4H_9$-n | $CH_3$ | 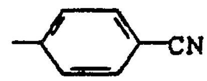 |
| $-C_4H_9$-n | $CH_3$ | 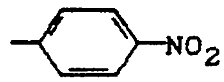 |
| $-C_4H_9$-n | $CH_3$ | 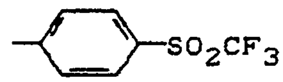 |
| $-C_4H_9$-n | $CH_3$ | 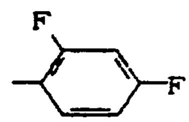 |
| $-CH_2-CH(CH_3)_2$ | $CH_3$ | 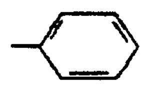 |
| $-CH_2-CH(CH_3)_2$ | $CH_3$ | 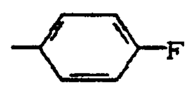 |
| $-CH_2-CH(CH_3)_2$ | $CH_3$ | 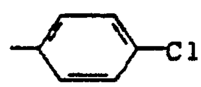 |
| $-CH_2-CH(CH_3)_2$ | $CH_3$ | 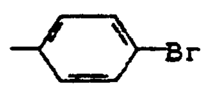 |
| $-CH_2-CH(CH_3)_2$ | $CH_3$ | 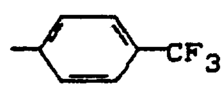 |

<u>Tabelle 1</u> (Fortsetzung)

| $R^1$ | $R^2$ | Ar |
|---|---|---|
| $-CH_2-CH(CH_3)_2$ | $CH_3$ | 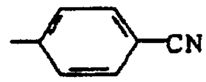 |
| $-CH_2-CH(CH_3)_2$ | $CH_3$ | 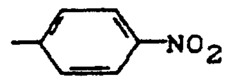 |
| $-CH_2-CH(CH_3)_2$ | $CH_3$ | 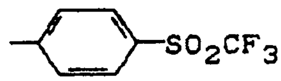 |
| $-CH_2-CH(CH_3)_2$ | $CH_3$ | 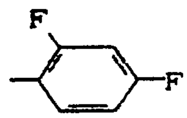 |
| $-CH_2-CH(CH_3)_2$ | $CH_3$ | 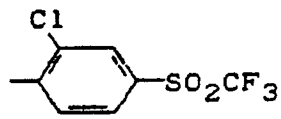 |
| 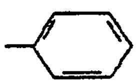 | $CH_3$ | 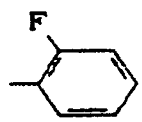 |
| 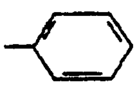 | $CH_3$ | 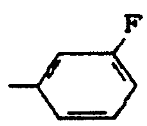 |
| 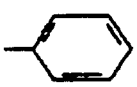 | $CH_3$ | 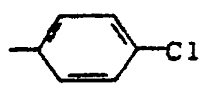 |
| 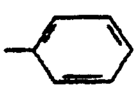 | $CH_3$ | 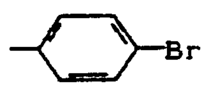 |
| 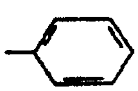 | $CH_3$ | 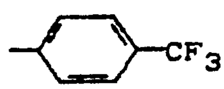 |

Tabelle 1 (Fortsetzung)

| $R^1$ | $R^2$ | Ar |
|---|---|---|
| 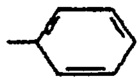 | $CH_3$ | 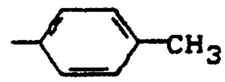 |
| 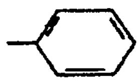 | $CH_3$ | 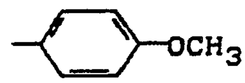 |
| 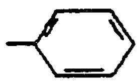 | $CH_3$ | 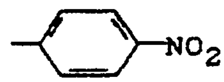 |
| 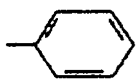 | $CH_3$ | 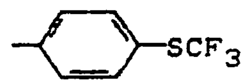 |
| 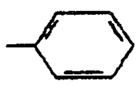 | $CH_3$ | 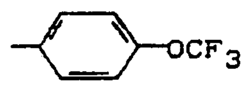 |
| 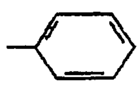 | $CH_3$ | 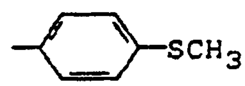 |
| 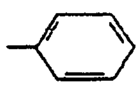 | $CH_3$ | 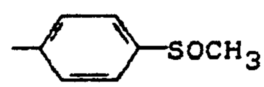 |
| 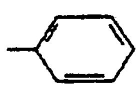 | $CH_3$ | 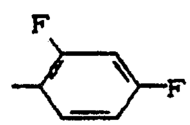 |
| 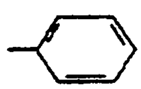 | $CH_3$ | 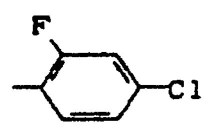 |
| 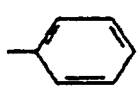 | $CH_3$ | 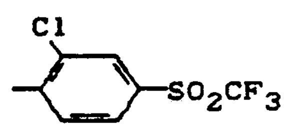 |

Tabelle 1 (Fortsetzung)

| $R^1$ | $R^2$ | Ar |
|---|---|---|
| 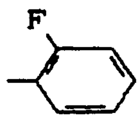 | $CH_3$ | 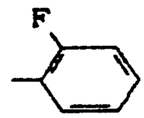 |
| 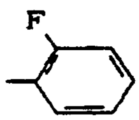 | $CH_3$ | 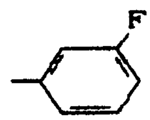 |
| 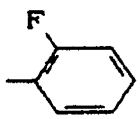 | $CH_3$ | 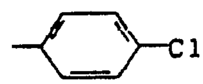 |
| 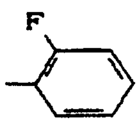 | $CH_3$ | 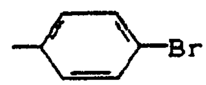 |
| 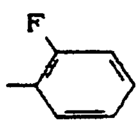 | $CH_3$ | 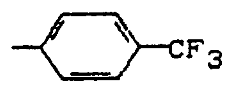 |
| 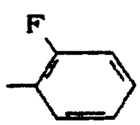 | $CH_3$ | 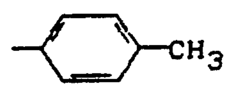 |
| 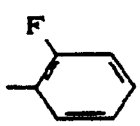 | $CH_3$ | 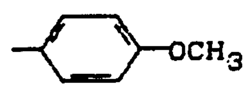 |
| 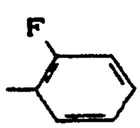 | $CH_3$ | 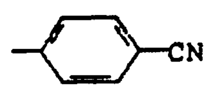 |

Tabelle 1 (Fortsetzung)

| $R^1$ | $R^2$ | Ar |
|---|---|---|
| 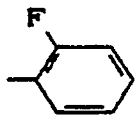 | $CH_3$ | 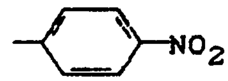 |
| 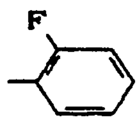 | $CH_3$ | 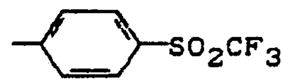 |
| 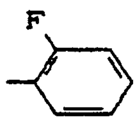 | $CH_3$ | 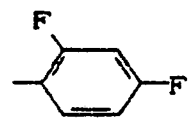 |
| 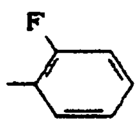 | $CH_3$ | 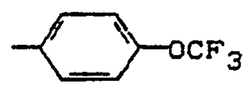 |
| 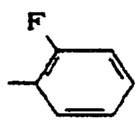 | $CH_3$ | 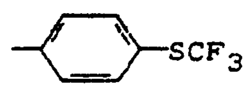 |
| 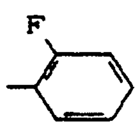 | $CH_3$ | 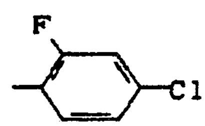 |
| 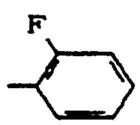 | $CH_3$ | 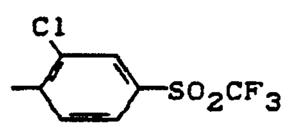 |
| 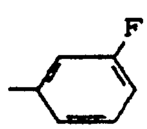 | $CH_3$ | 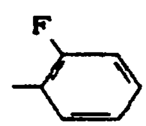 |

<u>Tabelle 1</u> (Fortsetzung)

| $R^1$ | $R^2$ | Ar |
| --- | --- | --- |
| 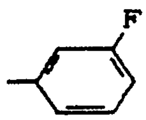 | $CH_3$ | 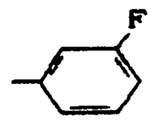 |
| 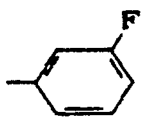 | $CH_3$ | 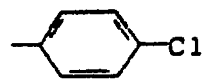 |
| 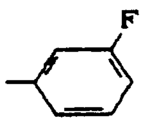 | $CH_3$ | 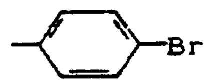 |
| 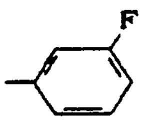 | $CH_3$ | 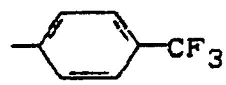 |
| 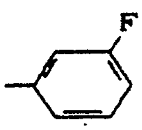 | $CH_3$ | 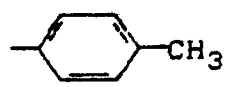 |
| 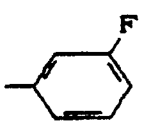 | $CH_3$ | 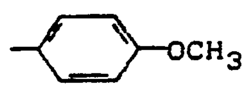 |
| 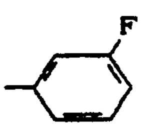 | $CH_3$ | 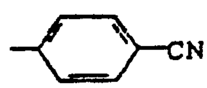 |
| 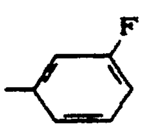 | $CH_3$ | 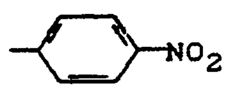 |

Tabelle 1 (Fortsetzung)

| $R^1$ | $R^2$ | Ar |
|---|---|---|
| 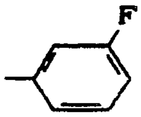 | $CH_3$ | 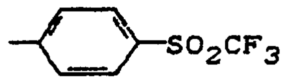 |
| 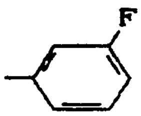 | $CH_3$ | 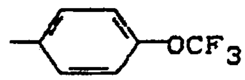 |
| 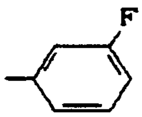 | $CH_3$ | 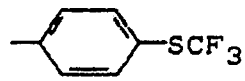 |
| 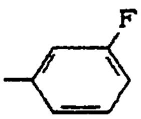 | $CH_3$ | 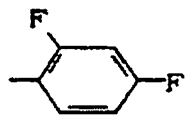 |
| 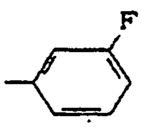 | $CH_3$ | 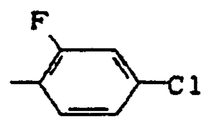 |
| 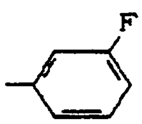 | $CH_3$ | 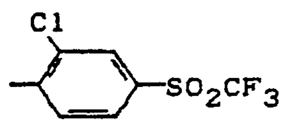 |
| 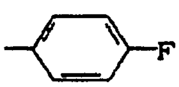 | $CH_3$ | 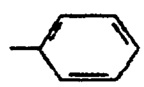 |
| 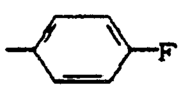 | $CH_3$ | 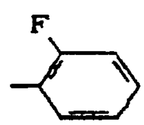 |

Tabelle 1 (Fortsetzung)

| $R^1$ | $R^2$ | Ar |
|---|---|---|
| 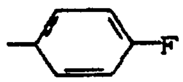 | $CH_3$ | 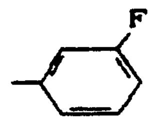 |
| 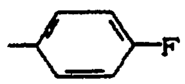 | $CH_3$ | 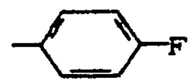 |
| 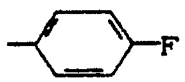 | $CH_3$ | 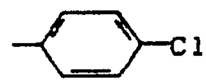 |
| 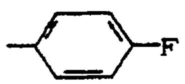 | $CH_3$ | 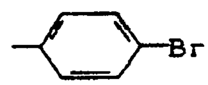 |
| 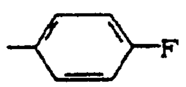 | $CH_3$ | 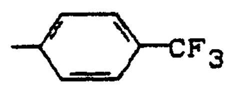 |
| 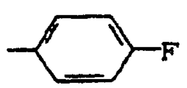 | $CH_3$ | 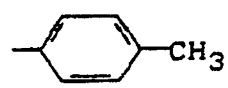 |
| 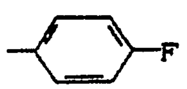 | $CH_3$ | 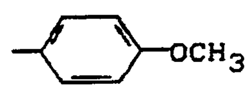 |
| 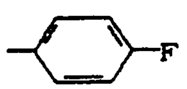 | $CH_3$ | 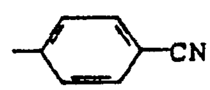 |
| 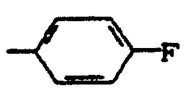 | $CH_3$ | 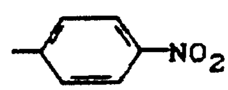 |
| 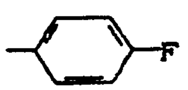 | $CH_3$ | 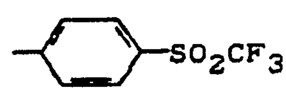 |

<u>Tabelle 1</u> (Fortsetzung)

| $R^1$ | $R^2$ | Ar |
|---|---|---|
| 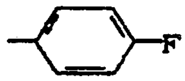 | $CH_3$ | 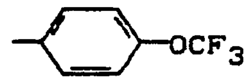 |
| 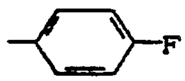 | $CH_3$ | 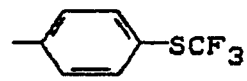 |
| 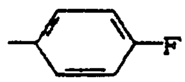 | $CH_3$ | 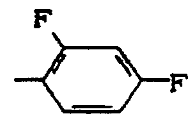 |
| 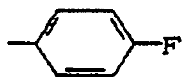 | $CH_3$ | 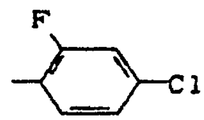 |
| 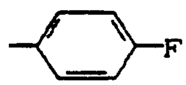 | $CH_3$ | 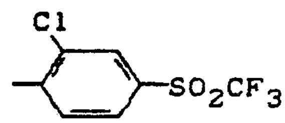 |
| 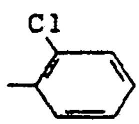 | $CH_3$ | 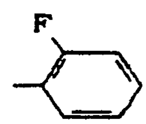 |
| 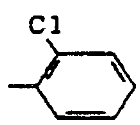 | $CH_3$ | 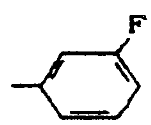 |
| 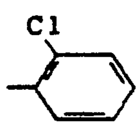 | $CH_3$ | 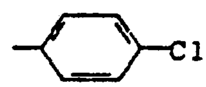 |
| 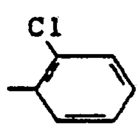 | $CH_3$ | 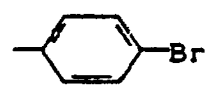 |

Tabelle 1 (Fortsetzung)

| $R^1$ | $R^2$ | Ar |
|---|---|---|
| 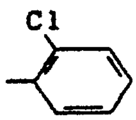 | $CH_3$ | 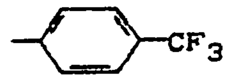 |
| 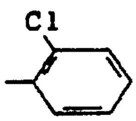 | $CH_3$ | 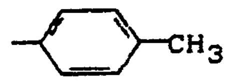 |
| 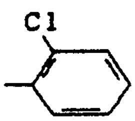 | $CH_3$ | 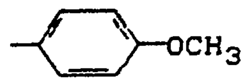 |
| 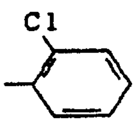 | $CH_3$ | 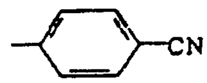 |
| 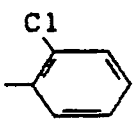 | $CH_3$ | 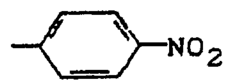 |
| 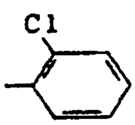 | $CH_3$ | 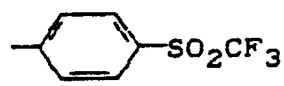 |
| 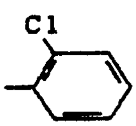 | $CH_3$ | 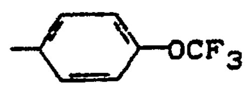 |
| 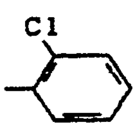 | $CH_3$ | 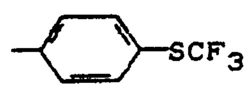 |

Tabelle 1 (Fortsetzung)

| $R^1$ | $R^2$ | Ar |
|---|---|---|
| 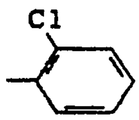 | $CH_3$ | 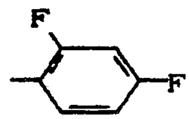 |
| 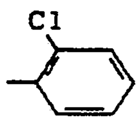 | $CH_3$ | 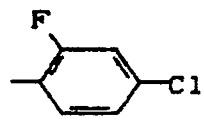 |
| 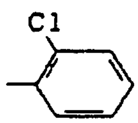 | $CH_3$ | 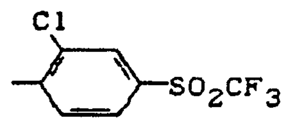 |
| 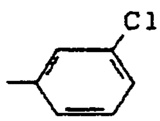 | $CH_3$ | 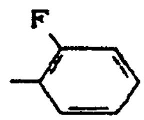 |
| 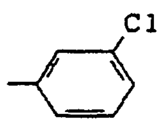 | $CH_3$ | 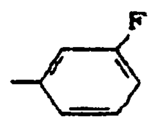 |
| 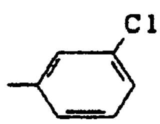 | $CH_3$ | 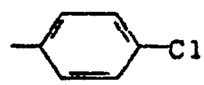 |
| 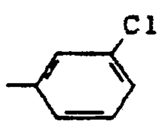 | $CH_3$ | 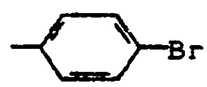 |
| 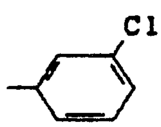 | $CH_3$ | 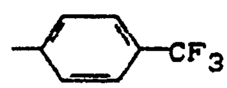 |

Tabelle 1 (Fortsetzung)

| $R^1$ | $R^2$ | Ar |
|---|---|---|
| 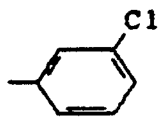 | $CH_3$ | 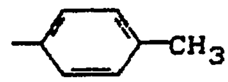 |
| 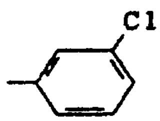 | $CH_3$ | 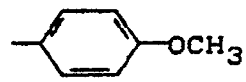 |
| 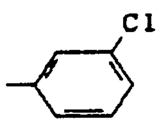 | $CH_3$ | 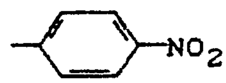 |
| 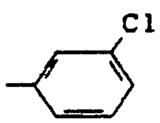 | $CH_3$ | 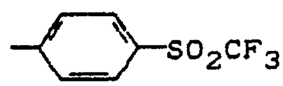 |
| 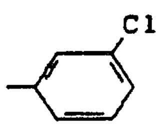 | $CH_3$ | 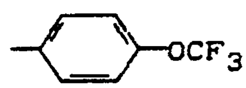 |
| 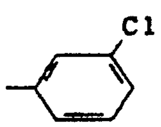 | $CH_3$ | 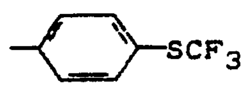 |
| 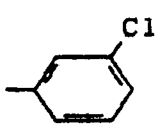 | $CH_3$ | 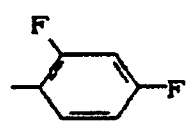 |
| 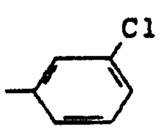 | $CH_3$ | 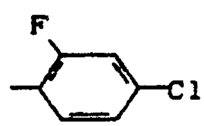 |

<u>Tabelle 1</u> (Fortsetzung)

| $R^1$ | $R^2$ | Ar |
|---|---|---|
| 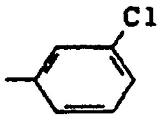 | $CH_3$ | 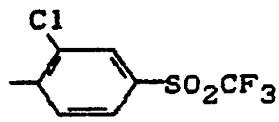 |
| 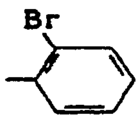 | $CH_3$ | 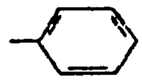 |
| 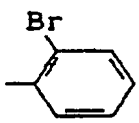 | $CH_3$ | 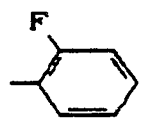 |
| 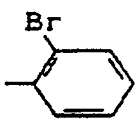 | $CH_3$ | 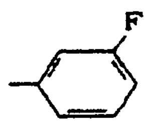 |
| 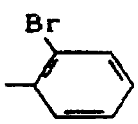 | $CH_3$ | 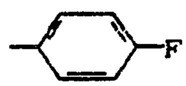 |
| 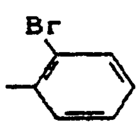 | $CH_3$ | 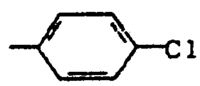 |
| 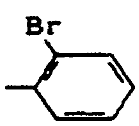 | $CH_3$ | 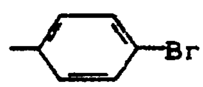 |
| 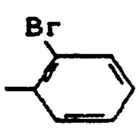 | $CH_3$ | 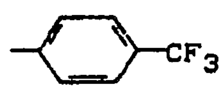 |

Tabelle 1 (Fortsetzung)

| $R^1$ | $R^2$ | Ar |
|---|---|---|
| 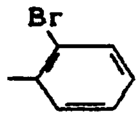 | $CH_3$ | 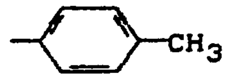 |
| 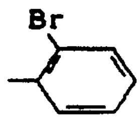 | $CH_3$ | 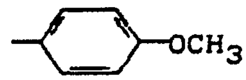 |
| 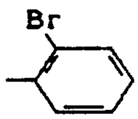 | $CH_3$ | 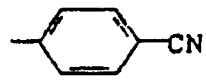 |
| 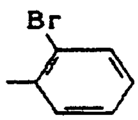 | $CH_3$ | 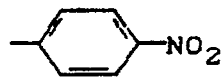 |
| 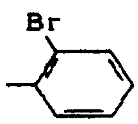 | $CH_3$ | 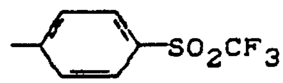 |
| 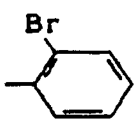 | $CH_3$ | 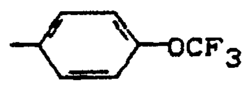 |
| 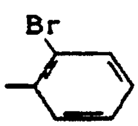 | $CH_3$ | 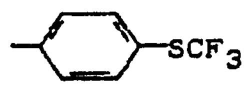 |
| 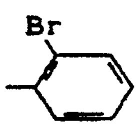 | $CH_3$ | 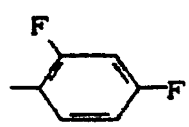 |

<u>Tabelle 1</u> (Fortsetzung)

| $R^1$ | $R^2$ | Ar |
|---|---|---|
| 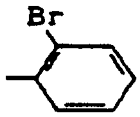 | $CH_3$ | 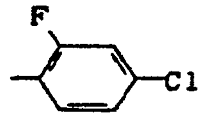 |
| 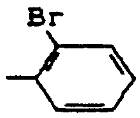 | $CH_3$ | 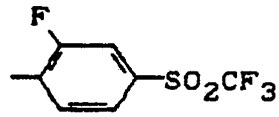 |
| 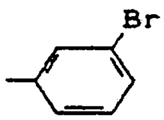 | $CH_3$ | 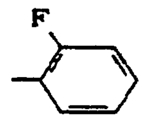 |
| 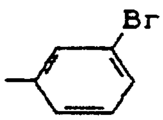 | $CH_3$ | 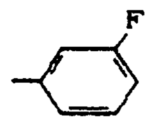 |
| 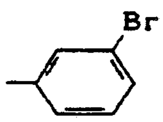 | $CH_3$ | 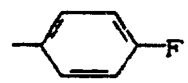 |
| 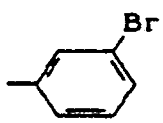 | $CH_3$ | 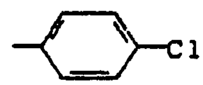 |
| 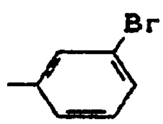 | $CH_3$ | 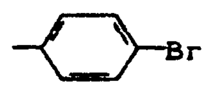 |
| 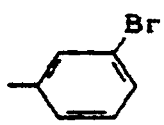 | $CH_3$ | 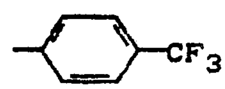 |

<u>Tabelle 1</u> (Fortsetzung)

| $R^1$ | $R^2$ | Ar |
|---|---|---|
| 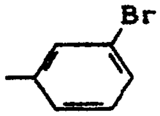 | $CH_3$ | 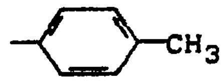 |
| 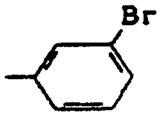 | $CH_3$ | 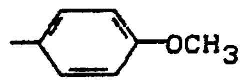 |
| 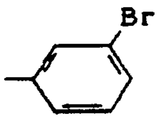 | $CH_3$ | 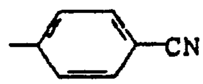 |
| 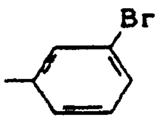 | $CH_3$ | 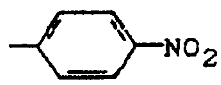 |
| 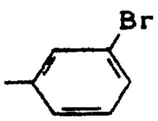 | $CH_3$ | 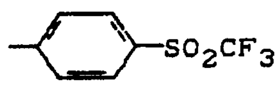 |
| 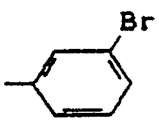 | $CH_3$ | 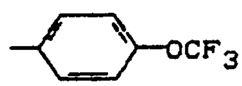 |
| 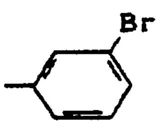 | $CH_3$ | 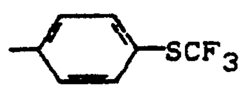 |
| 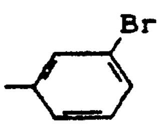 | $CH_3$ | 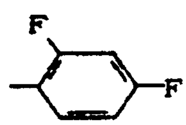 |

Tabelle 1 (Fortsetzung)

| $R^1$ | $R^2$ | Ar |
|---|---|---|
| 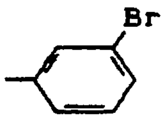 | $CH_3$ | 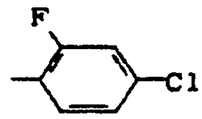 |
| 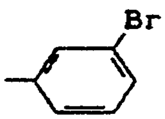 | $CH_3$ | 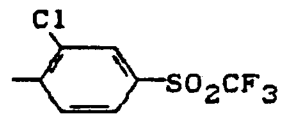 |
| 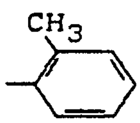 | $CH_3$ | 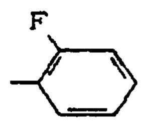 |
| 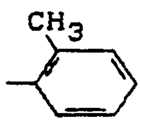 | $CH_3$ | 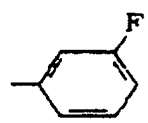 |
| 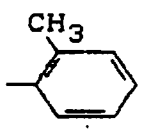 | $CH_3$ | 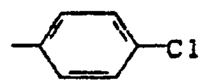 |
| 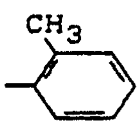 | $CH_3$ | 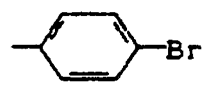 |
| 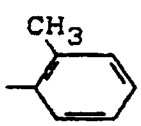 | $CH_3$ | 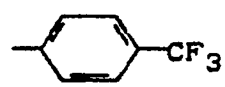 |
| 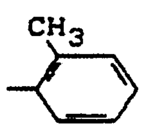 | $CH_3$ | 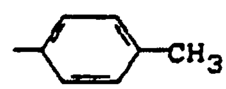 |

Tabelle 1 (Fortsetzung)

| $R^1$ | $R^2$ | Ar |
| --- | --- | --- |
| 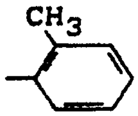 | $CH_3$ | 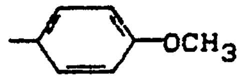 |
| 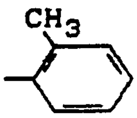 | $CH_3$ | 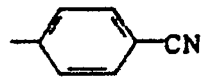 |
| 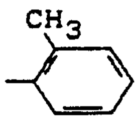 | $CH_3$ | 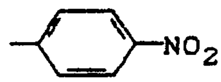 |
| 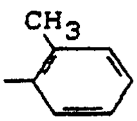 | $CH_3$ | 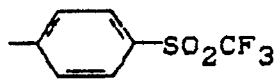 |
| 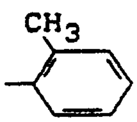 | $CH_3$ | 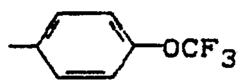 |
| 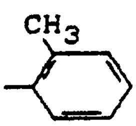 | $CH_3$ | 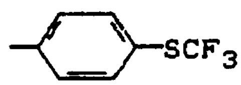 |
| 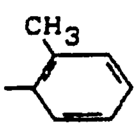 | $CH_3$ | 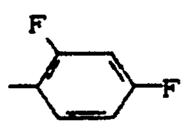 |
| 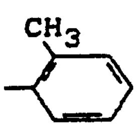 | $CH_3$ | 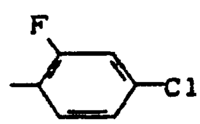 |

Tabelle 1 (Fortsetzung)

| $R^1$ | $R^2$ | Ar |
| --- | --- | --- |
| 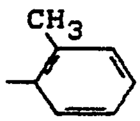 | $CH_3$ | 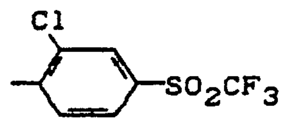 |
| 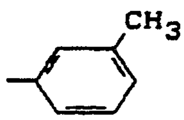 | $CH_3$ | 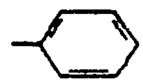 |
| 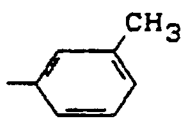 | $CH_3$ | 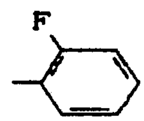 |
| 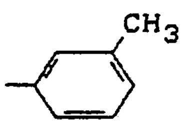 | $CH_3$ | 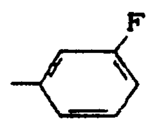 |
| 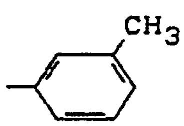 | $CH_3$ | 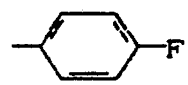 |
| 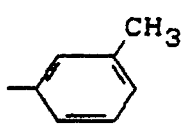 | $CH_3$ | 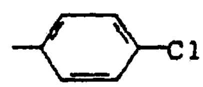 |
| 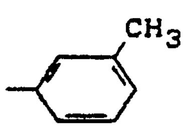 | $CH_3$ | 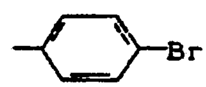 |
| 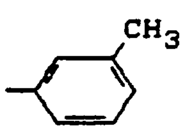 | $CH_3$ | 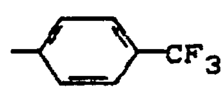 |

Tabelle 1 (Fortsetzung)

| $R^1$ | $R^2$ | Ar |
|---|---|---|
| 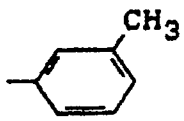 | $CH_3$ | 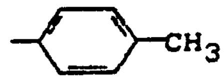 |
| 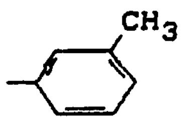 | $CH_3$ | 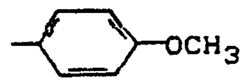 |
| 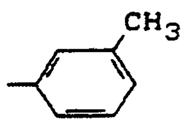 | $CH_3$ | 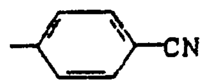 |
| 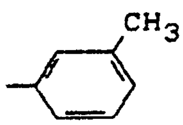 | $CH_3$ | 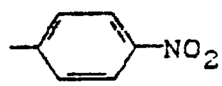 |
| 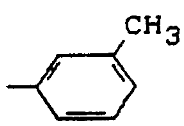 | $CH_3$ | 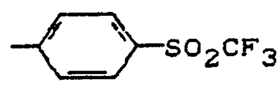 |
| 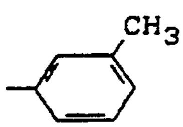 | $CH_3$ | 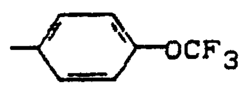 |
| 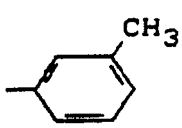 | $CH_3$ | 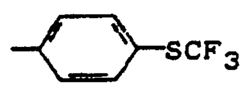 |
| 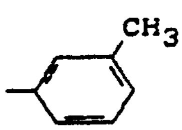 | $CH_3$ | 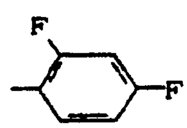 |

<u>Tabelle 1</u> (Fortsetzung)

| $R^1$ | $R^2$ | Ar |
|---|---|---|
| 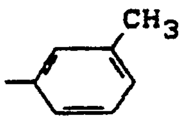 | $CH_3$ | 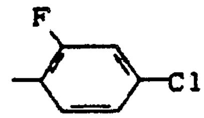 |
| 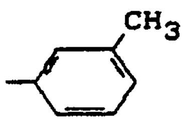 | $CH_3$ | 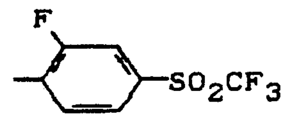 |
| 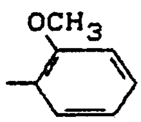 | $CH_3$ | 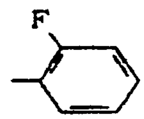 |
| 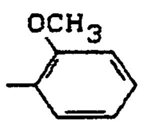 | $CH_3$ | 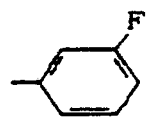 |
| 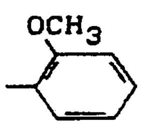 | $CH_3$ | 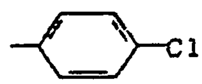 |
| 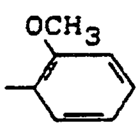 | $CH_3$ | 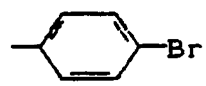 |
| 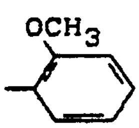 | $CH_3$ | 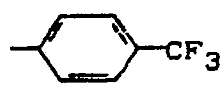 |
| 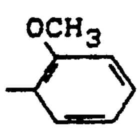 | $CH_3$ | 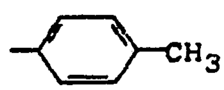 |

Tabelle 1 (Fortsetzung)

| $R^1$ | $R^2$ | Ar |
|---|---|---|
| 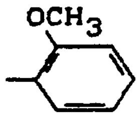 | $CH_3$ | 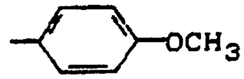 |
| 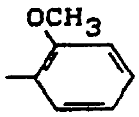 | $CH_3$ | 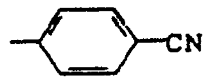 |
| 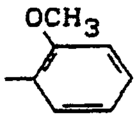 | $CH_3$ | 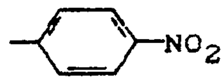 |
| 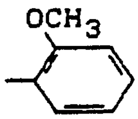 | $CH_3$ | 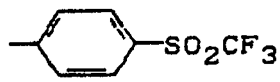 |
| 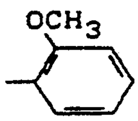 | $CH_3$ | 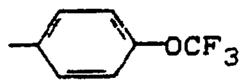 |
| 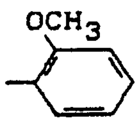 | $CH_3$ | 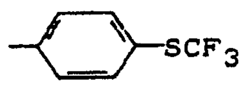 |
| 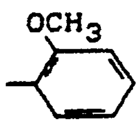 | $CH_3$ | 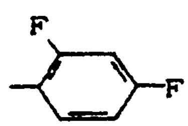 |
| 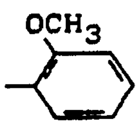 | $CH_3$ | 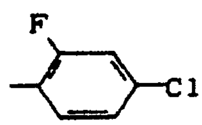 |

Tabelle 1 (Fortsetzung)

| $R^1$ | $R^2$ | Ar |
|---|---|---|
| 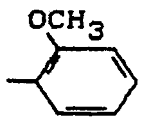 | $CH_3$ | 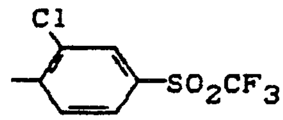 |
| 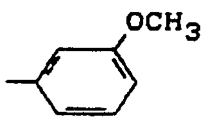 | $CH_3$ | 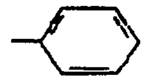 |
| 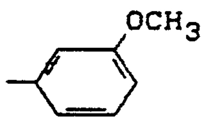 | $CH_3$ | 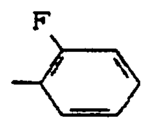 |
| 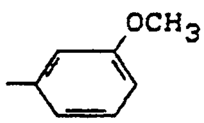 | $CH_3$ | 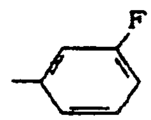 |
| 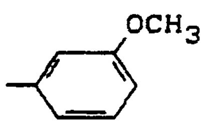 | $CH_3$ | 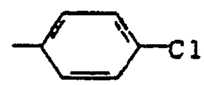 |
| 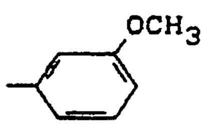 | $CH_3$ | 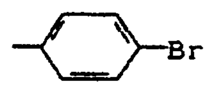 |
| 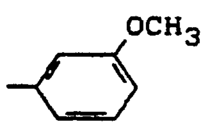 | $CH_3$ | 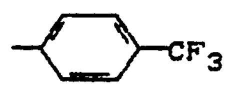 |
| 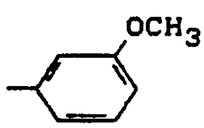 | $CH_3$ | 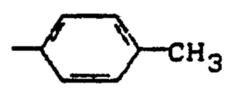 |

Tabelle 1 (Fortsetzung)

| $R^1$ | $R^2$ | Ar |
|---|---|---|
| 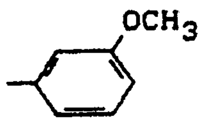 | $CH_3$ | 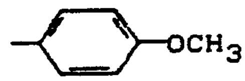 |
| 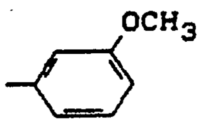 | $CH_3$ | 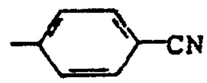 |
| 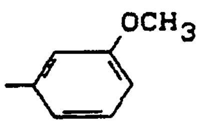 | $CH_3$ | 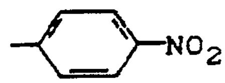 |
| 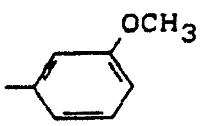 | $CH_3$ | 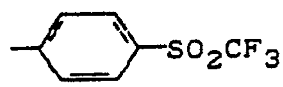 |
| 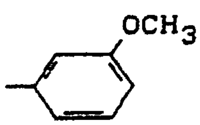 | $CH_3$ | 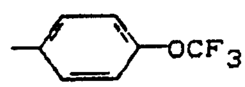 |
| 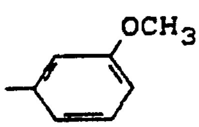 | $CH_3$ | 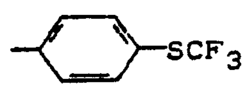 |
| 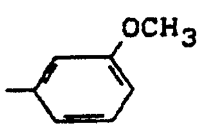 | $CH_3$ | 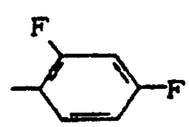 |
| 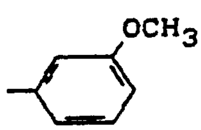 | $CH_3$ | 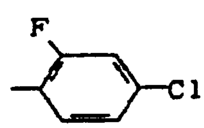 |

<u>Tabelle 1</u> (Fortsetzung)

| $R^1$ | $R^2$ | Ar |
|---|---|---|
| 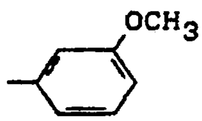 | $CH_3$ | 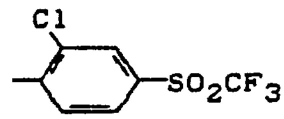 |
| 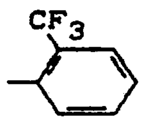 | $CH_3$ | 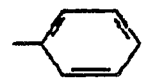 |
| 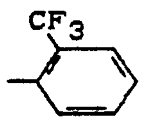 | $CH_3$ | 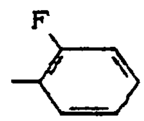 |
| 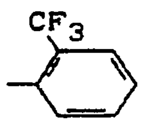 | $CH_3$ | 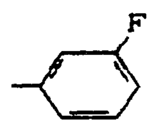 |
| 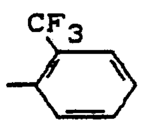 | $CH_3$ | 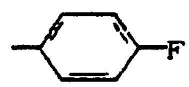 |
| 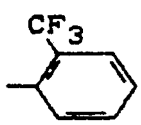 | $CH_3$ | 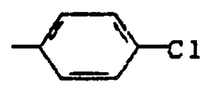 |
| 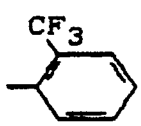 | $CH_3$ | 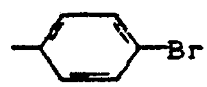 |
| 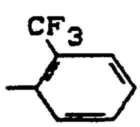 | $CH_3$ | 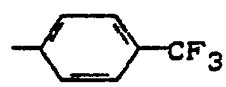 |

Tabelle 1 (Fortsetzung)

| $R^1$ | $R^2$ | Ar |
|---|---|---|
| 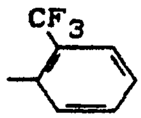 | $CH_3$ | 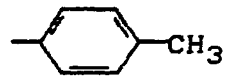 |
| 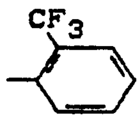 | $CH_3$ | 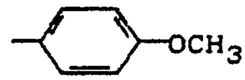 |
| 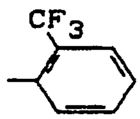 | $CH_3$ | 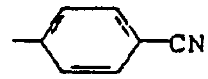 |
| 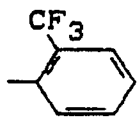 | $CH_3$ | 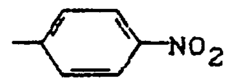 |
| 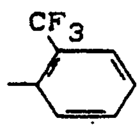 | $CH_3$ | 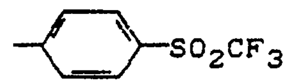 |
| 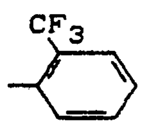 | $CH_3$ | 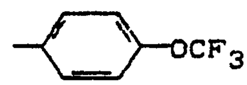 |
| 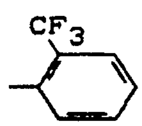 | $CH_3$ | 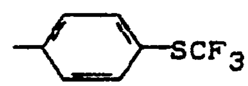 |
| 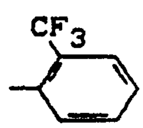 | $CH_3$ | 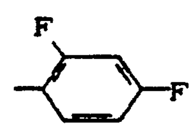 |

Tabelle 1 (Fortsetzung)

| $R^1$ | $R^2$ | Ar |
|---|---|---|
| 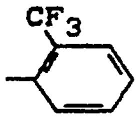 | $CH_3$ | 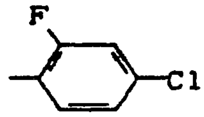 |
| 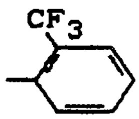 | $CH_3$ | 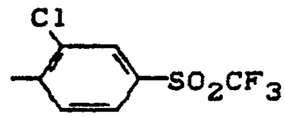 |
| 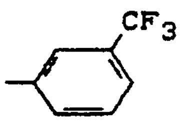 | $CH_3$ | 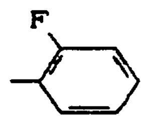 |
| 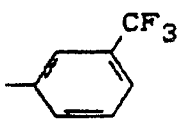 | $CH_3$ | 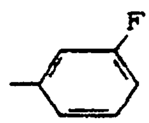 |
| 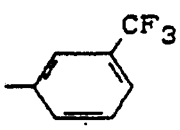 | $CH_3$ | 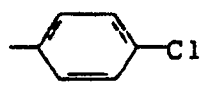 |
| 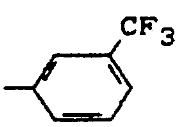 | $CH_3$ | 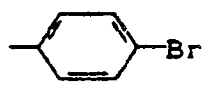 |
| 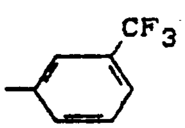 | $CH_3$ | 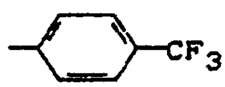 |
| 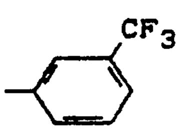 | $CH_3$ | 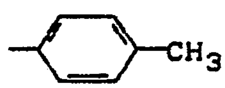 |

Tabelle 1 (Fortsetzung)

| $R^1$ | $R^2$ | Ar |
|---|---|---|
| 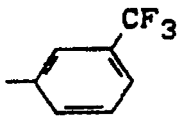 | $CH_3$ | 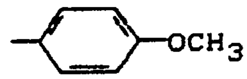 |
| 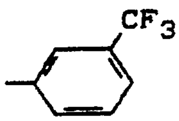 | $CH_3$ | 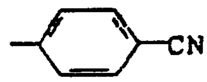 |
| 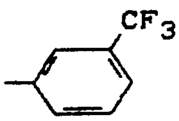 | $CH_3$ | 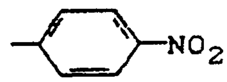 |
| 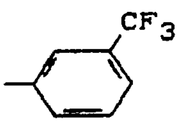 | $CH_3$ | 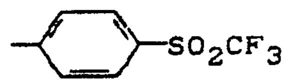 |
| 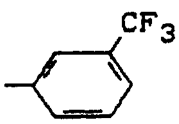 | $CH_3$ | 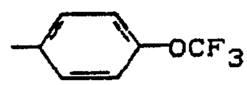 |
| 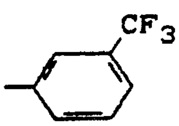 | $CH_3$ | 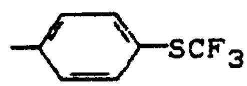 |
| 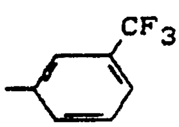 | $CH_3$ | 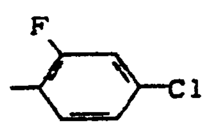 |
| 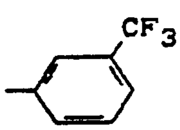 | $CH_3$ | 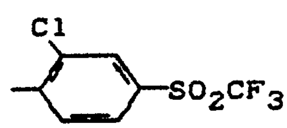 |

Tabelle 1 (Fortsetzung)

| $R^1$ | $R^2$ | Ar |
|---|---|---|
| 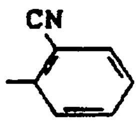 | $CH_3$ | 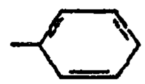 |
| 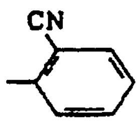 | $CH_3$ | 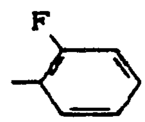 |
| 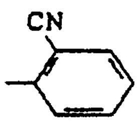 | $CH_3$ | 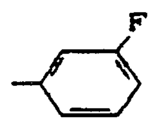 |
| 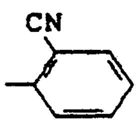 | $CH_3$ | 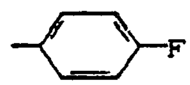 |
| 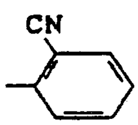 | $CH_3$ | 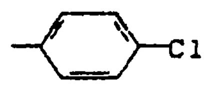 |
| 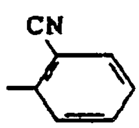 | $CH_3$ | 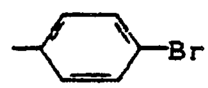 |
| 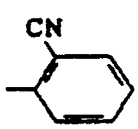 | $CH_3$ | 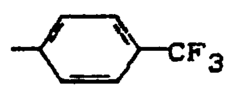 |
| 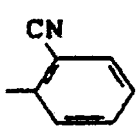 | $CH_3$ | 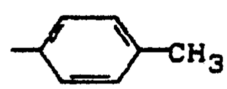 |

<u>Tabelle 1</u> (Fortsetzung)

| $R^1$ | $R^2$ | Ar |
|---|---|---|
| 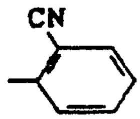 | $CH_3$ | 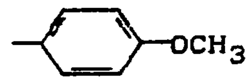 |
| 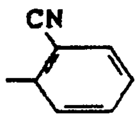 | $CH_3$ | 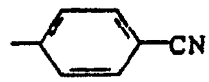 |
| 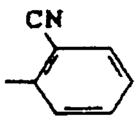 | $CH_3$ | 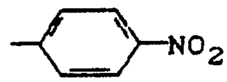 |
| 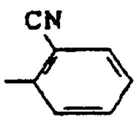 | $CH_3$ | 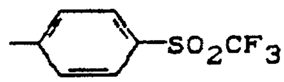 |
| 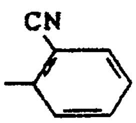 | $CH_3$ | 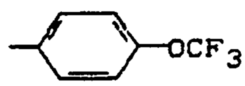 |
| 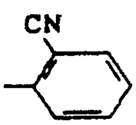 | $CH_3$ | 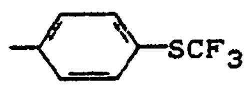 |
| 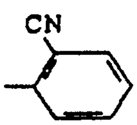 | $CH_3$ | 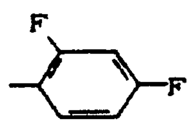 |
| 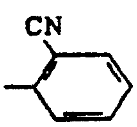 | $CH_3$ | 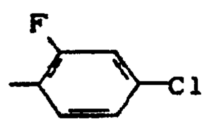 |

<u>Tabelle 1</u> (Fortsetzung)

| $R^1$ | $R^2$ | Ar |
|---|---|---|
| 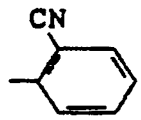 | $CH_3$ | 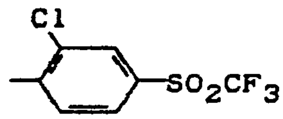 |
| 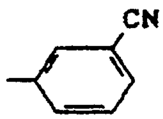 | $CH_3$ | 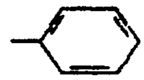 |
| 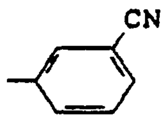 | $CH_3$ | 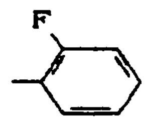 |
| 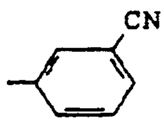 | $CH_3$ | 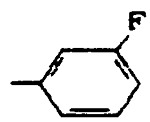 |
| 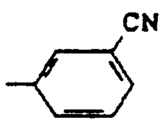 | $CH_3$ | 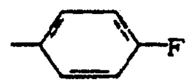 |
| 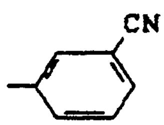 | $CH_3$ | 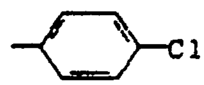 |
| 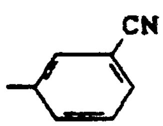 | $CH_3$ | 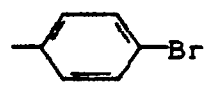 |
| 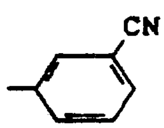 | $CH_3$ | 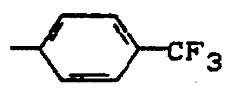 |

<u>Tabelle 1</u> (Fortsetzung)

| $R^1$ | $R^2$ | Ar |
|---|---|---|
| 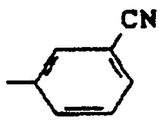 | $CH_3$ | 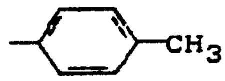 |
| 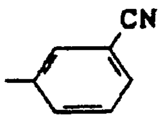 | $CH_3$ | 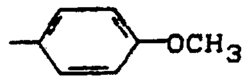 |
| 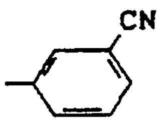 | $CH_3$ | 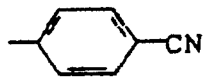 |
| 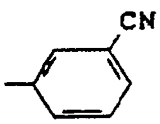 | $CH_3$ | 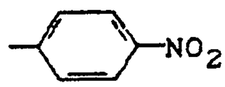 |
| 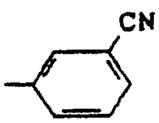 | $CH_3$ | 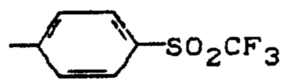 |
| 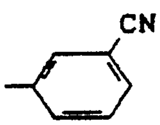 | $CH_3$ | 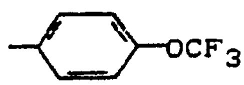 |
| 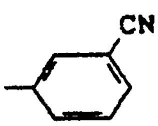 | $CH_3$ | 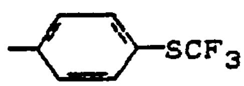 |
| 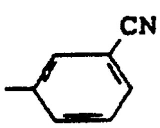 | $CH_3$ | 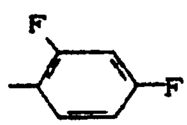 |

Tabelle 1 (Fortsetzung)

| $R^1$ | $R^2$ | Ar |
|---|---|---|
| 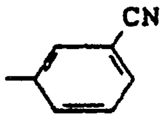 | $CH_3$ | 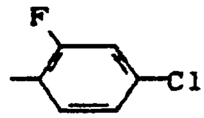 |
| 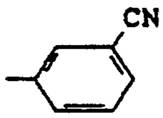 | $CH_3$ | 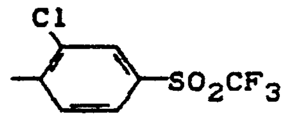 |
| 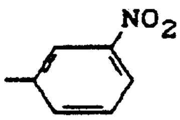 | $CH_3$ | 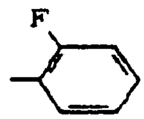 |
| 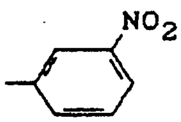 | $CH_3$ | 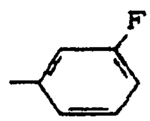 |
| 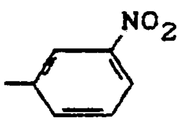 | $CH_3$ | 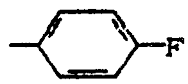 |
| 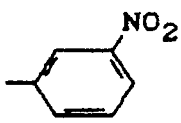 | $CH_3$ | 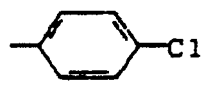 |
| 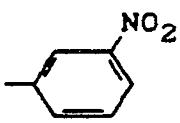 | $CH_3$ | 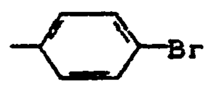 |
| 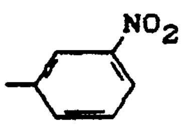 | $CH_3$ | 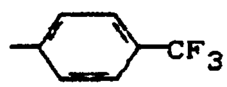 |

Tabelle 1 (Fortsetzung)

| $R^1$ | $R^2$ | Ar |
|---|---|---|
| 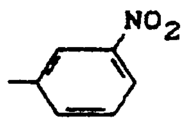 | $CH_3$ | 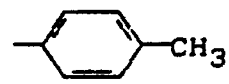 |
| 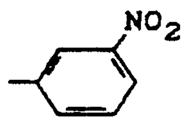 | $CH_3$ | 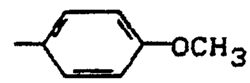 |
| 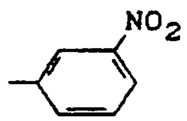 | $CH_3$ | 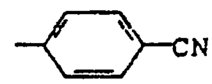 |
| 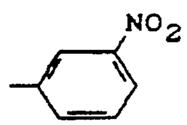 | $CH_3$ | 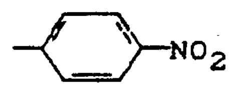 |
| 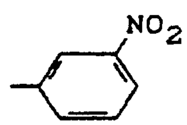 | $CH_3$ | 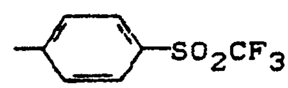 |
| 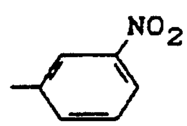 | $CH_3$ | 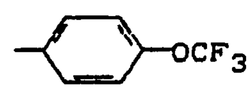 |
| 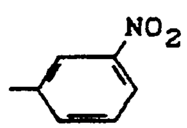 | $CH_3$ | 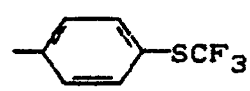 |
| 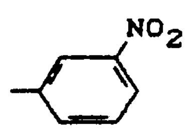 | $CH_3$ | 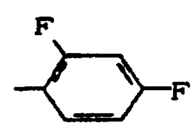 |

<u>Tabelle 1</u> (Fortsetzung)

| $R^1$ | $R^2$ | Ar |
|---|---|---|
| 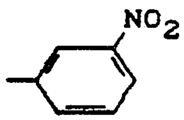 | $CH_3$ | 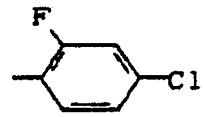 |
| 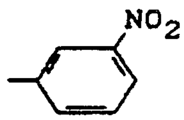 | $CH_3$ | 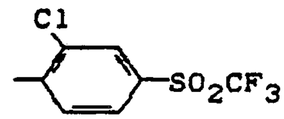 |
| 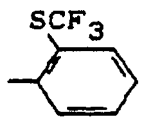 | $CH_3$ | 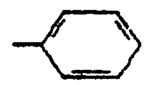 |
| 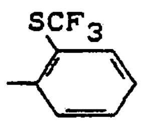 | $CH_3$ | 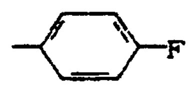 |
| 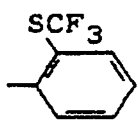 | $CH_3$ | 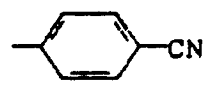 |
| 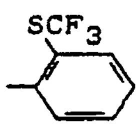 | $CH_3$ | 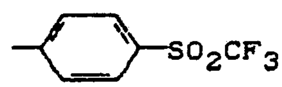 |
| 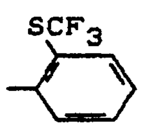 | $CH_3$ | 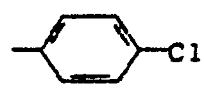 |
| 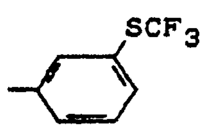 | $CH_3$ | 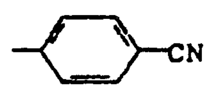 |

<u>Tabelle 1</u> (Fortsetzung)

| $R^1$ | $R^2$ | Ar |
|---|---|---|
| 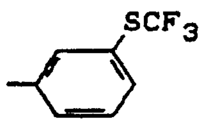 | $CH_3$ | 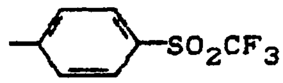 |
| 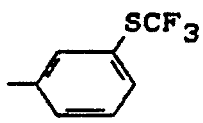 | $CH_3$ | 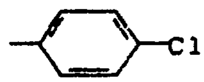 |
| 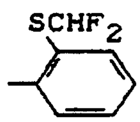 | $CH_3$ | 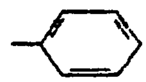 |
| 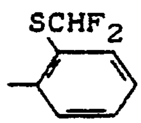 | $CH_3$ | 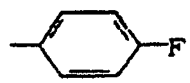 |
| 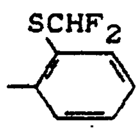 | $CH_3$ | 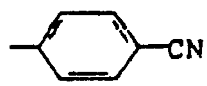 |
| 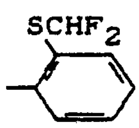 | $CH_3$ | 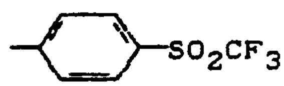 |
| 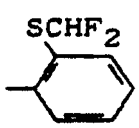 | $CH_3$ | 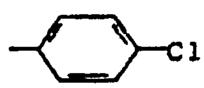 |
| 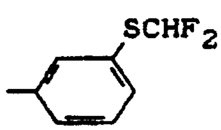 | $CH_3$ | 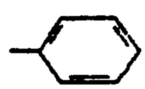 |

Tabelle 1 (Fortsetzung)

| $R^1$ | $R^2$ | Ar |
|---|---|---|
| 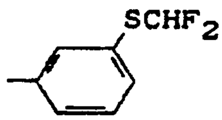 | $CH_3$ | 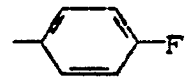 |
| 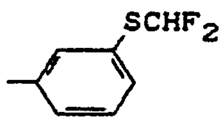 | $CH_3$ | 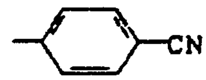 |
| 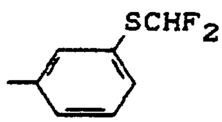 | $CH_3$ | 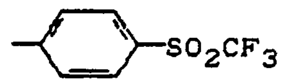 |
| 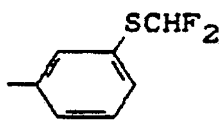 | $CH_3$ | 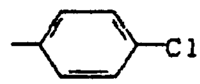 |
| 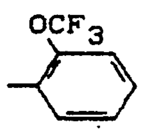 | $CH_3$ | 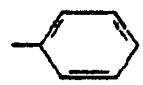 |
| 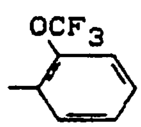 | $CH_3$ | 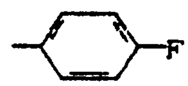 |
| 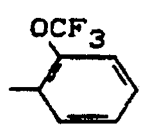 | $CH_3$ | 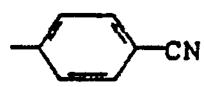 |
| 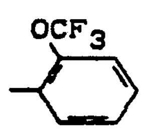 | $CH_3$ | 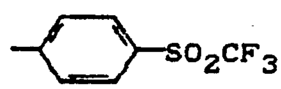 |

Tabelle 1 (Fortsetzung)

| $R^1$ | $R^2$ | Ar |
|---|---|---|
| 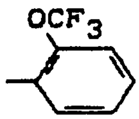 | $CH_3$ | 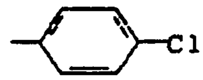 |
| 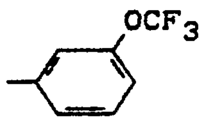 | $CH_3$ | 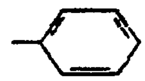 |
| 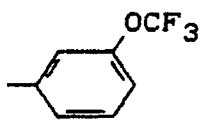 | $CH_3$ | 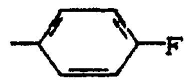 |
| 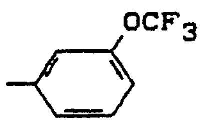 | $CH_3$ | 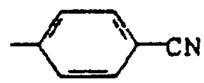 |
| 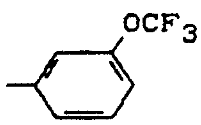 | $CH_3$ | 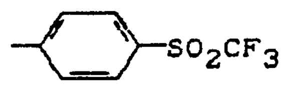 |
| 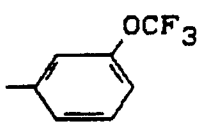 | $CH_3$ | 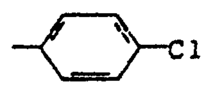 |
| 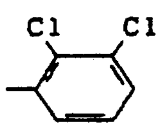 | $CH_3$ | 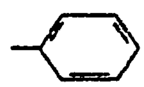 |
| 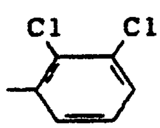 | $CH_3$ | 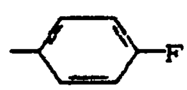 |

**Tabelle 1** (Fortsetzung)

| $R^1$ | $R^2$ | Ar |
|---|---|---|
| 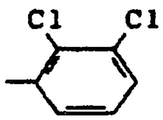 | $CH_3$ | 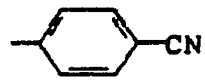 |
| 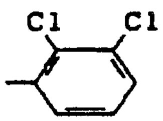 | $CH_3$ | 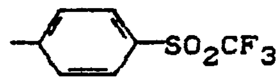 |
| 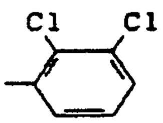 | $CH_3$ | 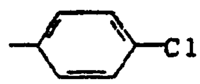 |
| 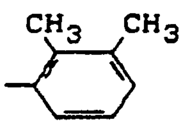 | $CH_3$ | 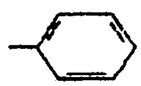 |
| 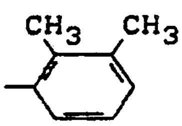 | $CH_3$ | 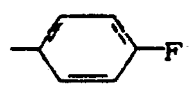 |
| 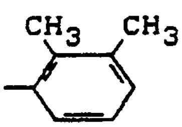 | $CH_3$ | 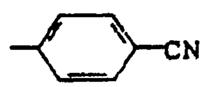 |
| 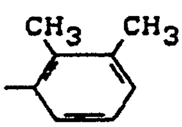 | $CH_3$ | 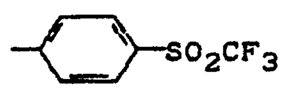 |
| 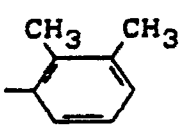 | $CH_3$ | 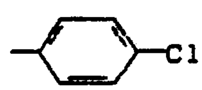 |

Tabelle 1 (Fortsetzung)

| $R^1$ | $R^2$ | Ar |
|---|---|---|
| 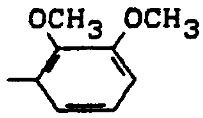 | $CH_3$ | 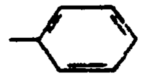 |
| 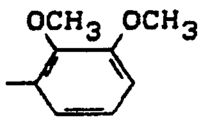 | $CH_3$ | 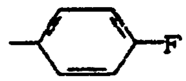 |
| 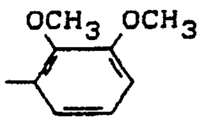 | $CH_3$ | 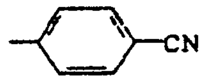 |
| 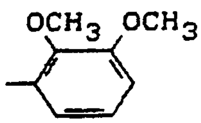 | $CH_3$ | 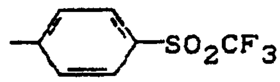 |
| 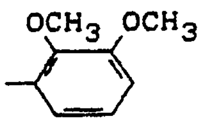 | $CH_3$ | 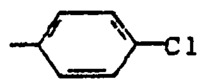 |
| 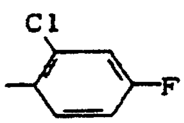 | $CH_3$ | 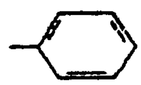 |
| 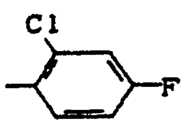 | $CH_3$ | 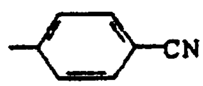 |
| 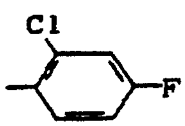 | $CH_3$ | 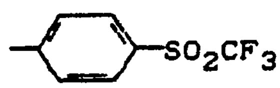 |

Tabelle 1 (Fortsetzung)

| $R^1$ | $R^2$ | Ar |
|---|---|---|
| 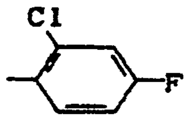 | $CH_3$ | 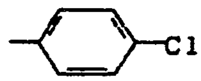 |
| 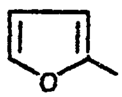 | $CH_3$ | 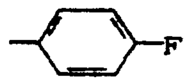 |
| 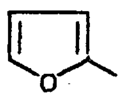 | $CH_3$ | 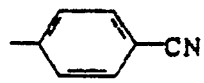 |
| 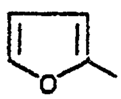 | $CH_3$ | 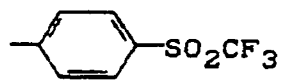 |
| 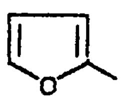 | $CH_3$ | 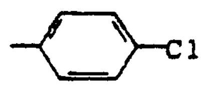 |
| 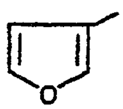 | $CH_3$ | 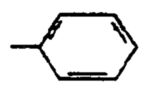 |
| 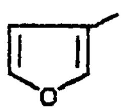 | $CH_3$ | 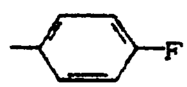 |
| 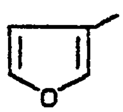 | $CH_3$ | 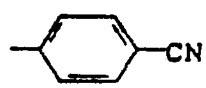 |
| 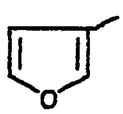 | $CH_3$ | 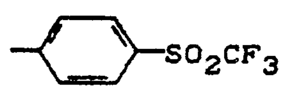 |
| 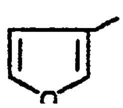 | $CH_3$ | 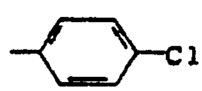 |

Tabelle 1 (Fortsetzung)

| $R^1$ | $R^2$ | Ar |
|---|---|---|
| 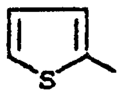 | $CH_3$ | 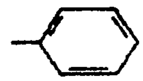 |
| 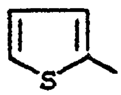 | $CH_3$ | 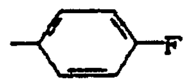 |
| 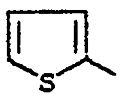 | $CH_3$ | 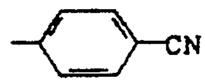 |
| 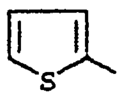 | $CH_3$ | 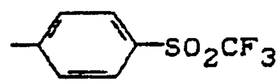 |
| 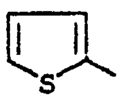 | $CH_3$ | 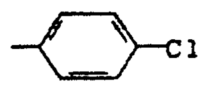 |
| 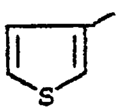 | $CH_3$ | 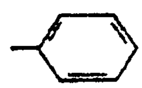 |
| 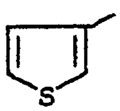 | $CH_3$ | 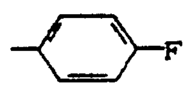 |
| 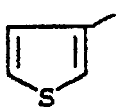 | $CH_3$ | 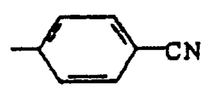 |
| 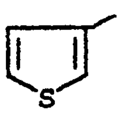 | $CH_3$ | 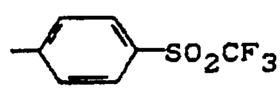 |
| 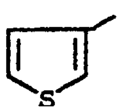 | $CH_3$ | 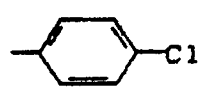 |

<u>Tabelle 1</u> (Fortsetzung)

| $R^1$ | $R^2$ | Ar |
|---|---|---|
| 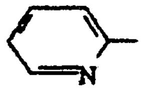 | $CH_3$ | 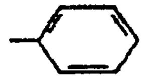 |
| 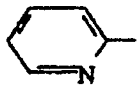 | $CH_3$ | 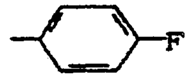 |
| 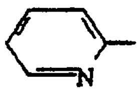 | $CH_3$ | 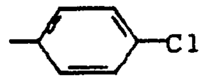 |
| 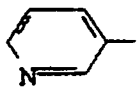 | $CH_3$ | 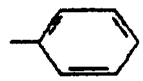 |
| 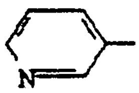 | $CH_3$ | 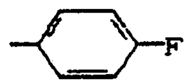 |
| 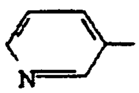 | $CH_3$ | 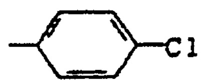 |
| 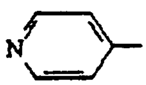 | $CH_3$ | 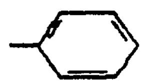 |
| 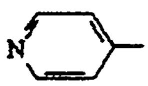 | $CH_3$ | 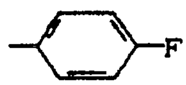 |
| 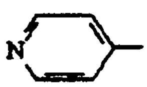 | $CH_3$ | 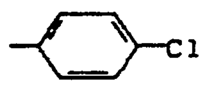 |
| 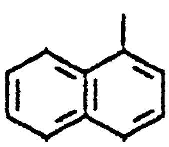 | $CH_3$ | 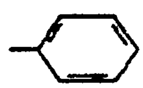 |

Tabelle 1 (Fortsetzung)

| $R^1$ | $R^2$ | Ar |
|---|---|---|
| 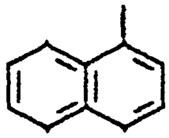 | $CH_3$ | 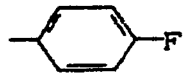 |
| 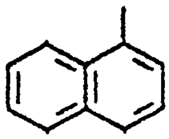 | $CH_3$ | 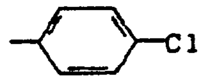 |
| 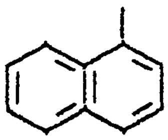 | $CH_3$ | 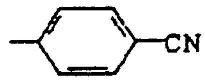 |
| 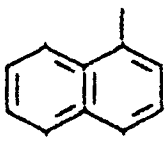 | $CH_3$ | 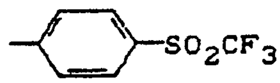 |
| 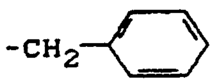 | $CH_3$ | 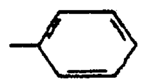 |
| 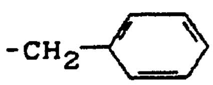 | $CH_3$ | 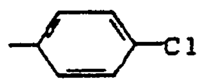 |
| 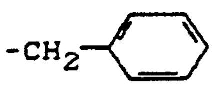 | $CH_3$ | 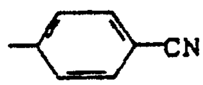 |
| 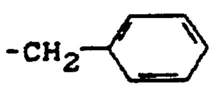 | $CH_3$ | 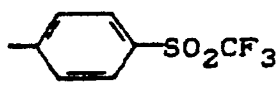 |
| 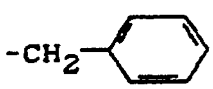 | $CH_3$ | 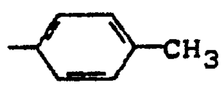 |

Tabelle 1 (Fortsetzung)

| $R^1$ | $R^2$ | Ar |
|---|---|---|
| 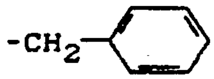 | $CH_3$ | 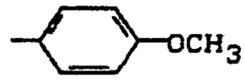 |
| 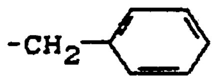 | $CH_3$ | 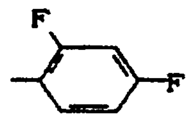 |
| 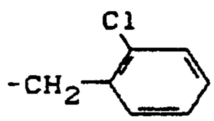 | $CH_3$ | 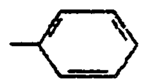 |
| 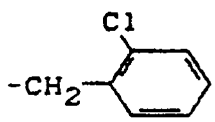 | $CH_3$ | 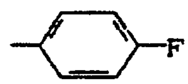 |
| 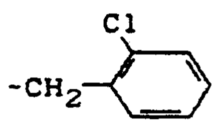 | $CH_3$ | 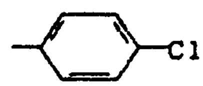 |
| 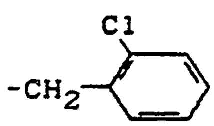 | $CH_3$ | 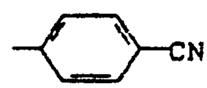 |
| 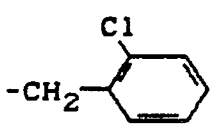 | $CH_3$ | 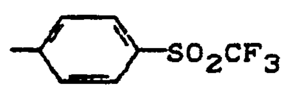 |
| 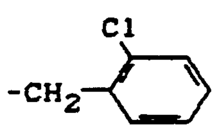 | $CH_3$ | 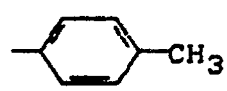 |

Tabelle 1 (Fortsetzung)

| $R^1$ | $R^2$ | Ar |
|---|---|---|
| 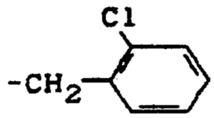 | $CH_3$ | 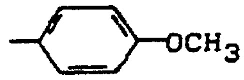 |
| 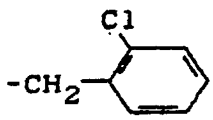 | $CH_3$ | 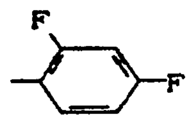 |
| 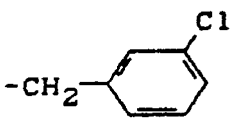 | $CH_3$ | 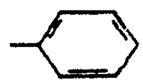 |
| 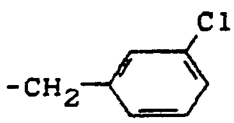 | $CH_3$ | 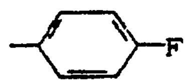 |
| 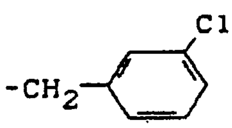 | $CH_3$ | 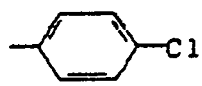 |
| 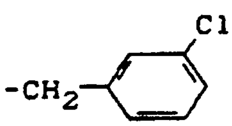 | $CH_3$ | 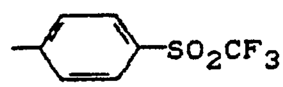 |
| 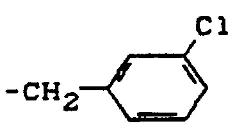 | $CH_3$ | 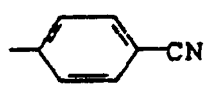 |
| 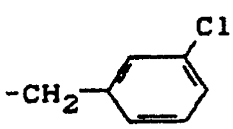 | $CH_3$ | 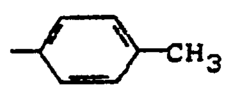 |

Tabelle 1 (Fortsetzung)

| $R^1$ | $R^2$ | Ar |
|---|---|---|
| 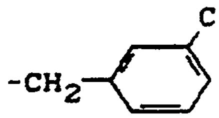 | $CH_3$ | 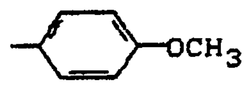 |
| 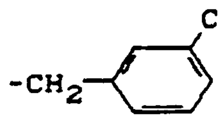 | $CH_3$ | 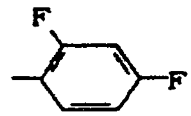 |
| 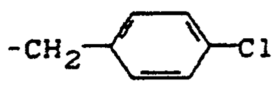 | $CH_3$ | 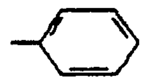 |
| 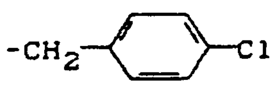 | $CH_3$ | 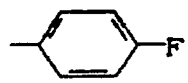 |
| 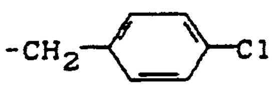 | $CH_3$ | 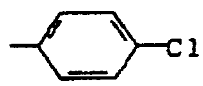 |
| 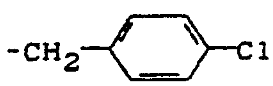 | $CH_3$ | 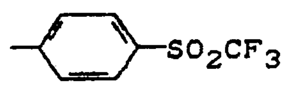 |
| 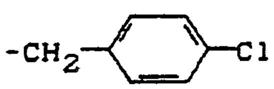 | $CH_3$ | 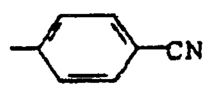 |
| 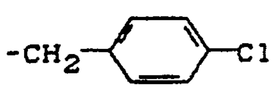 | $CH_3$ | 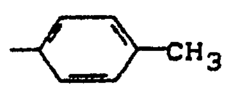 |
| 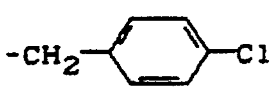 | $CH_3$ | 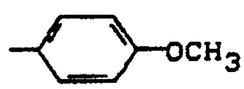 |
| 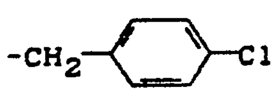 | $CH_3$ | 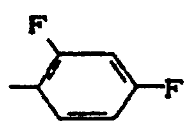 |

Tabelle 1 (Fortsetzung)

| $R^1$ | $R^2$ | Ar |
|---|---|---|
| 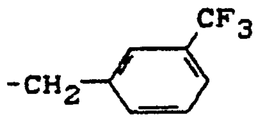 | $CH_3$ | 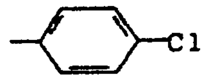 |
| 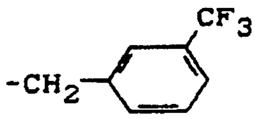 | $CH_3$ | 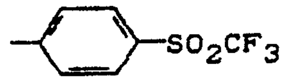 |
| 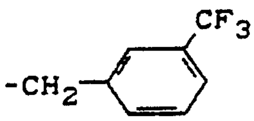 | $CH_3$ | 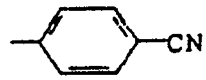 |
| 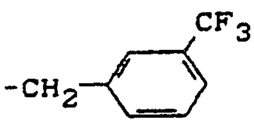 | $CH_3$ | 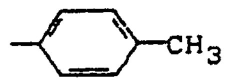 |
| 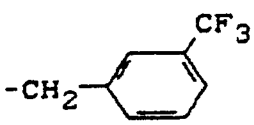 | $CH_3$ | 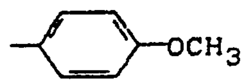 |
| 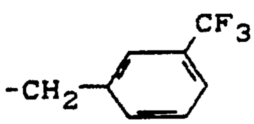 | $CH_3$ | 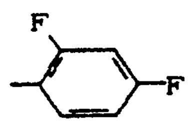 |
| 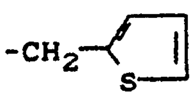 | $CH_3$ | 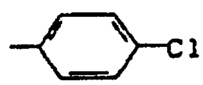 |
| 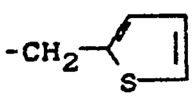 | $CH_3$ | 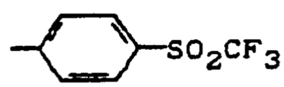 |
| 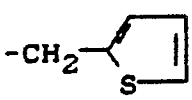 | $CH_3$ | 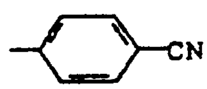 |

Tabelle 1 (Fortsetzung)

| $R^1$ | $R^2$ | Ar |
|---|---|---|
| 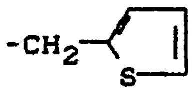 | $CH_3$ | 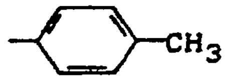 |
| 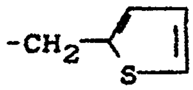 | $CH_3$ | 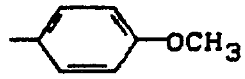 |
| 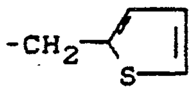 | $CH_3$ | 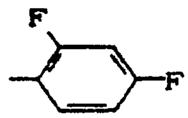 |
| 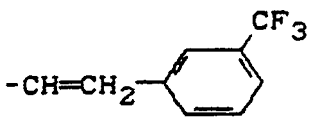 | $CH_3$ | 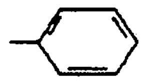 |
| 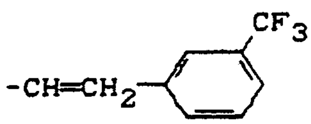 | $CH_3$ | 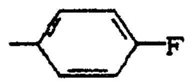 |
| 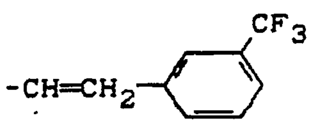 | $CH_3$ | 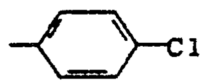 |
| 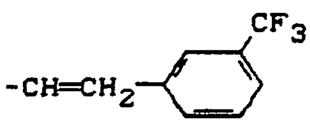 | $CH_3$ | 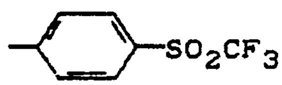 |
| 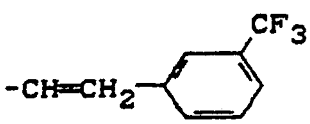 | $CH_3$ | 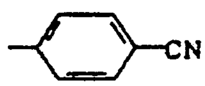 |
| 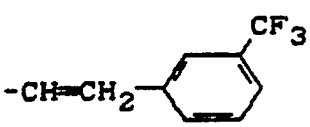 | $CH_3$ | 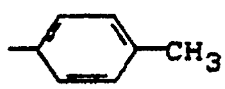 |

Tabelle 1 (Fortsetzung)

| $R^1$ | $R^2$ | Ar |
|---|---|---|
| 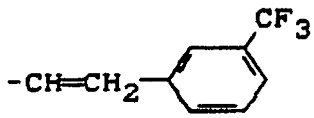 | $CH_3$ | 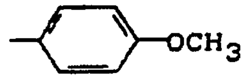 |
| 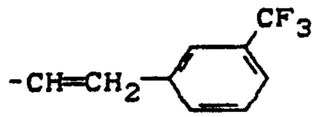 | $CH_3$ | 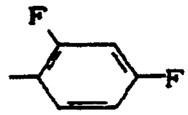 |
| 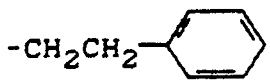 | $CH_3$ | 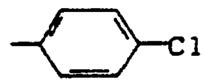 |
| 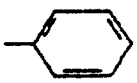 | $-CH_2CH_3$ | 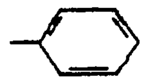 |
| 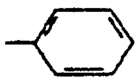 | $-CH_2CH_3$ | 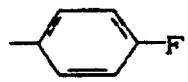 |
| 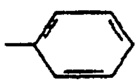 | $-CH_2CH_3$ | 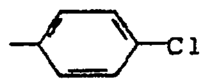 |
| 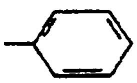 | $-CH_2CF_3$ | 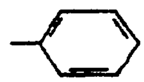 |
| 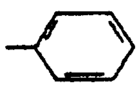 | $-CH_2CF_3$ | 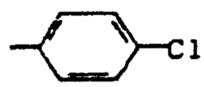 |
| 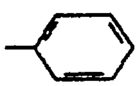 | $-CH(CH_3)_2$ | 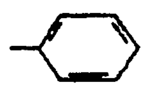 |
| 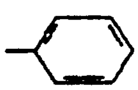 | $-CH(CH_3)_2$ | 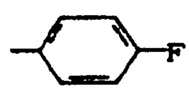 |

Tabelle 1 (Fortsetzung)

| $R^1$ | $R^2$ | Ar |
|---|---|---|
| 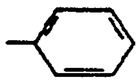 | $-CH(CH_3)_2$ | 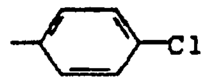 |
| 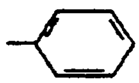 | $-C_3H_7-n$ | 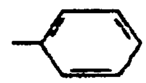 |
| 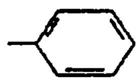 | $-C_3H_7-n$ | 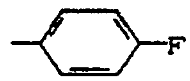 |
| 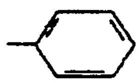 | $-C_3H_7-n$ | 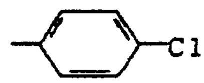 |
| 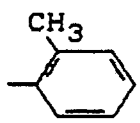 | $-CH_2CH_3$ | 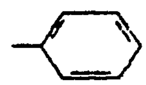 |
| 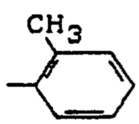 | $-CH_2CH_3$ | 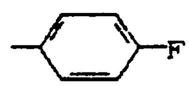 |
| 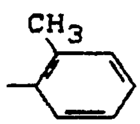 | $-CH_2CH_3$ | 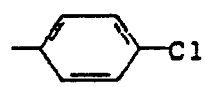 |
| 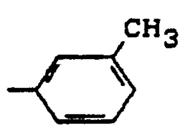 | $-CH_2CH_3$ | 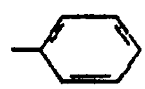 |
| 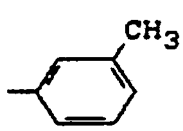 | $-CH_2CH_3$ | 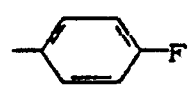 |

Tabelle 1 (Fortsetzung)

| $R^1$ | $R^2$ | Ar |
|---|---|---|
| 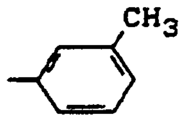 | $-CH_2CH_3$ | 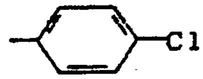 |
| 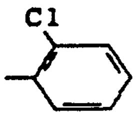 | $-CH_2CH_3$ | 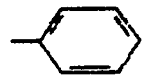 |
| 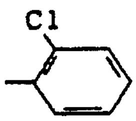 | $-CH_2CH_3$ | 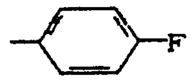 |
| 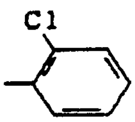 | $-CH_2CH_3$ | 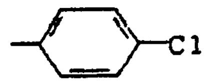 |
| 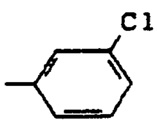 | $-CH_2CH_3$ | 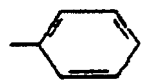 |
| 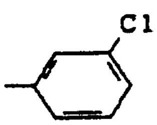 | $-CH_2CH_3$ | 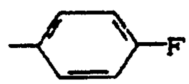 |
| 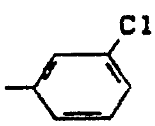 | $-CH_2CH_3$ | 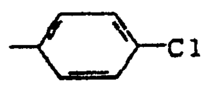 |
| 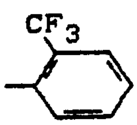 | $-CH_2CH_3$ | 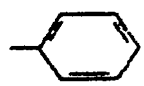 |

<u>Tabelle 1</u> (Fortsetzung)

| $R^1$ | $R^2$ | Ar |
|---|---|---|
| 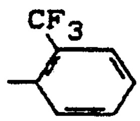 | $-CH_2CH_3$ | 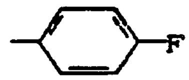 |
| 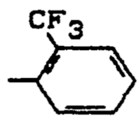 | $-CH_2CH_3$ | 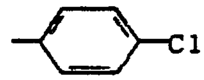 |
| 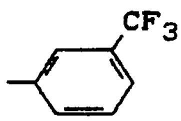 | $-CH_2CH_3$ | 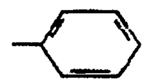 |
| 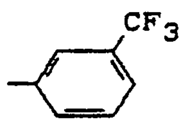 | $-CH_2CH_3$ | 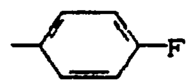 |
| 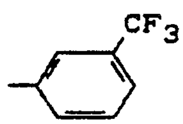 | $-CH_2CH_3$ | 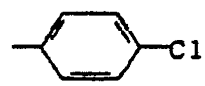 |
| 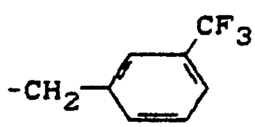 | $-CH_2CH_3$ | 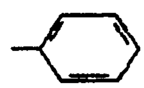 |
| 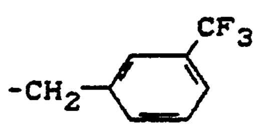 | $-CH_2CH_3$ | 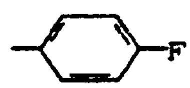 |
| 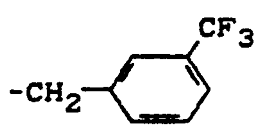 | $-CH_2CH_3$ | 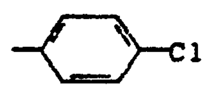 |

Tabelle 1 (Fortsetzung)

| $R^1$ | $R^2$ | Ar |
| --- | --- | --- |
| 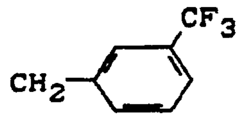 | $-CH_2CH_3$ | 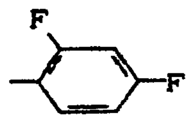 |
| 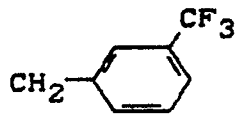 | $-CH(CH_3)_2$ | 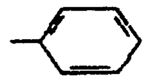 |
| 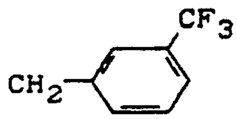 | $-CH(CH_3)_2$ | 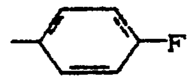 |
| 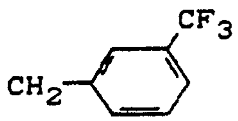 | $-CH(CH_3)_2$ | 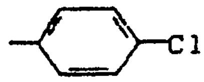 |
| 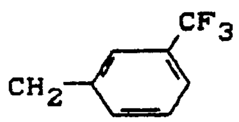 | $-CH(CH_3)_2$ | 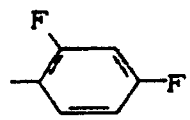 |
| 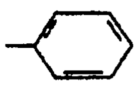 | $-CH_2-CH=CH_2$ | 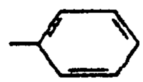 |
| 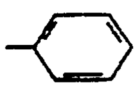 | $-CH_2-CH=CH_2$ | 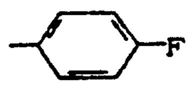 |
| 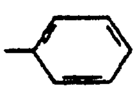 | $-CH_2-CH=CH_2$ | 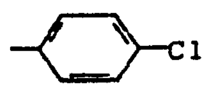 |
| 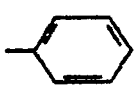 | $-CH_2-C\equiv CH$ | 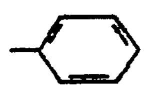 |

Tabelle 1 (Fortsetzung)

| $R^1$ | $R^2$ | Ar |
|---|---|---|
| 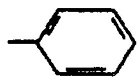 | $-CH_2-C\equiv CH$ | 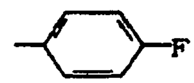 |
| 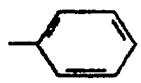 | $-CH_2-C\equiv CH$ | 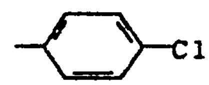 |

Verwendet man beispielsweise 1-(4-Fluorphenyl)-3-phenyl-4-(N,N-dimethylaminomethyliden)-2-pyrazolin-5-on und N-Methylhydroxylaminhydrochlorid als Ausgangsstoffe, so läßt sich der Reaktionsablauf des erfindungsgemäßen Verfahrens durch das folgende Formelschema darstellen:

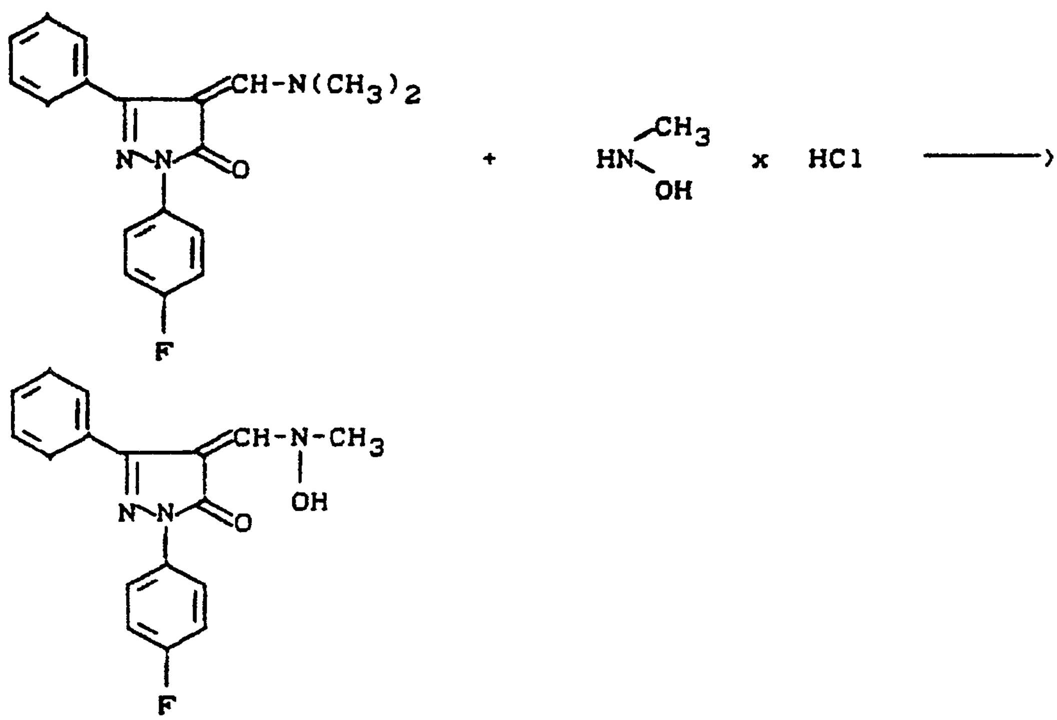

In Formel (II) haben $R^1$ und Ar vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und Ar angegeben wurden.

Beispiele für die Ausgangsstoffe der Formel (II) sind in Tabelle 2 aufgeführt.

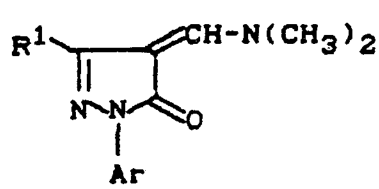

(II)

Tabelle 2

| $R^1$ | Ar |
|---|---|
| $-C_3H_7-n$ | 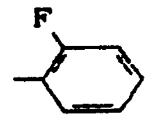 |
| $-C_3H_7-n$ | 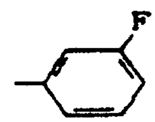 |
| $-C_3H_7-n$ | 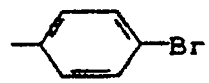 |
| $-C_3H_7-n$ | 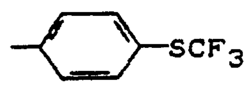 |
| $-C_3H_7-n$ | 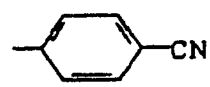 |
| $-C_3H_7-n$ | 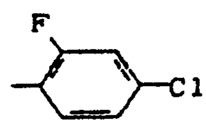 |
| $-CH_2OCH_3$ | 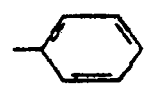 |
| $-CH_2OCH_3$ | 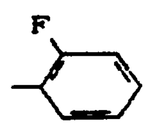 |
| $-CH_2OCH_3$ | 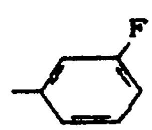 |

Tabelle 2 (Fortsetzung)

| $R^1$ | Ar |
|---|---|
| $-CH_2OCH_3$ | 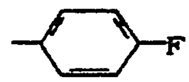 |
| $-CH_2OCH_3$ | 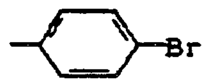 |
| $-CH_2OCH_3$ | 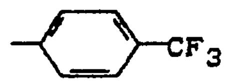 |
| $-CH_2OCH_3$ | 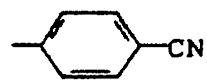 |
| $-CH_2OCH_3$ | 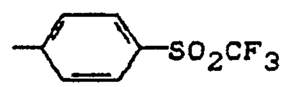 |
| $-CH_2OCH_3$ | 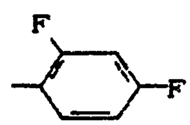 |
| $-C_4H_9-n$ | 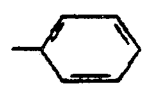 |
| $-C_4H_9-n$ | 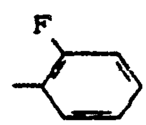 |
| $-C_4H_9-n$ | 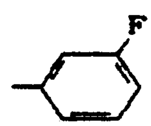 |
| $-C_4H_9-n$ | 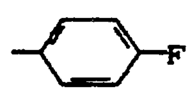 |

Tabelle 2 (Fortsetzung)

| $R^1$ | Ar |
|---|---|
| $-C_4H_9-n$ | 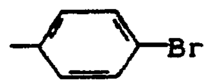 |
| $-C_4H_9-n$ | 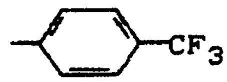 |
| $-C_4H_9-n$ | 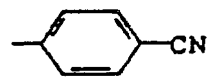 |
| $-C_4H_9-n$ | 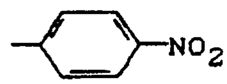 |
| $-C_4H_9-n$ | 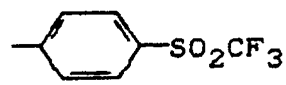 |
| $-C_4H_9-n$ | 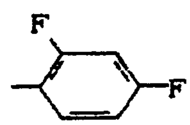 |
| $-CH_2-CH(CH_3)_2$ | 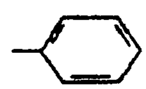 |
| $-CH_2-CH(CH_3)_2$ | 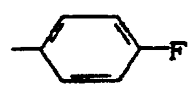 |
| $-CH_2-CH(CH_3)_2$ | 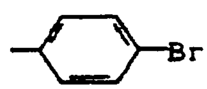 |
| $-CH_2-CH(CH_3)_2$ | 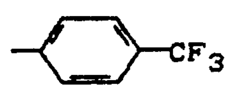 |

Tabelle 2 (Fortsetzung)

| $R^1$ | Ar |
|---|---|
| $-CH_2-CH(CH_3)_2$ | 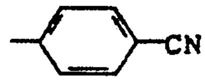 |
| $-CH_2-CH(CH_3)_2$ | 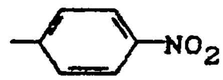 |
| $-CH_2-CH(CH_3)_2$ | 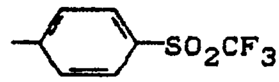 |
| $-CH_2-CH(CH_3)_2$ | 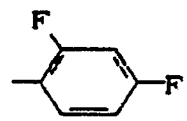 |
| $-CH_2-CH(CH_3)_2$ | 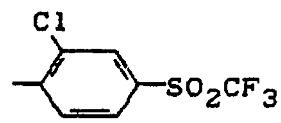 |
| 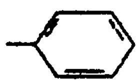 | 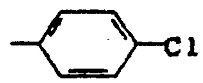 |
| 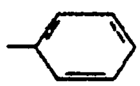 | 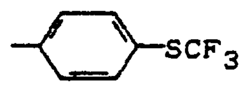 |
| 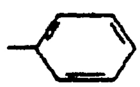 | 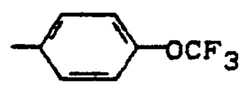 |
| 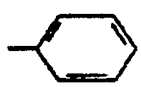 | 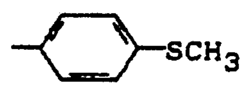 |
| 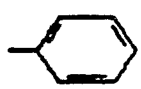 | 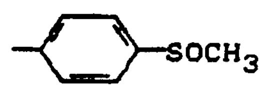 |

Tabelle 2 (Fortsetzung)

| $R^1$ | Ar |
|---|---|
| 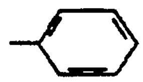 | 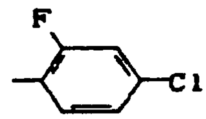 |
| 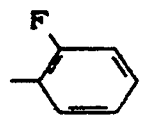 | 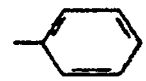 |
| 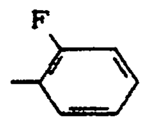 | 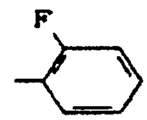 |
| 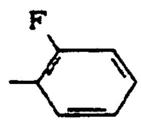 | 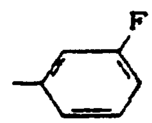 |
| 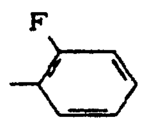 | 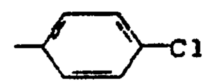 |
| 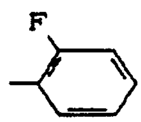 | 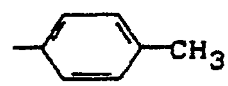 |
| 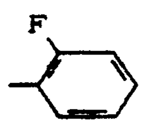 | 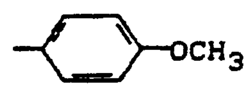 |
| 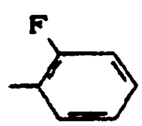 | 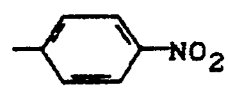 |

Tabelle 2 (Fortsetzung)

| $R^1$ | Ar |
|---|---|
| 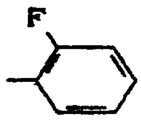 | 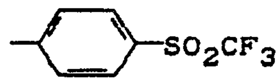 |
| 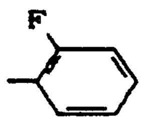 | 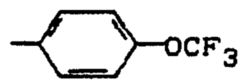 |
| 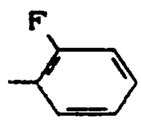 | 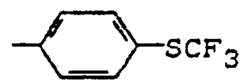 |
| 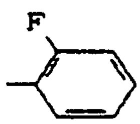 | 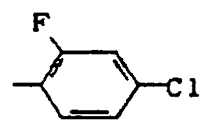 |
| 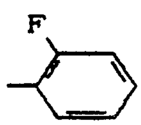 | 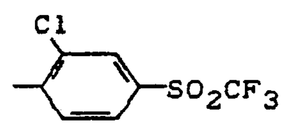 |
| 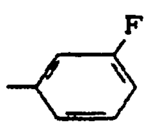 | 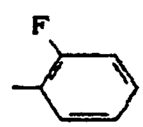 |
| 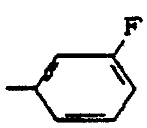 | 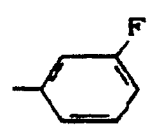 |
| 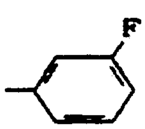 | 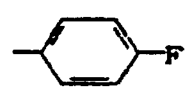 |

Tabelle 2 (Fortsetzung)

| $R^1$ | Ar |
|---|---|
| 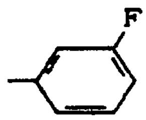 | 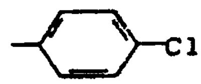 |
| 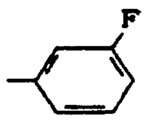 | 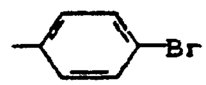 |
| 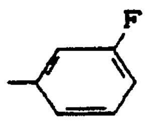 | 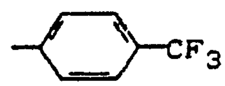 |
| 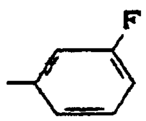 | 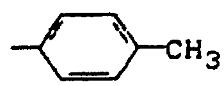 |
| 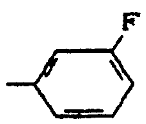 | 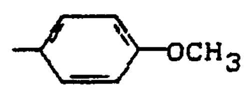 |
| 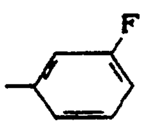 | 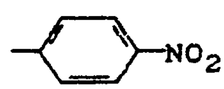 |
| 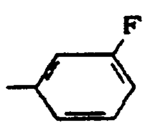 | 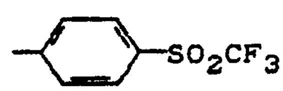 |
| 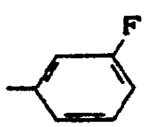 | 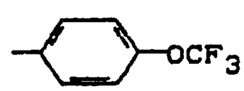 |

Tabelle 2 (Fortsetzung)

| $R^1$ | Ar |
|---|---|
| 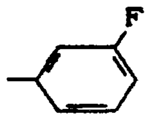 | 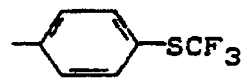 |
| 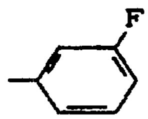 | 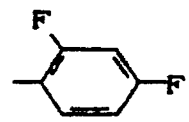 |
| 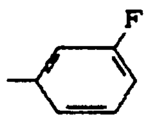 | 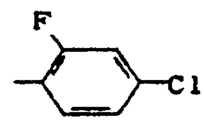 |
| 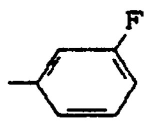 | 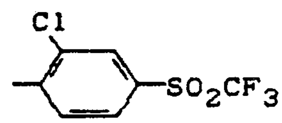 |
| 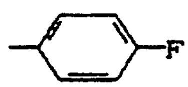 | 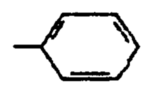 |
| 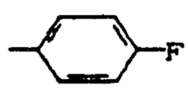 | 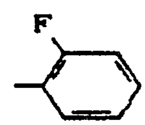 |
| 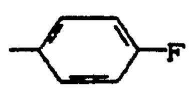 | 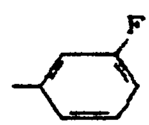 |
| 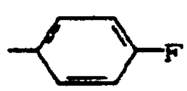 | 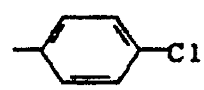 |
| 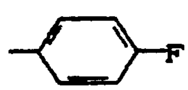 | 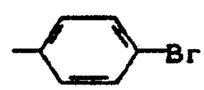 |

Tabelle 2 (Fortsetzung)

| $R^1$ | Ar |
|---|---|
| 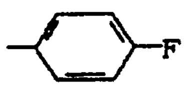 | 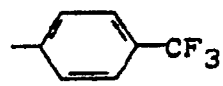 |
| 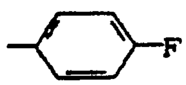 | 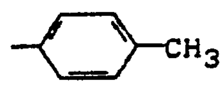 |
| 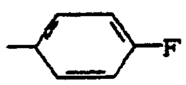 | 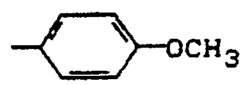 |
| 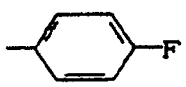 | 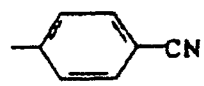 |
| 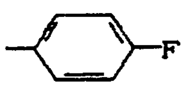 | 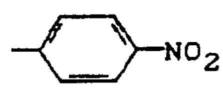 |
| 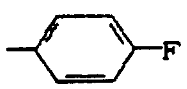 | 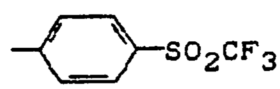 |
| 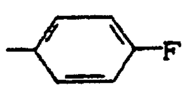 | 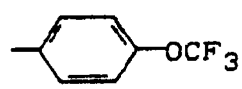 |
| 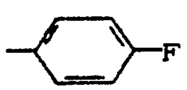 | 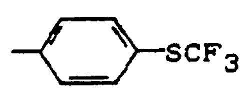 |
| 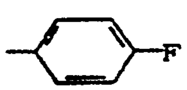 | 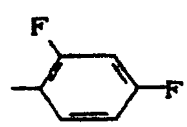 |
| 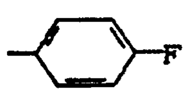 | 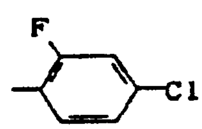 |

Tabelle 2 (Fortsetzung)

| $R^1$ | Ar |
|---|---|
| 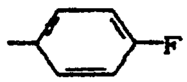 | 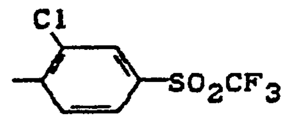 |
| 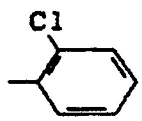 | 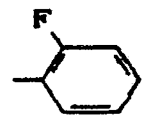 |
| 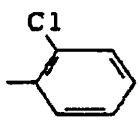 | 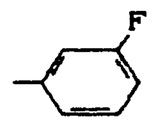 |
| 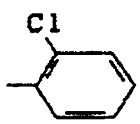 | 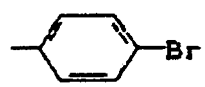 |
| 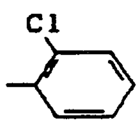 | 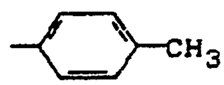 |
| 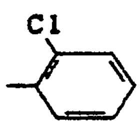 | 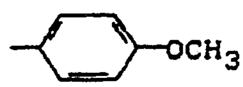 |
| 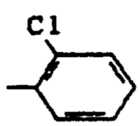 | 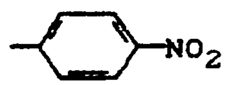 |
| 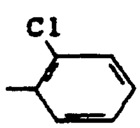 | 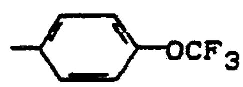 |

Tabelle 2 (Fortsetzung)

| $R^1$ | Ar |
|---|---|
| 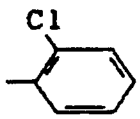 | 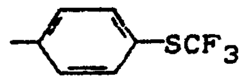 |
| 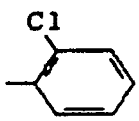 | 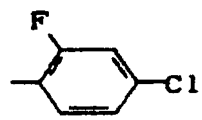 |
| 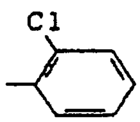 | 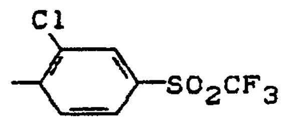 |
| 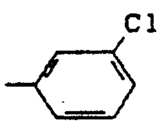 | 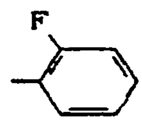 |
| 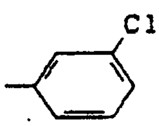 | 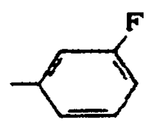 |
| 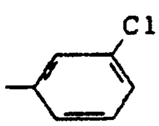 | 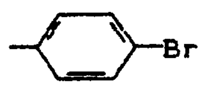 |
| 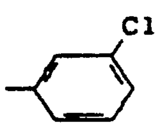 | 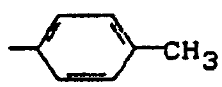 |
| 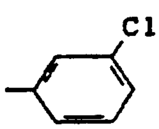 | 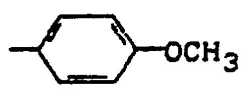 |

<u>Tabelle 2</u> (Fortsetzung)

| $R^1$ | Ar |
|---|---|
| 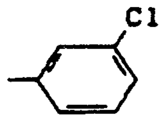 | 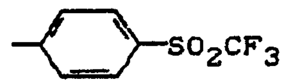 |
| 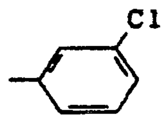 | 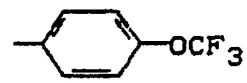 |
| 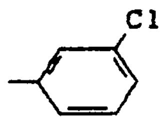 | 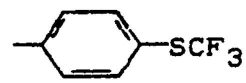 |
| 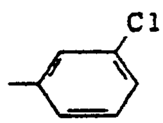 | 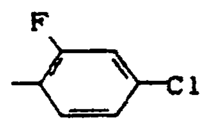 |
| 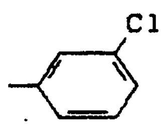 | 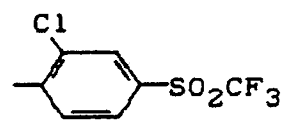 |
| 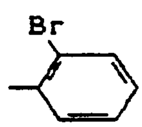 | 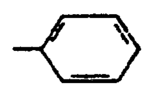 |
| 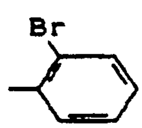 | 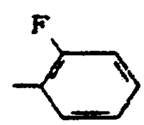 |
| 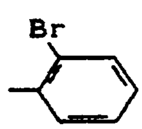 | 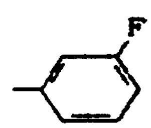 |

Tabelle 2 (Fortsetzung)

| $R^1$ | Ar |
| --- | --- |
| 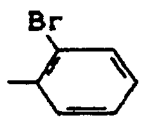 | 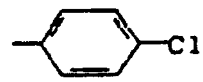 |
| 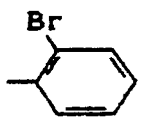 | 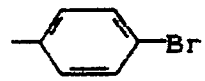 |
| 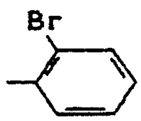 | 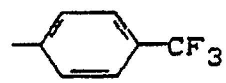 |
| 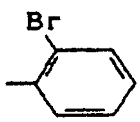 | 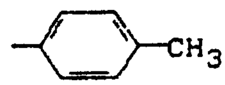 |
| 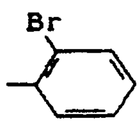 | 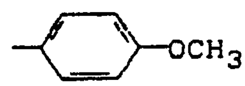 |
| 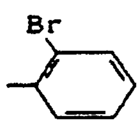 | 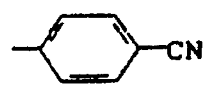 |
| 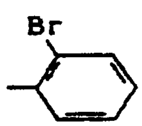 | 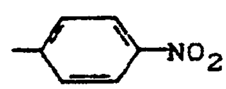 |
| 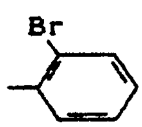 | 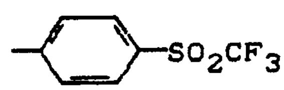 |

Tabelle 2 (Fortsetzung)

| $R^1$ | Ar |
|---|---|
| 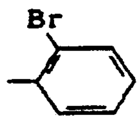 | 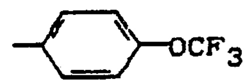 |
| 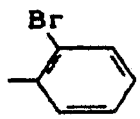 | 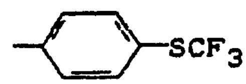 |
| 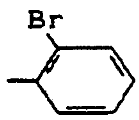 | 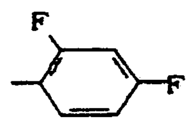 |
| 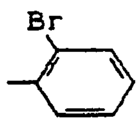 | 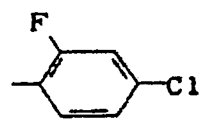 |
| 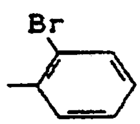 | 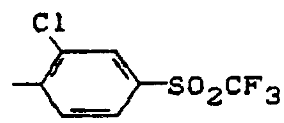 |
| 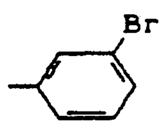 | 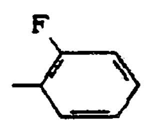 |
| 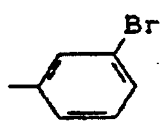 | 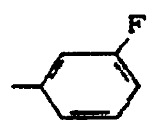 |
| 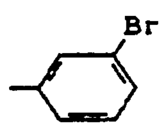 | 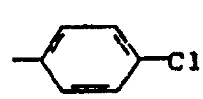 |

Tabelle 2 (Fortsetzung)

| $R^1$ | Ar |
|---|---|
| 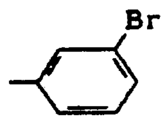 | 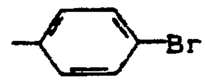 |
| 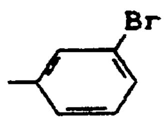 | 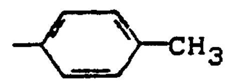 |
| 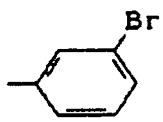 | 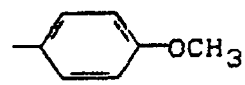 |
| 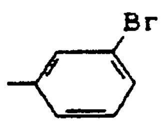 | 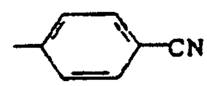 |
| 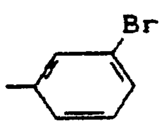 | 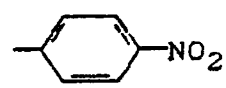 |
| 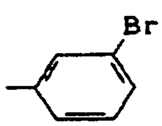 | 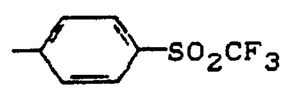 |
| 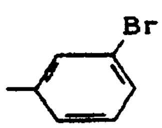 | 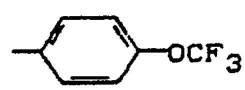 |
| 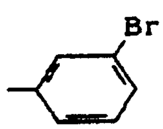 | 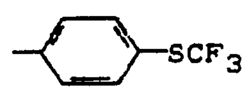 |

<u>Tabelle 2</u> (Fortsetzung)

| $R^1$ | Ar |
|---|---|
| 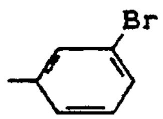 | 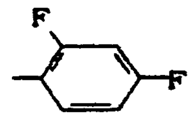 |
| 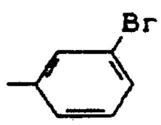 | 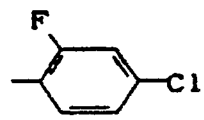 |
| 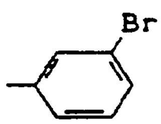 | 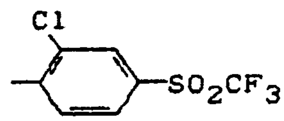 |
| 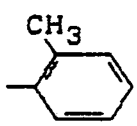 | 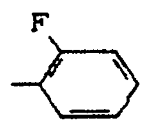 |
| 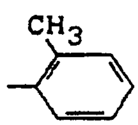 | 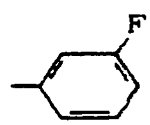 |
| 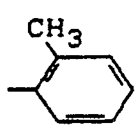 | 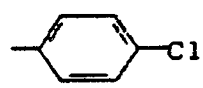 |
| 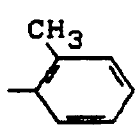 | 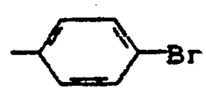 |
| 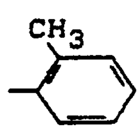 | 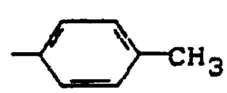 |

Tabelle 2 (Fortsetzung)

| $R^1$ | Ar |
|---|---|
| 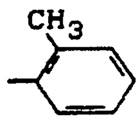 | 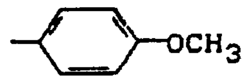 |
| 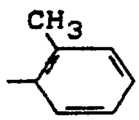 | 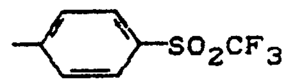 |
| 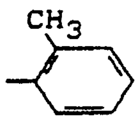 | 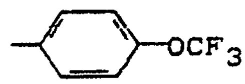 |
| 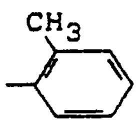 | 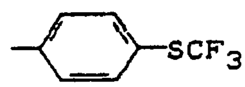 |
| 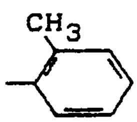 | 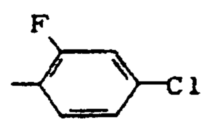 |
| 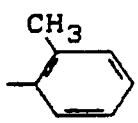 | 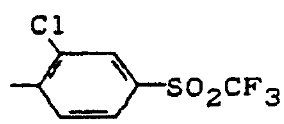 |
| 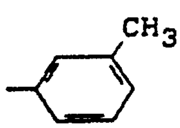 | 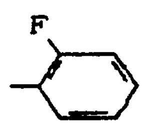 |
| 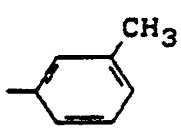 | 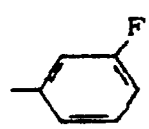 |

Tabelle 2 (Fortsetzung)

| $R^1$ | Ar |
|---|---|
| 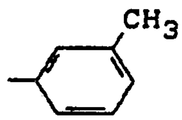 | 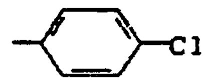 |
| 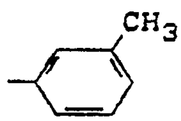 | 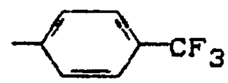 |
| 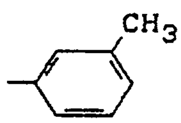 | 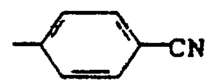 |
| 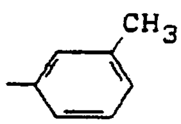 | 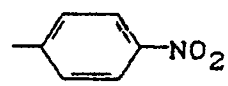 |
| 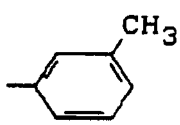 | 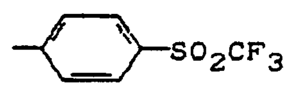 |
| 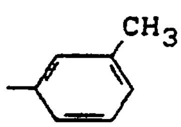 | 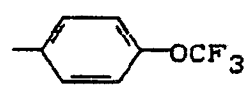 |
| 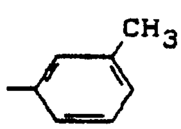 | 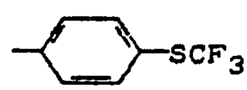 |
| 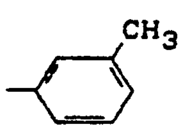 | 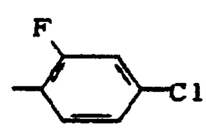 |

Tabelle 2 (Fortsetzung)

| $R^1$ | Ar |
|---|---|
| 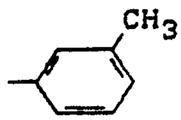 | 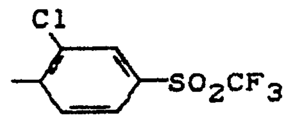 |
| 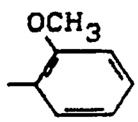 | 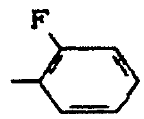 |
| 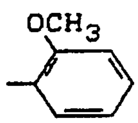 | 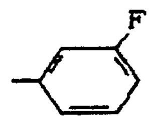 |
| 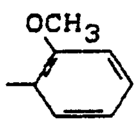 | 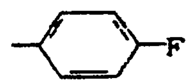 |
| 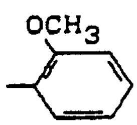 | 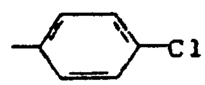 |
| 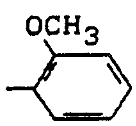 | 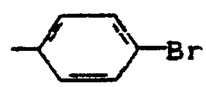 |
| 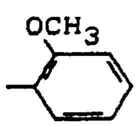 | 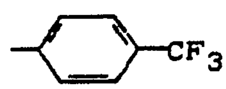 |
| 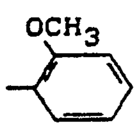 | 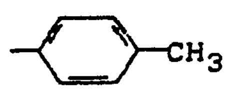 |

<u>Tabelle 2</u> (Fortsetzung)

| $R^1$ | Ar |
|---|---|
| 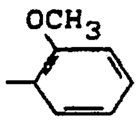 | 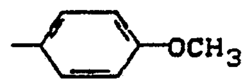 |
| 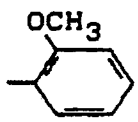 | 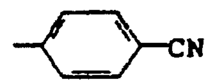 |
| 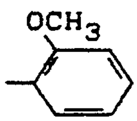 | 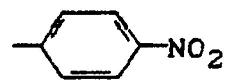 |
| 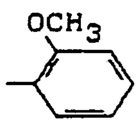 | 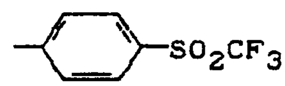 |
| 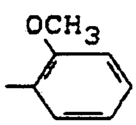 | 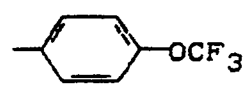 |
| 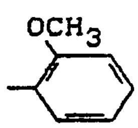 | 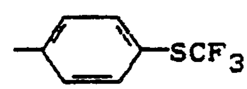 |
| 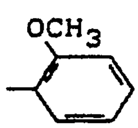 | 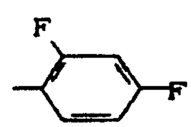 |
| 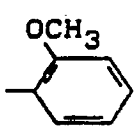 | 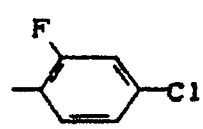 |

Tabelle 2 (Fortsetzung)

| $R^1$ | Ar |
|---|---|
| 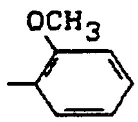 | 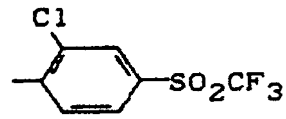 |
| 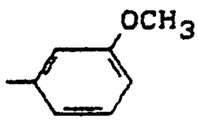 | 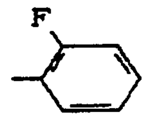 |
| 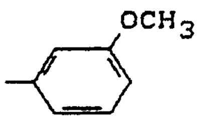 | 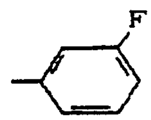 |
| 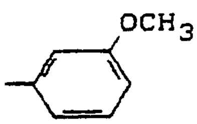 | 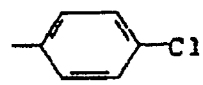 |
| 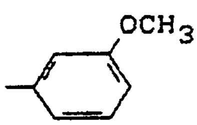 | 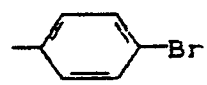 |
| 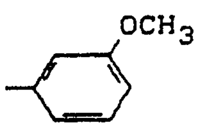 | 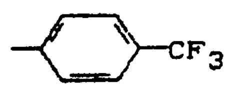 |
| 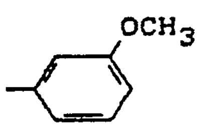 | 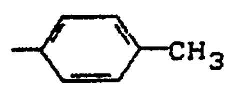 |
| 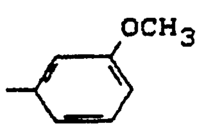 | 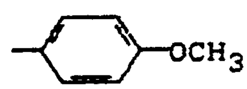 |

Tabelle 2 (Fortsetzung)

| $R^1$ | Ar |
|---|---|
| 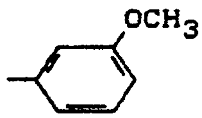 | 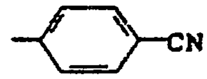 |
| 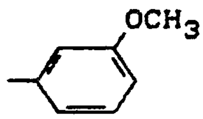 | 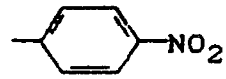 |
| 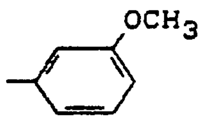 | 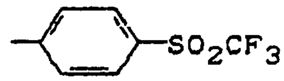 |
| 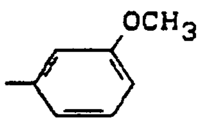 | 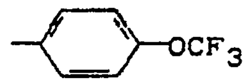 |
| 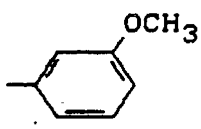 | 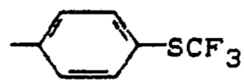 |
| 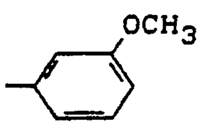 | 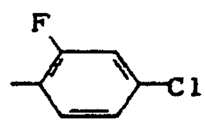 |
| 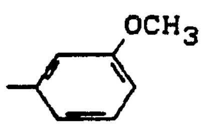 | 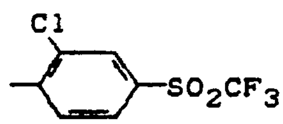 |
| 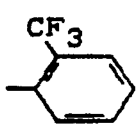 | 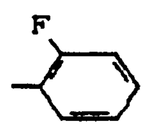 |

Tabelle 2 (Fortsetzung)

| $R^1$ | Ar |
|---|---|
| 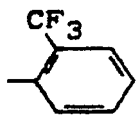 | 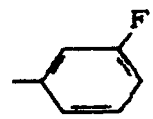 |
| 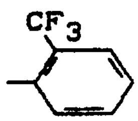 | 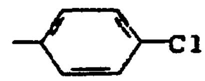 |
| 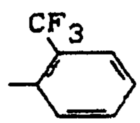 | 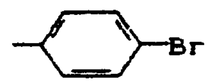 |
| 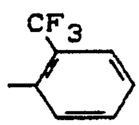 | 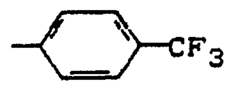 |
| 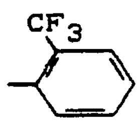 | 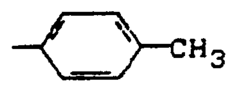 |
| 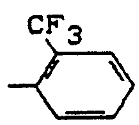 | 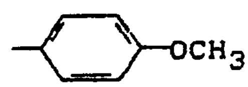 |
| 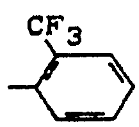 | 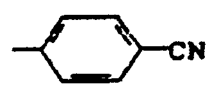 |
| 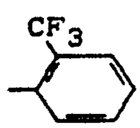 | 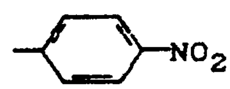 |

Tabelle 2 (Fortsetzung)

| $R^1$ | Ar |
|---|---|
| 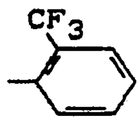 | 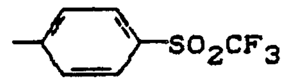 |
| 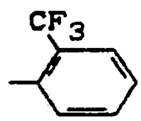 | 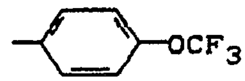 |
| 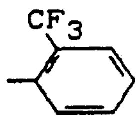 | 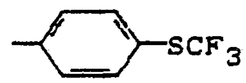 |
| 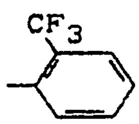 | 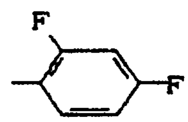 |
| 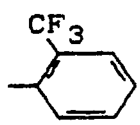 | 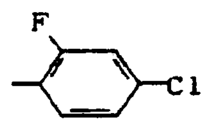 |
| 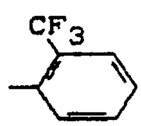 | 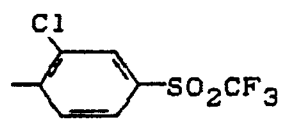 |
| 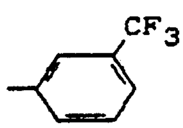 | 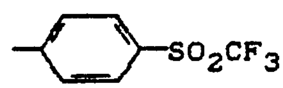 |
| 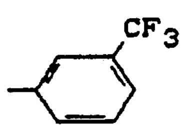 | 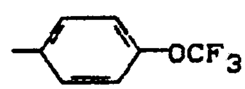 |

Tabelle 2 (Fortsetzung)

| $R^1$ | Ar |
|---|---|
| 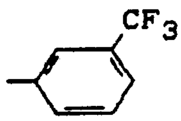 | 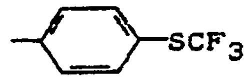 |
| 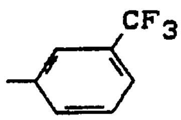 | 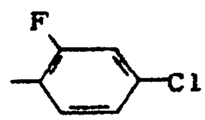 |
| 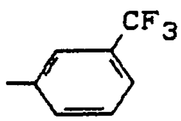 | 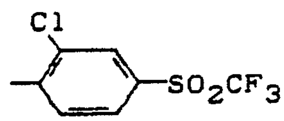 |
| 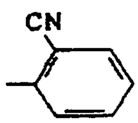 | 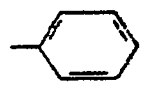 |
| 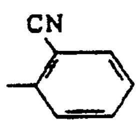 | 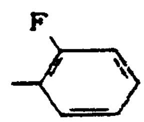 |
| 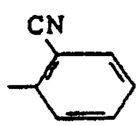 | 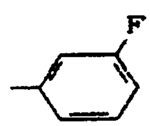 |
| 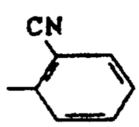 | 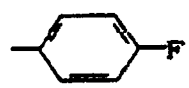 |
| 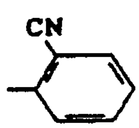 | 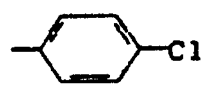 |

<u>Tabelle 2</u> (Fortsetzung)

| $R^1$ | Ar |
|---|---|
| 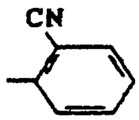 | 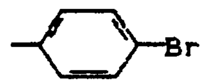 |
| 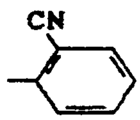 | 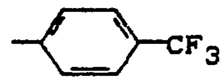 |
| 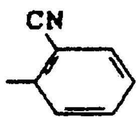 | 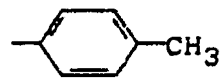 |
| 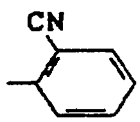 | 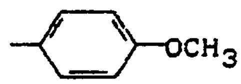 |
| 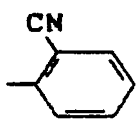 | 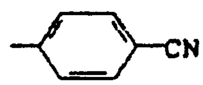 |
| 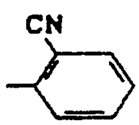 | 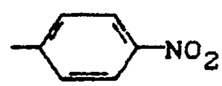 |
| 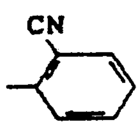 | 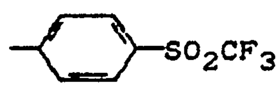 |
| 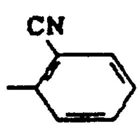 | 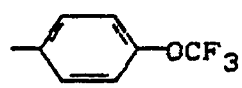 |

Tabelle 2 (Fortsetzung)

| $R^1$ | Ar |
|---|---|
| 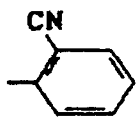 | 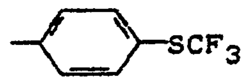 |
| 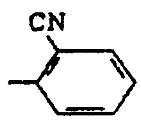 | 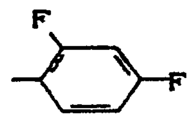 |
| 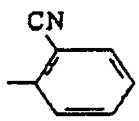 | 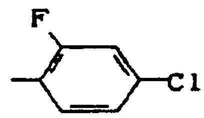 |
| 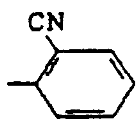 | 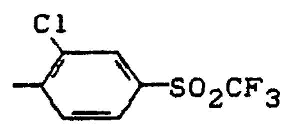 |
| 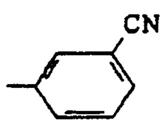 | 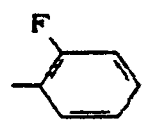 |
| 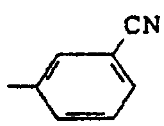 | 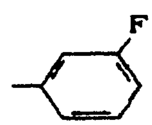 |
| 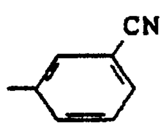 | 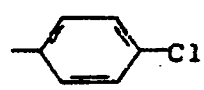 |
| 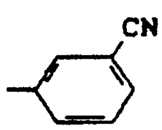 | 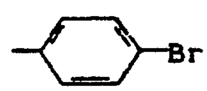 |

Tabelle 2 (Fortsetzung)

| $R^1$ | Ar |
|---|---|
| 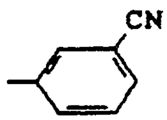 | 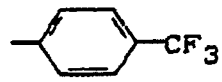 |
| 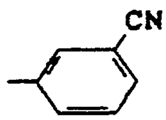 | 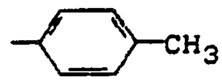 |
| 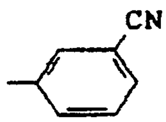 | 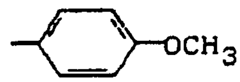 |
| 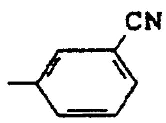 | 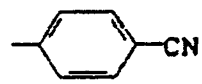 |
| 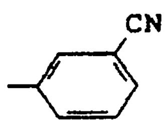 | 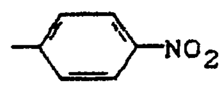 |
| 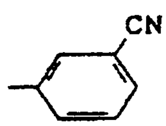 | 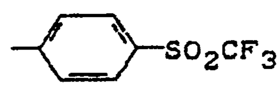 |
| 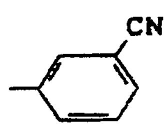 | 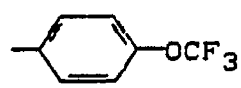 |
| 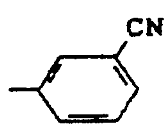 | 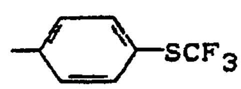 |

Tabelle 2 (Fortsetzung)

| $R^1$ | Ar |
|---|---|
| 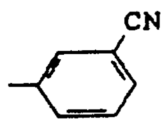 | 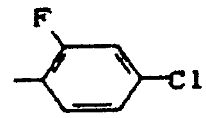 |
| 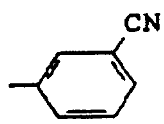 | 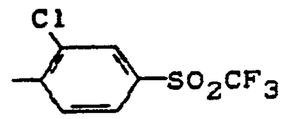 |
| 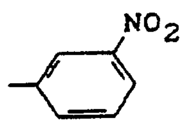 | 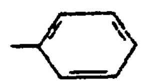 |
| 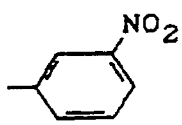 | 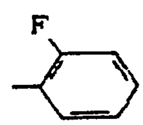 |
| 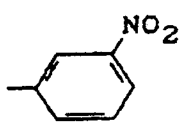 | 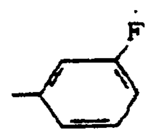 |
| 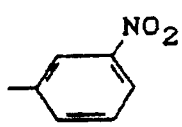 | 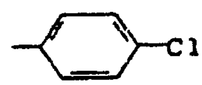 |
| 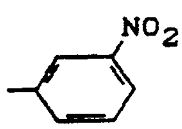 | 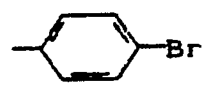 |
| 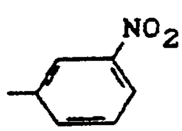 | 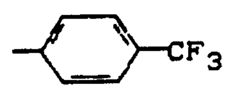 |

Tabelle 2 (Fortsetzung)

| $R^1$ | Ar |
|---|---|
| 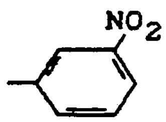 | 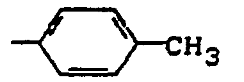 |
| 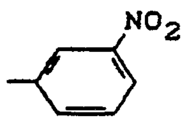 | 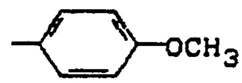 |
| 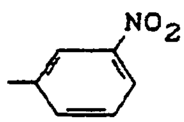 | 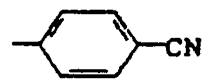 |
| 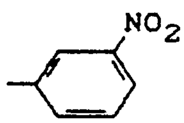 | 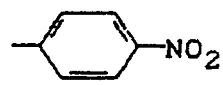 |
| 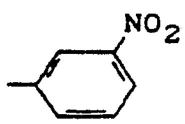 | 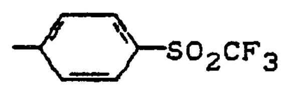 |
| 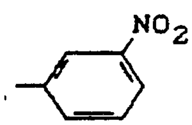 | 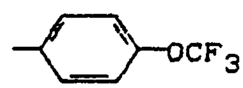 |
| 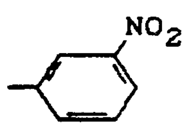 | 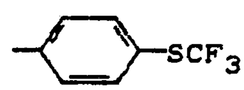 |
| 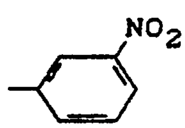 | 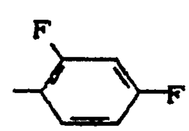 |

Tabelle 2 (Fortsetzung)

| $R^1$ | Ar |
|---|---|
| 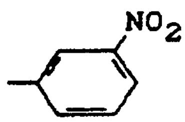 | 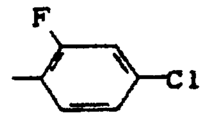 |
| 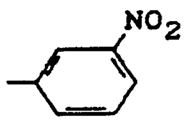 | 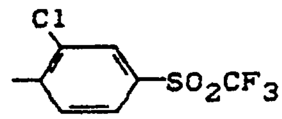 |
| 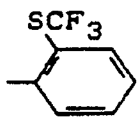 | 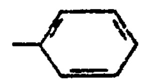 |
| 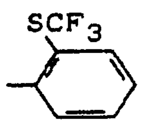 | 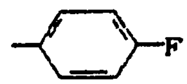 |
| 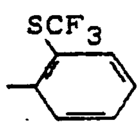 | 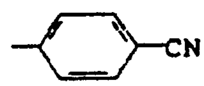 |
| 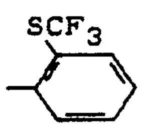 | 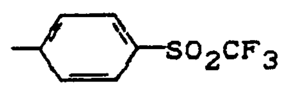 |
| 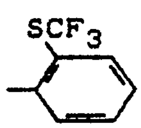 | 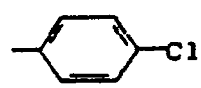 |
| 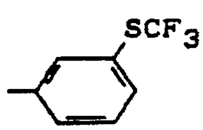 | 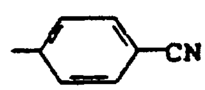 |

<u>Tabelle 2</u> (Fortsetzung)

| $R^1$ | Ar |
|---|---|
| 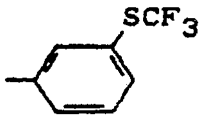 | 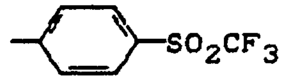 |
| 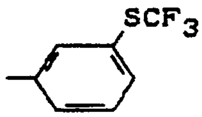 | 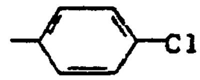 |
| 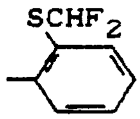 | 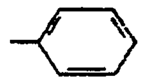 |
| 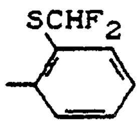 | 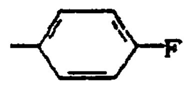 |
| 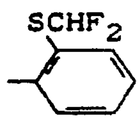 | 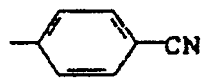 |
| 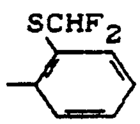 | 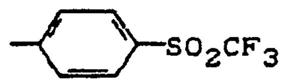 |
| 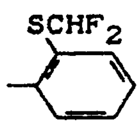 | 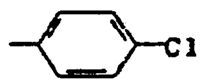 |
| 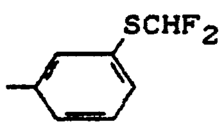 | 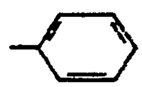 |

Tabelle 2 (Fortsetzung)

| $R^1$ | Ar |
|---|---|
| 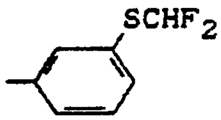 | 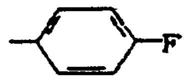 |
| 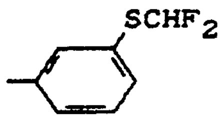 | 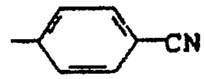 |
| 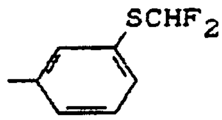 | 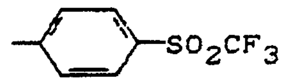 |
| 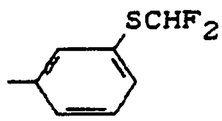 | 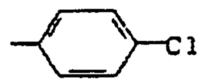 |
| 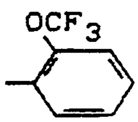 | 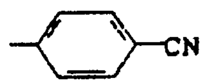 |
| 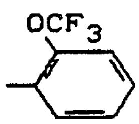 | 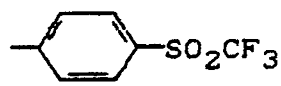 |
| 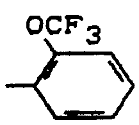 | 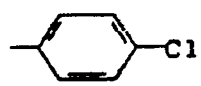 |
| 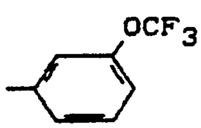 | 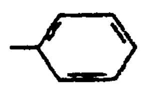 |

Tabelle 2 (Fortsetzung)

| $R^1$ | Ar |
|---|---|
| 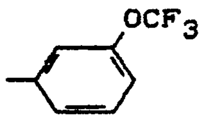 | 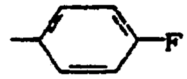 |
| 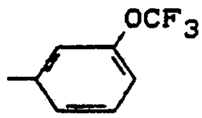 | 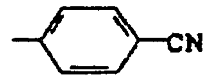 |
| 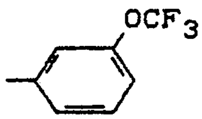 | 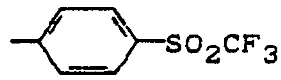 |
| 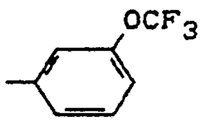 | 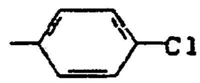 |
| 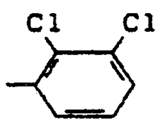 | 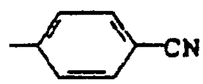 |
| 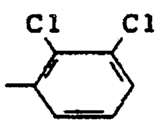 | 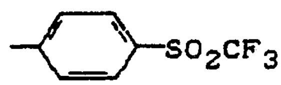 |
| 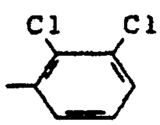 | 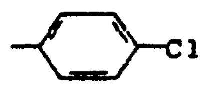 |
| 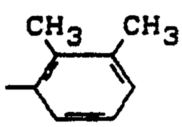 | 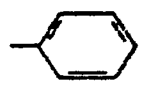 |

Tabelle 2 (Fortsetzung)

| $R^1$ | Ar |
|---|---|
| 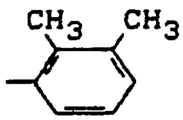 | 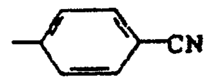 |
| 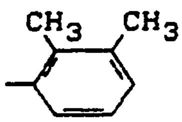 | 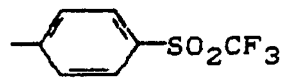 |
| 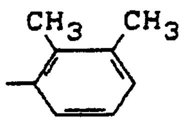 | 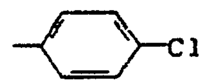 |
| 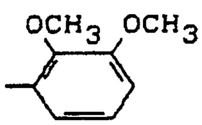 | 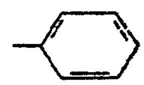 |
| 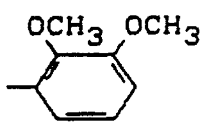 | 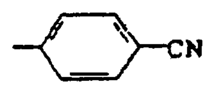 |
| 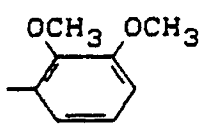 | 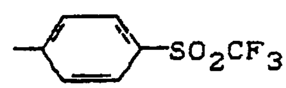 |
| 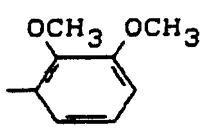 | 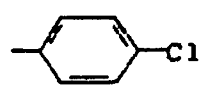 |
| 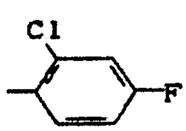 | 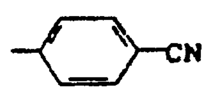 |

<u>Tabelle 2</u> (Fortsetzung)

| $R^1$ | Ar |
|---|---|
| 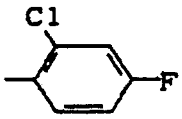 | 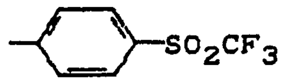 |
| 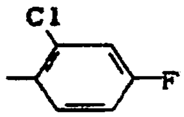 | 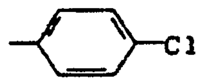 |
| 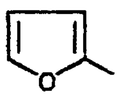 | 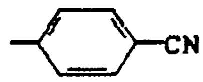 |
| 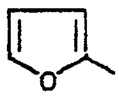 | 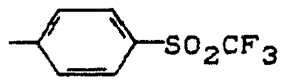 |
| 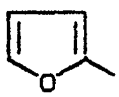 | 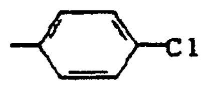 |
| 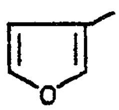 | 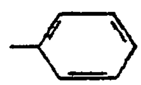 |
| 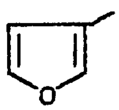 | 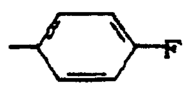 |
| 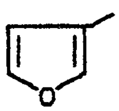 | 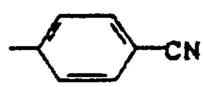 |
| 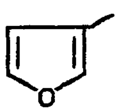 | 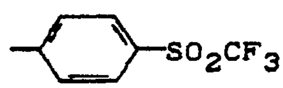 |

Tabelle 2 (Fortsetzung)

| $R^1$ | Ar |
|---|---|
| 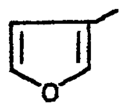 | 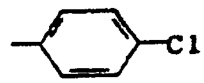 |
| 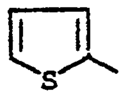 | 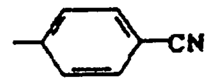 |
| 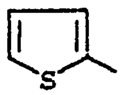 | 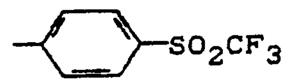 |
| 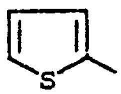 | 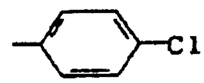 |
| 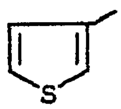 | 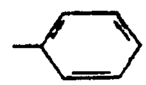 |
| 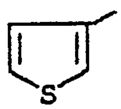 | 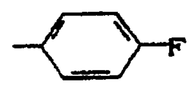 |
| 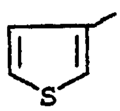 | 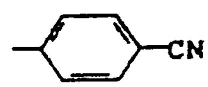 |
| 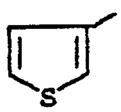 | 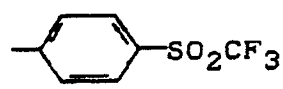 |
| 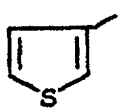 | 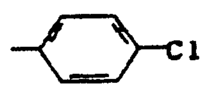 |
| 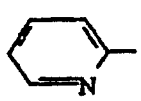 | 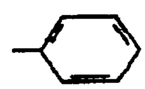 |

Tabelle 2 (Fortsetzung)

| $R^1$ | Ar |
|---|---|
| 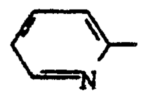 | 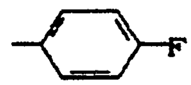 |
| 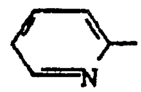 | 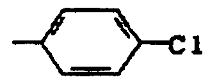 |
| 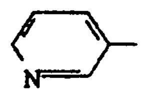 | 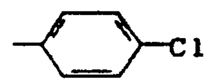 |
| 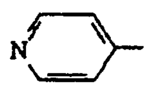 | 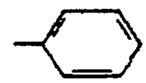 |
| 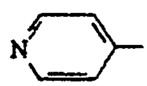 | 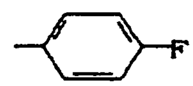 |
| 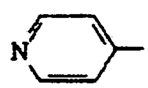 | 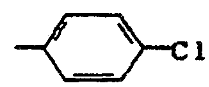 |
| 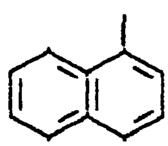 | 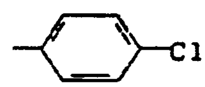 |
| 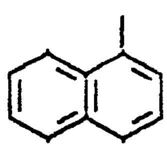 | 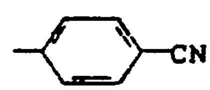 |
| 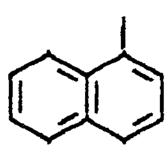 | 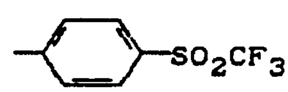 |

Tabelle 2 (Fortsetzung)

| $R^1$ | Ar |
|---|---|
| 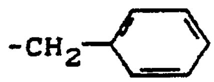 | 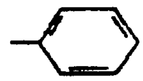 |
| 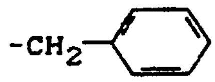 | 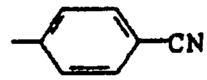 |
| 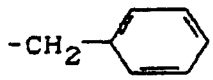 | 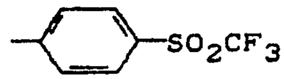 |
| 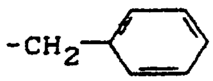 | 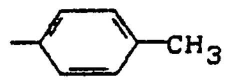 |
| 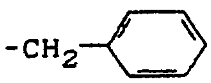 | 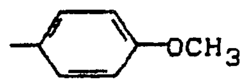 |
| 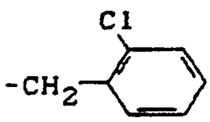 | 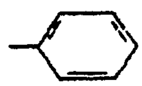 |
| 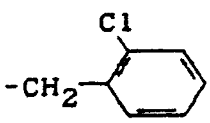 | 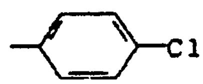 |
| 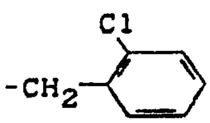 | 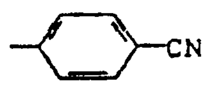 |
| 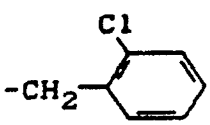 | 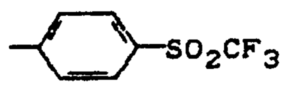 |

Tabelle 2 (Fortsetzung)

| $R^1$ | Ar |
|---|---|
| 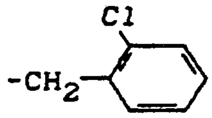 | 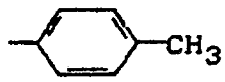 |
| 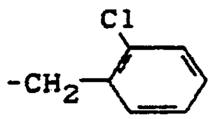 | 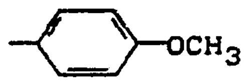 |
| 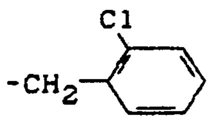 | 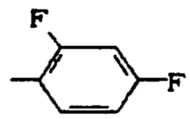 |
| 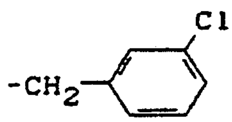 | 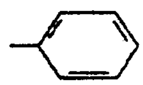 |
| 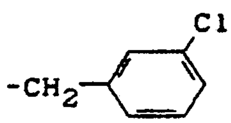 | 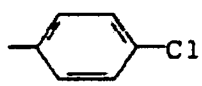 |
| 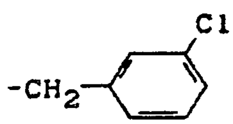 | 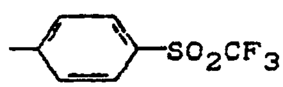 |
| 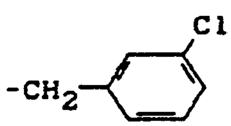 | 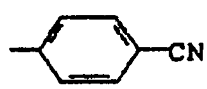 |
| 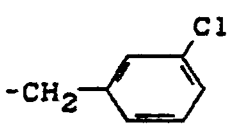 | 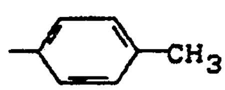 |

Tabelle 2 (Fortsetzung)

| $R^1$ | Ar |
|---|---|
| 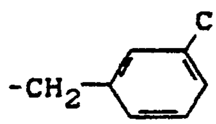 | 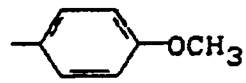 |
| 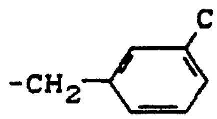 | 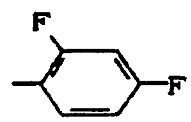 |
| 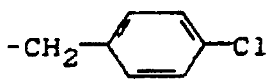 | 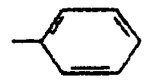 |
| 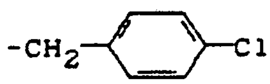 | 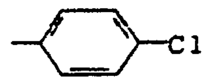 |
| 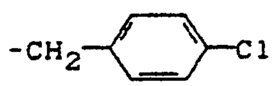 | 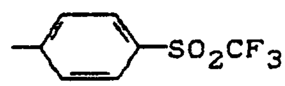 |
| 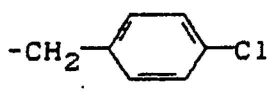 | 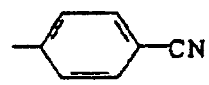 |
| 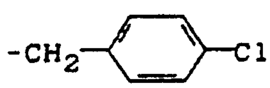 | 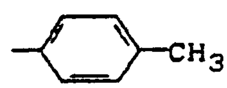 |
| 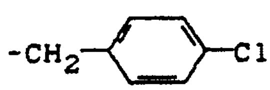 | 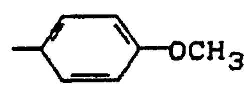 |
| 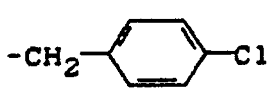 | 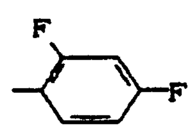 |
| 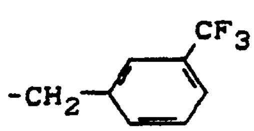 | 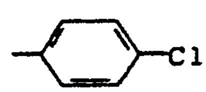 |

Tabelle 2 (Fortsetzung)

| $R^1$ | Ar |
|---|---|
| 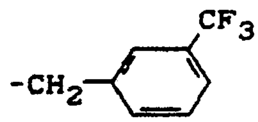 | 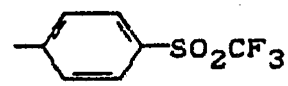 |
| 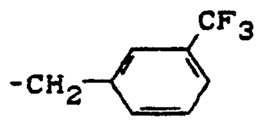 | 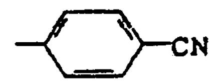 |
| 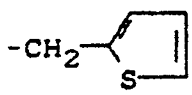 | 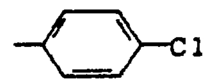 |
| 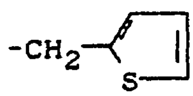 | 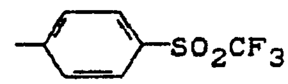 |
| 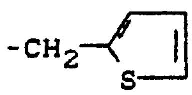 | 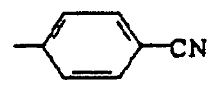 |
| 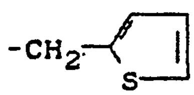 | 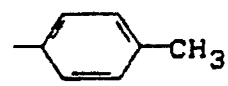 |
| 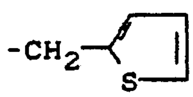 | 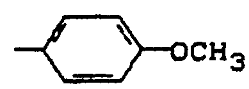 |
| 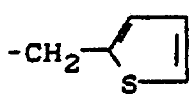 | 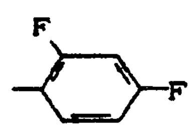 |
| 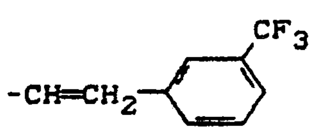 | 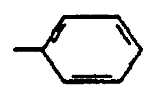 |

<u>Tabelle 2</u> (Fortsetzung)

| $R^1$ | Ar |
|---|---|
| 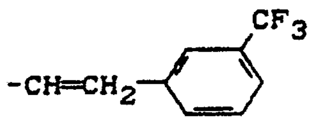 | 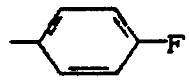 |
| 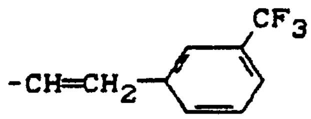 | 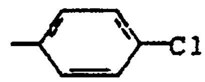 |
| 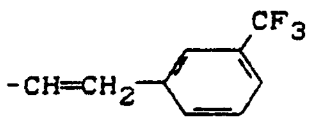 | 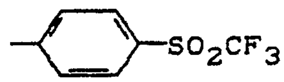 |
| 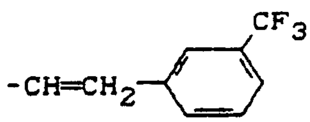 | 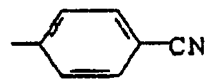 |
| 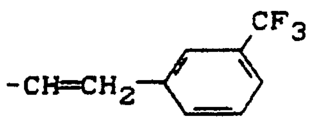 | 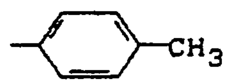 |
| 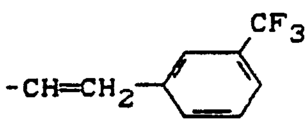 | 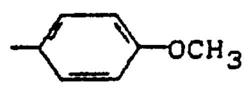 |
| 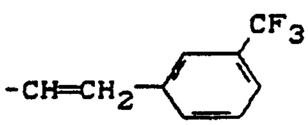 | 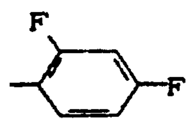 |
| 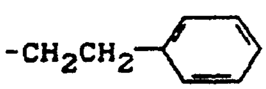 | 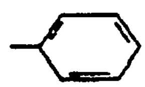 |
| 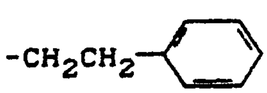 | 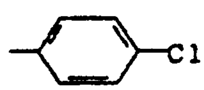 |

Tabelle 2 (Fortsetzung)

| $R^1$ | Ar |
|---|---|
| 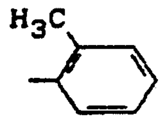 | 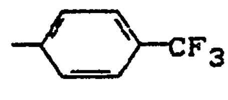 |

Die Dimethylaminomethyliden-pyrazolin-5-on-Derivate der Formel (II) sind bekannt und/oder können nach bekannten Verfahren hergestellt werden (vgl. EP 0 274 642).

In Formel (III) hat $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ angegeben wurde und

HX steht für das Äquivalent einer Mineralsäure wie z.B. Chlorwasserstoffsäure oder einer Carbonsäure wie z.B. Oxalsäure, vorzugsweise für Chlorwasserstoffsäure.

Die Hydroxylamin-hydrochlorid-Derivate der Formel (III) sind allgemein bekannte Verbindungen der organischen Chemie.

Das erfindungsgemäße Verfahren zur Herstellung der neuen Pyrazolin-5-on-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Gly koldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen 0 °C und +100 °C, vorzugsweise bei Temperaturen zwischen +10 °C und +50 °C.

Zur Durchführung des erfindungsgemäßen Verfahrens setzt man pro Mol an Dimethylaminomethyliden-pyrazolin-5-on-Derivat der Formel (II) im allgemeinen 1 bis 3 Mol, vorzugsweise 1 bis 1,5 Mol an Hydroxylamin-hydrochlorid-Derivat der Formel (III) ein. Die Aufarbeitung und Isolierung der Reaktionsprodukte erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffs als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B.

auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei eignen sich die erfindungsgemäßen Wirkstoffe besonders gut zur selektiven Bekämpfung von Unkräutern in annuellen Kulturen im Vor- und Nachauflaufverfahren.

Bei Anwendung im Nachauflaufverfahren können die erfindungsgemäßen Wirkstoffe alleine oder in Kombination mit emulgierbaren Ölen, oberflächenaktiven Substanzen und weiteren Additiven ausgebracht werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); 2-Chlor-N-{[(4-

methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); Methyl-2-[4,5-dihydro-4-methyl-4-(1-methylethyl)-5-oxo-1H-imidazol-2-yl]-4(5)-methylbenzoat(IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenylpyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üb licher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,01 und 5 kg pro ha.

Die Herstellung und die Verwendung der erfindungsgemäßen Wirkstoffe geht aus den nachfolgenden Beispielen hervor.

Herstellungsbeispiel

Beispiel 1:

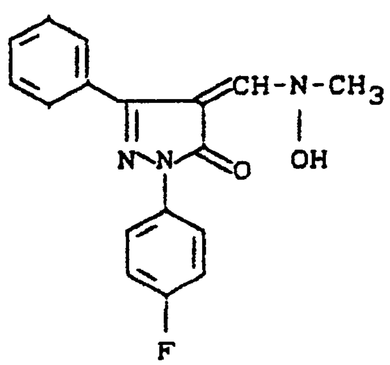

1,54 g (0,005 Mol) 1-(4-Fluorphenyl)-3-phenyl-4-(N,N-dimethylaminomethyliden)-2-pyrazolin-5-on werden in 50 ml Methanol gelöst, mit 0,41 g (0,005 Mol) N-Methylhydroxylamin-hydrochlorid versetzt und anschließend bis zum vollständigen Umsatz (chromatographische Kontrolle) bei Raumtemperatur gerührt. Anschließend wird die Reaktionslösung stark eingeengt, der erhaltene Feststoff mit Wasser aufgenommen und abgesaugt. Zur Reinigung wird der Feststoff mehrmals mit Wasser und zum Schluß mit Petrolether gewaschen.

Man erhält 1,3 g (83% der Theorie) an 1-(4-Fluorphenyl)-3-phenyl-4-(N-methyl-N-hydroxyaminomethyliden)-pyrazolin-5-on vom Schmelzpunkt 157 °C.

Analog zum Herstellungsbeispiel 1 und gemäß den allgemeinen Angaben zum erfindungsgemäßen Verfahren erhält man die in Tabelle 3 aufgeführten Pyrazolin-5-on-Derivate der allgemeinen Formel (I).

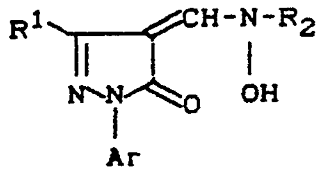

(I)

Tabelle 3

| Bsp.Nr. | $R^1$ | $R^2$ | Ar | Schmelzpunkt |
|---|---|---|---|---|
| 2 | $-C_3H_7-n$ | $CH_3$ | 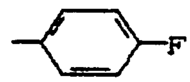 | 149° C |
| 3 | $-C_3H_7-n$ | $CH_3$ | 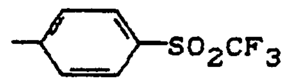 | 143° C |
| 4 | $-CH_2OCH_3$ | $CH_3$ | 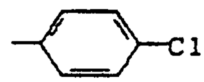 | 127-128° C |
| 5 | $-CH_2OCH_3$ | $CH_3$ | 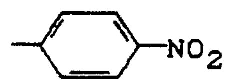 | 151° C |
| 6 | 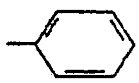 | $CH_3$ | 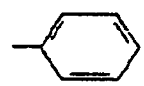 | 110° C |
| 7 | 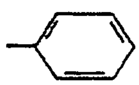 | $CH_3$ | 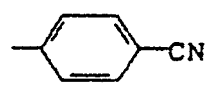 | 185° C |
| 8 | 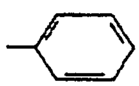 | $CH_3$ | 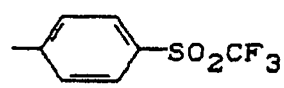 | 214-215° C |
| 9 | 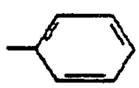 | $CH_3$ | 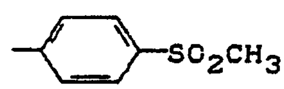 | 185° C |
| 10 | 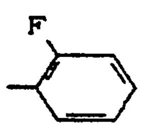 | $CH_3$ | 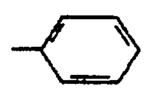 | 135-136° C |
| 11 | 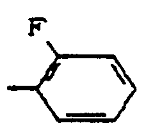 | $CH_3$ | 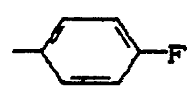 | 197-201° C |

Tabelle 3 (Fortsetzung)

| Bsp.Nr. | $R^1$ | $R^2$ | Ar | Schmelzpunkt |
|---|---|---|---|---|
| 12 | 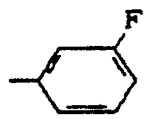 | $CH_3$ | 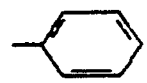 | 137-138° C |
| 13 | 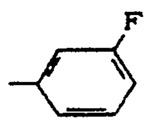 | $CH_3$ | 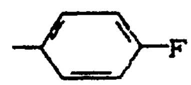 | 168-169° C |
| 14 | 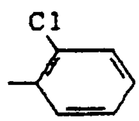 | $CH_3$ | 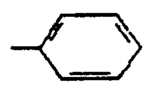 | 155-156° C |
| 15 | 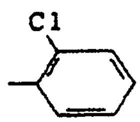 | $CH_3$ | 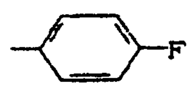 | 145-146° C |
| 16 | 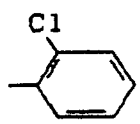 | $CH_3$ | 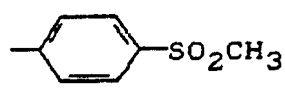 | 197-198° C |
| 17 | 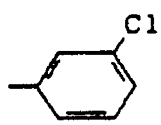 | $CH_3$ | 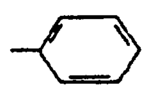 | 145-146° C |
| 18 | 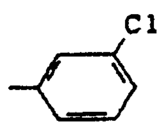 | $CH_3$ | 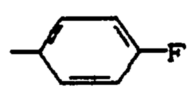 | 171° C |
| 19 | 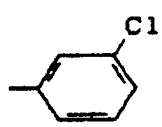 | $CH_3$ | 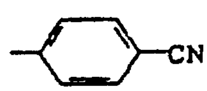 | 100° C |

Tabelle 3 (Fortsetzung)

| Bsp.Nr. | $R^1$ | $R^2$ | Ar | Schmelzpunkt |
|---|---|---|---|---|
| 20 | 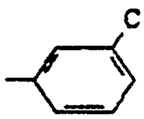 | $CH_3$ | 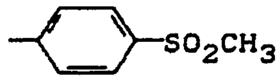 | 193-194° C |
| 21 | 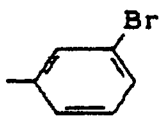 | $CH_3$ | 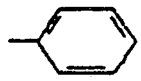 | 148° C |
| 22 | 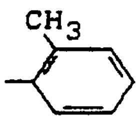 | $CH_3$ | 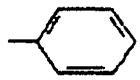 | 132° C |
| 23 | 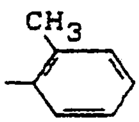 | $CH_3$ | 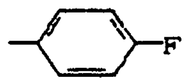 | 86-87° C |
| 24 | 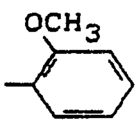 | $CH_3$ | 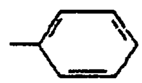 | 124-125° C |
| 25 | 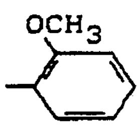 | $CH_3$ | 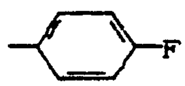 | 130-131° C |
| 26 | 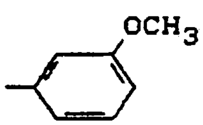 | $CH_3$ | 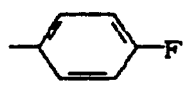 | 161-162° C |
| 27 | 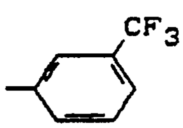 | $CH_3$ | 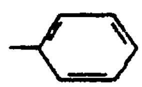 | 189-190° C |

Tabelle 3 (Fortsetzung)

| Bsp.Nr. | $R^1$ | $R^2$ | Ar | Schmelzpunkt |
|---|---|---|---|---|
| 28 | 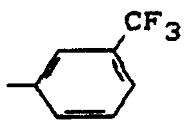 | $CH_3$ | 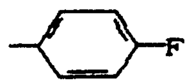 | 173-174° C |
| 29 | 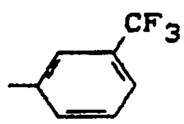 | $CH_3$ | 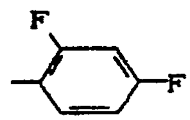 | 133-134° C |
| 30 | 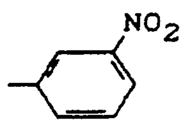 | $CH_3$ | 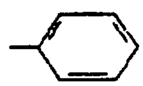 | 250-251° C |
| 31 | 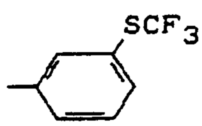 | $CH_3$ | 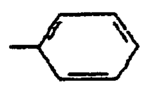 | 116° C |
| 32 | 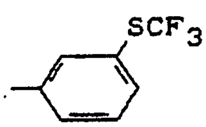 | $CH_3$ | 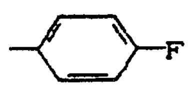 | 114° C |
| 33 | 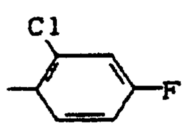 | $CH_3$ | 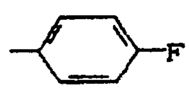 | 166-167° C |
| 34 | 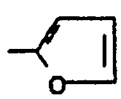 | $CH_3$ | 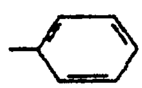 | 96° C |
| 35 | 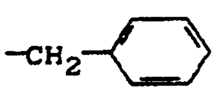 | $CH_3$ | 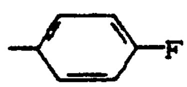 | 134° C |

<u>Tabelle 3</u> (Fortsetzung)

| Bsp.Nr. | $R^1$ | $R^2$ | Ar | Schmelz-punkt |
|---|---|---|---|---|
| 36 | 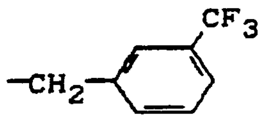 | $CH_3$ | 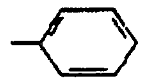 | 77-78° C |
| 37 | 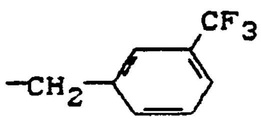 | $CH_3$ | 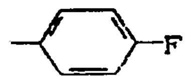 | 107° C |
| 38 | 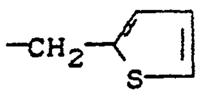 | $CH_3$ | 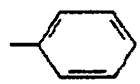 | 64-65° C |
| 39 | 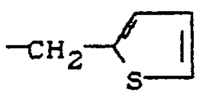 | $CH_3$ | 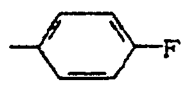 | 126-127° C |
| 40 | 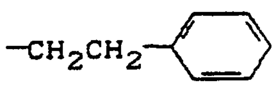 | $CH_3$ | 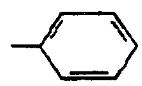 | 260° C |
| 41 | 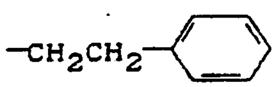 | $CH_3$ | 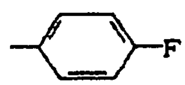 | 150° C |
| 42 | 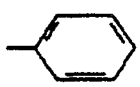 | $-CH_2CF_3$ | 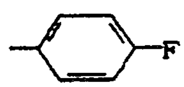 | 168° C |
| 43 | $-C_3H_7$-n | $CH_3$ | 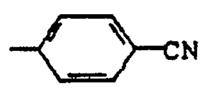 | 184-185° C |
| 44 | $-C_3H_7$-n | $CH_3$ | 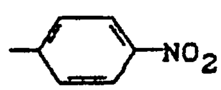 | 185-186° C |
| 45 | $-C_3H_7$-n | $CH_3$ | 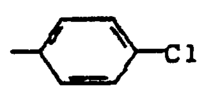 | 109-110° C |

Tabelle 3 (Fortsetzung)

| Bsp.Nr. | $R^1$ | $R^2$ | Ar | Schmelzpunkt |
|---|---|---|---|---|
| 46 | $-C_3H_7$-n | $CH_3$ | 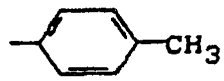 | 95-96° C |
| 47 | $-C_3H_7$-n | $CH_3$ | 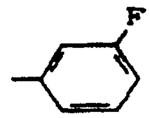 | 106-107° C |
| 48 | $-C_4H_9$-n | $CH_3$ | 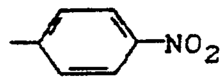 | 188-189° C |
| 49 | $-C_4H_9$-n | $CH_3$ | 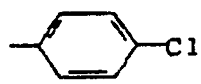 | 76-77° C |
| 50 | $-C_4H_9$-n | $CH_3$ | 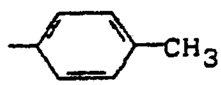 | 84-85° C |
| 51 | $-CH_2-$ 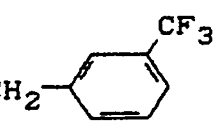 | $CH_3$ | 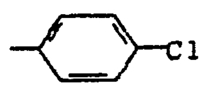 | 118-119° C |
| 52 | $-CH_2-$ 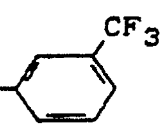 | $CH_3$ | 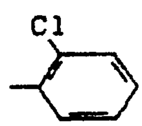 | 78-79° C |
| 53 | $-CH_2-$ 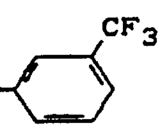 | $CH_3$ | 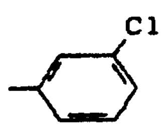 | 72-73° C |

Herstellung der Ausgangsverbindungen

Beispiel II-1

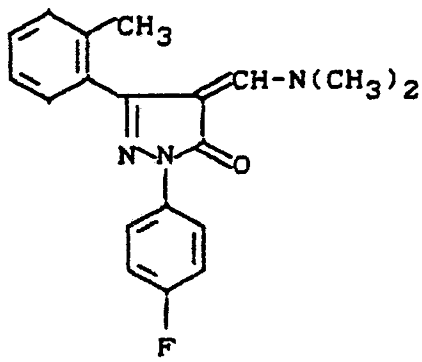

9,7 g (0,036 Mol) 1-(4-Fluorphenyl)-3-(2-methylphenyl)-2-pyrazolin-5-on werden in 40 ml Toluol gelöst und bei Raumtemperatur mit 5,7 g (0,047 Mol) N,N-Dimethylformamiddimethylacetal versetzt. Die Reaktionsmischung wird 16 Stunden bei Raumtemperatur gerührt und anschließend stark eingeengt. Durch Zusatz von Petrolether/Ether wird das Produkt kristallisiert.

Man erhält 9,8 g (84% der Theorie) an 4-(N,N-Dimethylaminomethyliden-1-(4-fluorphenyl)-3-(2-methylphenyl)-pyrazolin-5-on vom Schmelzpunkt 95 °C.

Analog zu Beispiel (II-1) und gemäß den allgemeinen Angaben zur Herstellung werden die in Tabelle 4 aufgeführten Ausgangsverbindungen der Formel (II) erhalten.

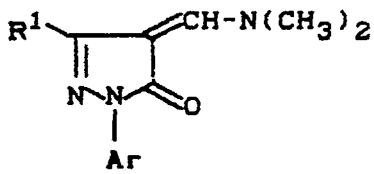

(II)

Tabelle 4

| Bsp.Nr. | $R^1$ | Ar | Schmelzpunkt |
|---|---|---|---|
| II-2 | $-C_3H_7-n$ | 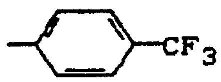 | 111-112° C |
| II-3 | 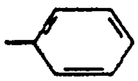 | 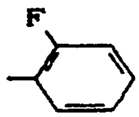 | 162° C |
| II-4 | 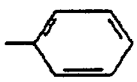 | 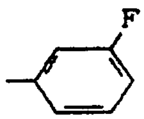 | 104° C |
| II-5 | 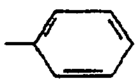 | 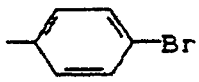 | 187° C |
| II-6 | 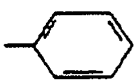 | 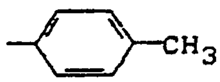 | 143° C |
| II-7 | 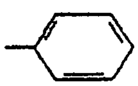 | 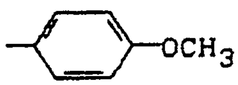 | 136° C |
| II-8 | 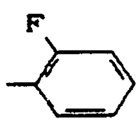 | 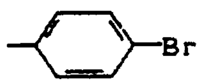 | 193° C |
| II-9 | 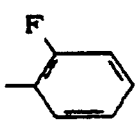 | 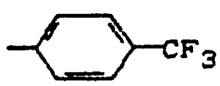 | 165° C |
| II-10 | 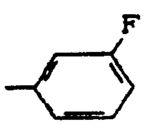 | 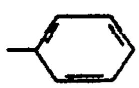 | 170° C |

<u>Tabelle 4</u> (Fortsetzung)

| Bsp.Nr. | $R^1$ | Ar | Schmelzpunkt |
|---|---|---|---|
| II-11 | 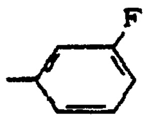 | 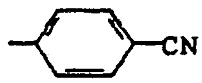 | 188° C |
| II-12 | 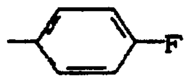 | 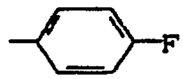 | 103° C |
| II-13 | 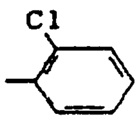 | 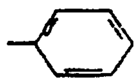 | 112-113° C |
| II-14 | 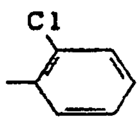 | 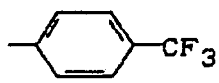 | 142° C |
| II-15 | 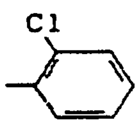 | 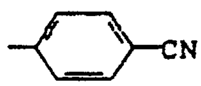 | 201° C |
| II-16 | 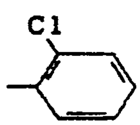 | 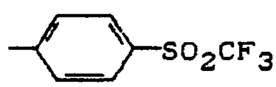 | 150-151° C |
| II-17 | 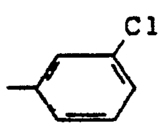 | 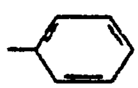 | 170° C |
| II-18 | 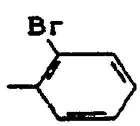 | 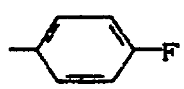 | 92-94° C |

<u>Tabelle 4</u> (Fortsetzung)

| Bsp.Nr. | $R^1$ | Ar | Schmelzpunkt |
|---|---|---|---|
| II-19 | 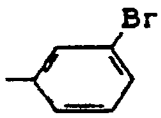 | 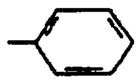 | 180° C |
| II-20 | 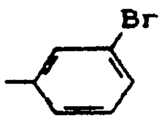 | 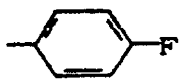 | 133° C |
| II-21 | 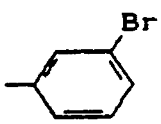 | 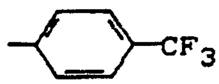 | 144° C |
| II-22 | 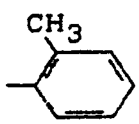 | 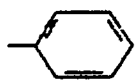 | 139° C |
| II-23 | 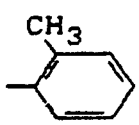 | 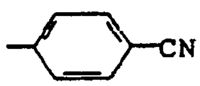 | 143-144° C |
| II-24 | 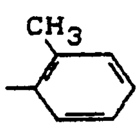 | 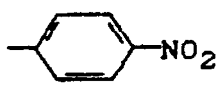 | 126-128° C |
| II-25 | 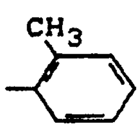 | 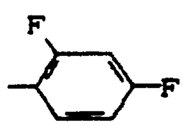 | 126-128° C |
| II-26 | 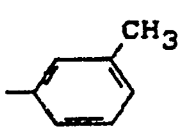 | 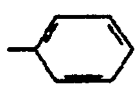 | 154° C |

Tabelle 4 (Fortsetzung)

| Bsp.Nr. | $R^1$ | Ar | Schmelzpunkt |
|---|---|---|---|
| II-27 | 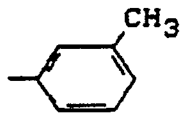 | 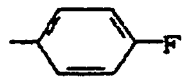 | 143-144° C |
| II-28 | 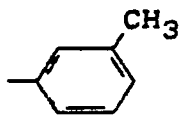 | 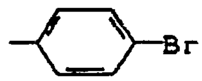 | 142° C |
| II-29 | 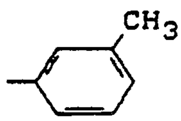 | 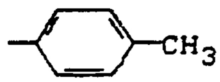 | 130° C |
| II-30 | 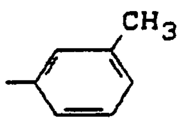 | 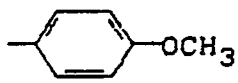 | 98-100° C |
| II-31 | 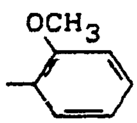 | 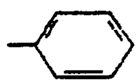 | 160° C |
| II-32 | 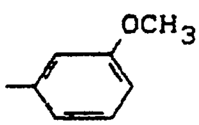 | 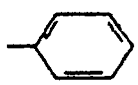 | 125° C |
| II-33 | 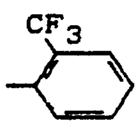 | 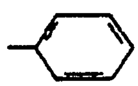 | 106° C |
| II-34 | 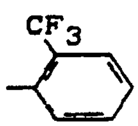 | 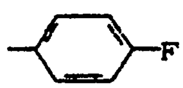 | 55° C |

<u>Tabelle 4</u> (Fortsetzung)

| Bsp.Nr. | $R^1$ | Ar | Schmelzpunkt |
|---|---|---|---|
| II-35 | 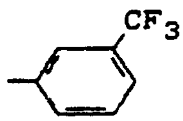 | 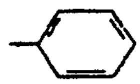 | 190° C |
| II-36 | 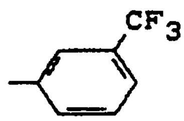 | 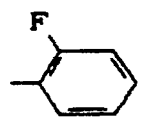 | 80° C |
| II-37 | 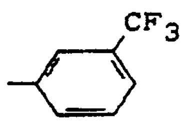 | 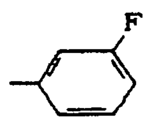 | 175° C |
| II-38 | 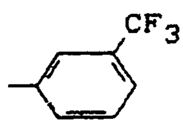 | 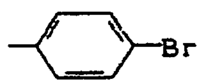 | 172° C |
| II-39 | 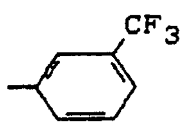 | 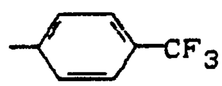 | 141° C |
| II-40 | 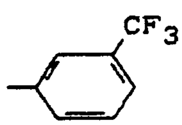 | 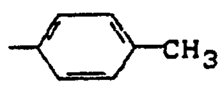 | 145° C |
| II-41 | 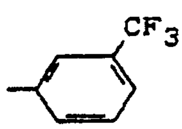 | 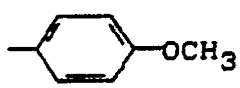 | 141° C |
| II-42 | 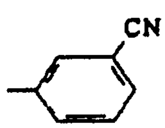 | 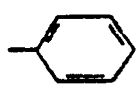 | 162-163° C |

Tabelle 4 (Fortsetzung)

| Bsp.Nr. | $R^1$ | Ar | Schmelzpunkt |
|---|---|---|---|
| II-43 | 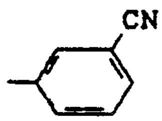 | 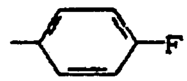 | 219° C |
| II-44 | 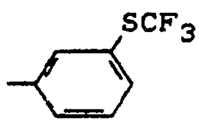 | 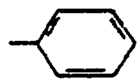 | 151° C |
| II-45 | 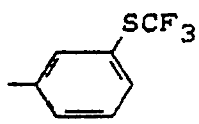 | 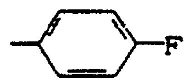 | 153° C |
| II-46 | 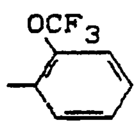 | 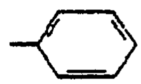 | 87-88° C |
| II-47 | 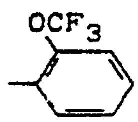 | 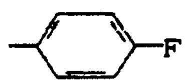 | 86-88° C |
| II-48 | 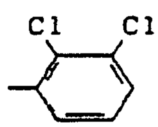 | 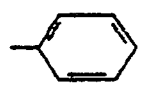 | 217° C |
| II-49 | 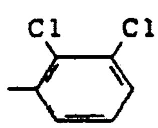 | 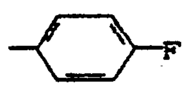 | 167° C |
| II-50 | 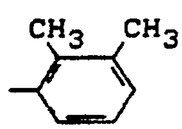 | 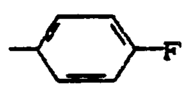 | 187° C |

Tabelle 4 (Fortsetzung)

| Bsp.Nr. | $R^1$ | Ar | Schmelzpunkt |
|---|---|---|---|
| II-51 | 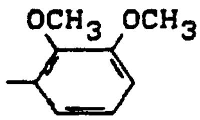 | 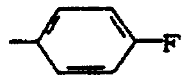 | 87° C |
| II-52 | 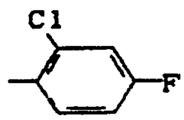 | 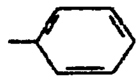 | 125° C |
| II-53 | 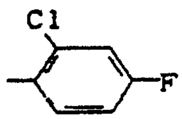 | 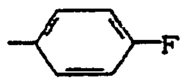 | 162° C |
| II-54 | 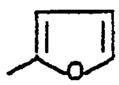 | 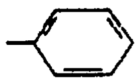 | 125° C |
| II-55 | 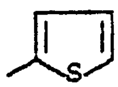 | 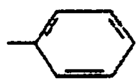 | 136-138° C |
| II-56 | 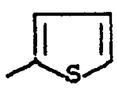 | 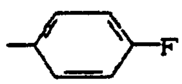 | 145-147° C |
| II-57 | 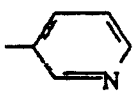 | 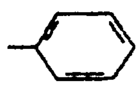 | 137-138° C |
| II-58 | 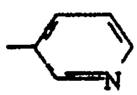 | 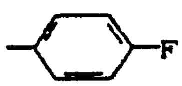 | 141° C |
| II-59 | 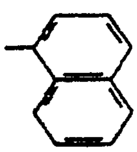 | 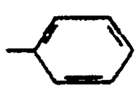 | 133° C |

Tabelle 4 (Fortsetzung)

| Bsp.Nr. | $R^1$ | Ar | Schmelzpunkt |
|---|---|---|---|
| II-60 | 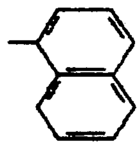 | 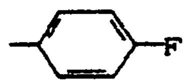 | 146° C |
| II-61 | 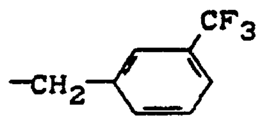 | 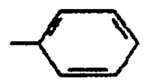 | 125° C |
| II-62 | 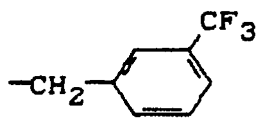 | 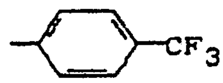 | 144° C |
| II-63 | 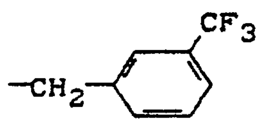 | 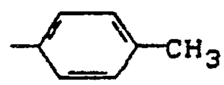 | 115° C |
| II-64 | 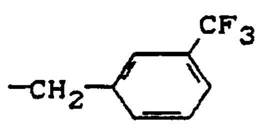 | 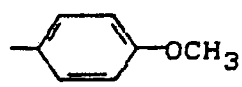 | 118° C |
| II-65 | 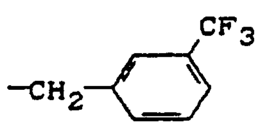 | 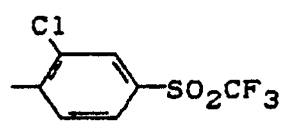 | 114° C |
| II-66 | 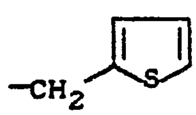 | 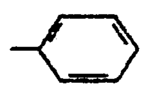 | 147° C |
| II-67 | 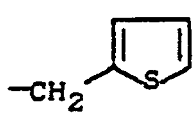 | 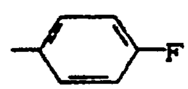 | 116-117° C |

Tabelle 4 (Fortsetzung)

| Bsp.Nr. | $R^1$ | Ar | Schmelzpunkt |
|---|---|---|---|
| II-68 | 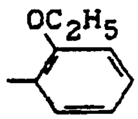 | 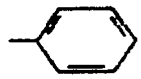 | 159-164° C |
| II-69 | 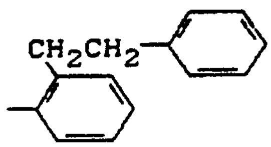 | 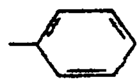 | 99° C |
| II-70 | 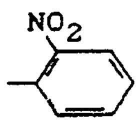 | 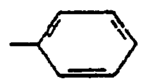 | 98° C |
| II-71 | 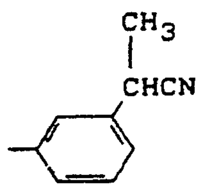 | 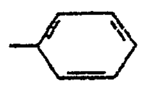 | 165-166° C |
| II-72 | 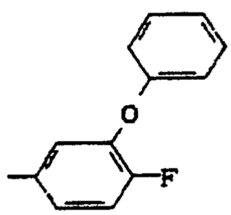 | 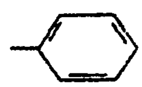 | 119° C |
| II-73 | 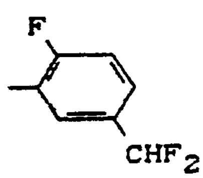 | 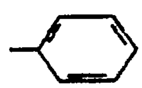 | 130° C |
| II-74 | 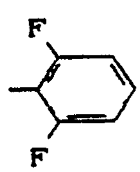 | 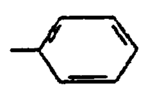 | 169-171° C |

Tabelle 4 (Fortsetzung)

| Bsp.Nr. | $R^1$ | Ar | Schmelzpunkt |
|---|---|---|---|
| II-75 | 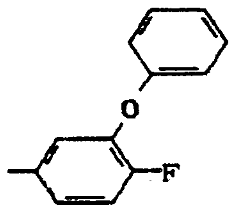 | 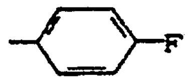 | 198° C |
| II-76 | 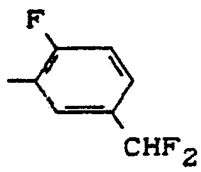 | 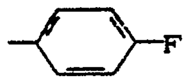 | 139° C |
| II-77 | 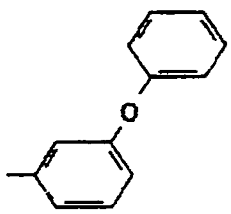 | 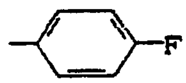 | 130° C |
| II-78 | 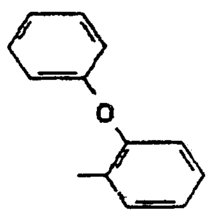 | 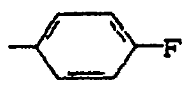 | 133-136° C |
| II-79 | 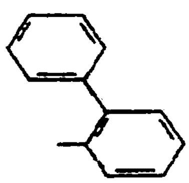 | 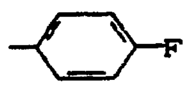 | 40° C |
| II-80 | 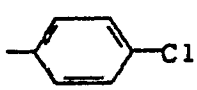 | 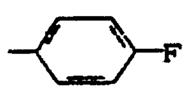 | 128° C |

Tabelle 4 (Fortsetzung)

| Bsp.Nr. | $R^1$ | Ar | Schmelzpunkt |
|---|---|---|---|
| II-81 | 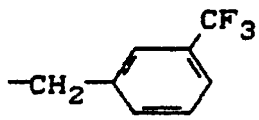 | 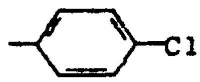 | 144-145° C |
| II-82 | 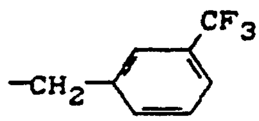 | 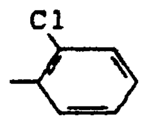 | 121-122° C |
| II-83 | 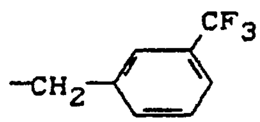 | 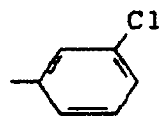 | 105-106° C |
| II-84 | $-C_3H_7$-n | 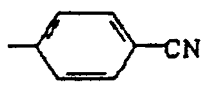 | 162-163° C |
| II-85 | $-C_3H_7$-n | 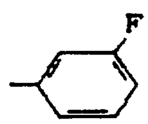 | 108-109° C |
| II-86 | $-C_4H_9$-n | 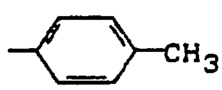 | |
| II-87 | $-C_4H_9$-n | 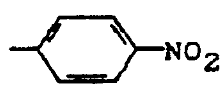 | 130-131° C |

Anwendungsbeispiele

Beispiel A

Pre-emergence-Test

**Lösungsmittel: 5 Gewichtsteile Aceton**

**Emulgator: 1 Gewichtsteil Alkylarylpolyglykolether**

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge

des Wirkstoffs pro Flächeneinheit. Nach drei Wochen wird der Schädigungsgrad der Pflanzen bonitiert in % Schädigung im Vergleich zur Entwicklung dar unbehandelten Kontrolle. Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 12, 13, 17, 18 und 37.

## Ansprüche

**1.** Pyrazolin-5-on-Derivate der allgemeinen Formel (I)

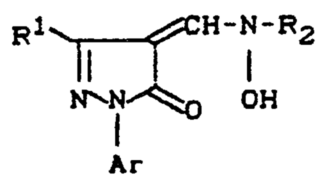

(I)

in welcher

$R^1$ für Alkyl, Cycloalkyl, Halogenalkyl, für jeweils gegebenenfalls substituiertes Alkenyl oder Alkinyl, wobei als Substituent gegebenenfalls substituiertes Phenyl ausgewählt ist, für Halogenalkenyl, Alkoxy, Alkoxyalkyl, Alkylthioalkyl, Alkylsulfonylalkyl, Alkylsulfinylalkyl, Alkoxycarbonylalkyl, jeweils gegebenenfalls substituiertes Aryl, Aralkyl, Aryloxyalkyl oder Arylthioalkyl, für einen gegebenenfalls substituierten Heterocyclus, für Heterocyclylalkyl oder für die Gruppierungen -NH-CO-$R^3$ oder -CO-O-$R^4$ steht, worin

$R^3$ und $R^4$ jeweils unabhängig voneinander für Alkyl oder Aryl stehen,

$R^2$ für Wasserstoff, Alkyl, Cycloalkyl, Halogenalkyl, Alkenyl, Alkinyl, Halogenalkenyl, Alkoxyalkyl, Alkylthioalkyl, Alkylsulfonylalkyl, Alkylsulfinylalkyl oder Alkoxycarbonylalkyl steht,

Ar für gegebenenfalls substituiertes Aryl oder für einen gegebenenfalls substituierten und/oder gegebenenfalls anellierten Heterocyclus steht,

ausgenommen die Verbindungen 1-(3-Chlorphenyl)-3-methylthiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on.

**2.** Pyrazolin-5-on-Derivate der Formel (I) gemäß Anspruch 1, in welcher

$R^1$ für geradkettiges oder verzweigtes Alkyl mit 1 bis 8 Kohlenstoffatomen, Cycloalkyl mit 3 bis 7 Kohlenstoffatomen, geradkettiges oder verzweigtes Halogenalkyl mit 1 bis 4 Kohlenstoffatomen und 1 bis 9 gleichen oder verschiedenen Halogenatomen, jeweils geradkettiges oder verzweigtes und jeweils gegebenenfalls einfach bis mehrfach substituiertes Alkenyl oder Alkinyl mit jeweils 2 bis 6 Kohlenstoffatomen steht, wobei als Substituenten unsubstituiertes oder einfach bis mehrfach, gleich oder verschieden substituiertes Phenyl infrage kommen und wobei als Phenylsubstituenten die unter Ar aufgeführten Arylsubstitu enten genannt seien; $R^1$ weiterhin für geradkettiges oder verzweigtes Halogenalkenyl mit 2 bis 4 Kohlenstoffatomen und 1 bis 7 gleichen oder verschiedenen Halogenatomen, geradkettiges oder verzweigtes Alkoxy mit 1 bis 4 Kohlenstoffatomen, geradkettiges oder verzweigtes Alkoxyalkyl mit jeweils 1 bis 8 Kohlenstoffatomen im Alkoxy- und Alkylteil, jeweils geradkettiges oder verzweigtes Alkylthioalkyl, Alkylsulfonylalkyl oder Alkylsulfinylalkyl mit jeweils 1 bis 4 Kohlenstoffatomen in den einzelnen Alkylteilen, geradkettiges oder verzweigtes Alkoxycarbonylalkyl mit jeweils 1 bis 4 Kohlenstoffatomen im Alkoxy- und Alkylteil, für jeweils gegebenenfalls einfach bis mehrfach, gleich oder verschieden substituiertes Phenyl oder Naphthyl, für jeweils gegebenenfalls einfach bis mehrfach, gleich oder verschieden im Arylteil substituiertes Phenylalkyl oder Naphthylalkyl mit jeweils 1 bis 8 Kohlenstoffatomen im geradkettigen oder verzweigten Alkylteil, für jeweils gegebenenfalls 1- bis 2-fach im Phenylteil substituiertes Phenoxyalkyl oder Phenylthioalkyl mit

jeweils 1 bis 4 Kohlenstoffatomen im geradkettigen oder verzweigten Alkylteil steht, wobei als Phenyl-und Naphthylsubstituenten jeweils die unter Ar aufgeführten Arylsubstituenten genannt seien; $R^1$ weiterhin für jeweils gegebenenfalls einfach bis mehrfach, gleich oder verschieden im Heterocyclylteil substituiertes Furyl, Thienyl, Pyrrolyl, Pyridinyl, Furylalkyl, Thienylalkyl oder Pyrrolylalkyl mit jeweils gegebenenfalls 1 bis 8 Kohlenstoffatomen im geradkettigen oder verzweigten Alkylteil steht, wobei als Heterocyclylsubstituenten die unter Ar aufgeführten Arylsubstituenten infrage kommen; $R^1$ weiterhin für die Gruppierungen -NHCOR$^3$ oder -COOR$^4$ steht, worin

$R^3$ und $R^4$ jeweils unabhängig voneinander für geradkettiges oder verzweigtes Alkyl mit 1 bis 4 Kohlenstoffatomen oder Phenyl stehen,

$R^2$ für Wasserstoff, geradkettiges oder verzweigtes Alkyl mit 1 bis 8 Kohlenstoffatomen, Cycloalkyl mit 3 bis 7 Kohlenstoffatomen, geradkettiges oder verzweigtes Halogenalkyl mit 1 bis 8 Kohlenstoffatomen und 1 bis 17 gleichen oder verschiedenen Halogenatomen, für jeweils geradkettiges oder verzweigtes Alkenyl oder Alkinyl mit jeweils 2 bis 6 Kohlenstoffatomen, geradkettiges oder verzweigtes Halogenalkenyl mit 2 bis 4 Kohlenstoffatomen und 1 bis 9 gleichen oder verschiedenen Halogenatomen, geradkettiges oder verzweigtes Alkoxyalkyl mit jeweils 1 bis 4 Kohlenstoffatomen im Alkoxy- und Alkylteil, für jeweils geradkettiges oder verzweigtes Alkylthioalkyl, Alkylsulfonylalkyl oder Alkylsulfinylalkyl mit jeweils 1 bis 4 Kohlenstoffatomen in den einzelnen Alkyteilen, für geradkettiges oder verzweigtes Alkoxycarbonylalkyl mit jeweils 1 bis 4 Kohlenstoffatomen im Alkoxy-und Alkylteil, steht und

Ar für jeweils gegebenenfalls einfach bis mehrfach, gleich oder verschieden substituiertes Phenyl oder Naphthyl steht, wobei als Substituenten infrage kommen: Halogen, Nitro, Cyano, Carboxy, jeweils geradkettiges oder verzweigtes Alkyl, Alkoxy oder Alkylthio mit jeweils 1 bis 6 Kohlenstoffatomen, geradkettiges oder verzweigtes Cyanalkyl mit 1 bis 6 Kohlenstoffatomen, geradkettiges oder verzweigtes Alkoxycarbonyl mit 1 bis 6 Kohlenstoffatomen im Alkoxyteil, geradkettiges oder verzweigtes Alkinyloxy mit 2 bis 6 Kohlenstoffatomen, jeweils geradkettiges oder verzweigtes Halogenalkyl, Halogenalkoxy oder Halogenalkylthio mit jeweils 1 bis 6 Kohlenstoffatomen und jeweils 1 bis 13 gleichen oder verschiedenen Halogenatomen, geradkettiges oder verzweigtes Alkylsulfonyl mit 1 bis 6 Kohlenstoffatomen, geradkettiges oder verzweigtes Halogenalkylsulfonyl mit 1 bis 6 Kohlenstoffatomen und 1 bis 13 gleichen oder verschiedenen Halogenatomen, Dialkylamino mit 1 bis 6 Kohlenstoffatomen in den jeweiligen geradkettigen oder verzweigten Alkylteilen, jeweils gegebenenfalls einfach bis mehrfach, gleich oder verschieden substituiertes Phenyl, Naphthyl, Phenoxy oder Naphthoxy, jeweils gegebenenfalls einfach bis mehrfach, gleich oder verschieden substituiertes Phenylalkyl oder Naphthylalkyl mit jeweils 1 bis 6 Kohlenstoffatomen im geradkettigen oder verzweigten Alkylteil; Ar weiterhin für jeweils gegebenenfalls einfach bis mehrfach, gleich oder verschieden substituiertes und jeweils gegebenenfalls durch einen Benzolring anelliertes Furyl, Thienyl, Pyrrolyl oder Pyridyl, wobei als Substituenten jeweils die oben genannten Arylsubstituenten infrage kommen,

ausgenommen die Verbindungen 1-(3-Chlorphenyl)-3-methylthiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on.

**3.** Pyrazolin-5-on-Derivate der Formel (I) gemäß Anspruch 1, in welcher

$R^1$ für geradkettiges oder verzweigtes Alkyl mit 1 bis 6 Kohlenstoffatomen, Cycloalkyl mit 3 bis 6 Kohlenstoffatomen, Halogenalkyl mit 1 oder 2 Kohlenstoffatomen und 1 bis 5 Fluor- und/oder Chloratomen, jeweils geradkettiges oder verzweigtes, jeweils gegebenenfalls 1-oder 2-fach substituiertes Alkenyl oder Alkinyl mit jeweils 2 bis 4 Kohlenstoffatomen steht, wobei als Substituenten unsubstituiertes oder 1- oder 2-fach gleich oder verschieden substituiertes Phenyl infrage kommen und wobei als Phenylsubstituenten die unter Ar aufgeführten Arylsubstituenten genannt seien; $R^1$ weiterhin für Halogenalkenyl mit 2 oder 3 Kohlenstoffatomen und 1 bis 5 Fluor- und/oder Chloratomen, Alkoxy mit 1 oder 2 Kohlenstoffatomen, geradkettiges oder verzweigtes Alkoxyalkyl mit jeweils 1 bis 6 Kohlenstoffatomen im Alkoxy- und Alkylteil, für Alkylthioalkyl, Alkylsulfonylalkyl oder Alkylsulfinylalkyl mit jeweils 1 oder 2 Kohlenstoffatomen in den einzelnen Alkylteilen, Alkoxycarbonylalkyl mit jeweils 1 oder 2 Kohlenstoffatomen im Alkoxy- und Alkylteil, für jeweils gegebenenfalls 1- bis 5-fach, gleich oder verschieden substituiertes Phenyl oder Naphthyl, jeweils gegebenenfalls 1- bis 5-fach, gleich oder verschieden im Arylteil substituiertes Phenylalkyl oder Naphthylalkyl mit jeweils 1 bis 6 Kohlenstoffatomen im geradkettigen oder verzweigten Alkylteil steht, wobei als Phenyl- und Naphthylsubstituenten jeweils die unter Ar aufgeführten Arylsubstituenten genannt seien; $R^1$ weiterhin für Phenoxyalkyl oder Phenylthioalkyl mit jeweils 1 oder 2 Kohlenstoffatomen im Alkylteil, für jeweils 1- bis 3-fach gleich oder verschieden substituiertes Furyl, Thienyl, Pyrrolyl oder Pyridinyl, für jeweils 1- bis 3-fach gleich oder verschieden im Heterocyclylteil substituiertes Furylalkyl, Thienylalkyl oder Pyrrolylalkyl mit jeweils 1 bis 6 Kohlenstoffatomen im geradkettigen oder verzweigten Alkylteil steht, wobei als Heterocyclylsubstituenten jeweils die unter Ar aufgeführten Arylsubstituenten infrage kommen; weiterhin $R^1$ für die Gruppierungen $-NHCOR^3$ oder $-COOR^4$ steht, worin

$R^3$ und $R^4$ jeweils unabhängig voneinander für Methyl, Ethyl oder Phenyl steht,

$R^2$ für Wasserstoff, geradkettiges oder verzweigtes Alkyl mit 1 bis 6 Kohlenstoffatomen, Cycloalkyl mit 3 bis 6 Kohlenstoffatomen, geradkettiges oder verzweigtes Halogenalkyl mit 1 bis 6 Kohlenstoffatomen und 1 bis 13 Fluor- und/oder Chloratomen, jeweils geradkettiges oder verzweigtes Alkenyl oder Alkinyl mit 2 bis 4 Kohlenstoffatomen, Halogenalkenyl mit 2 oder 3 Kohlenstoffatomen und 1 bis 5 Fluor- und/oder Chloratomen, Alkoxyalkyl mit jeweils 1 oder 2 Kohlenstoffatomen im Alkoxy- und Alkylteil, für Alkylthioalkyl, Alkylsulfonylalkyl oder Alkylsulfinylalkyl mit jeweils 1 oder 2 Kohlenstoffatomen in den einzelnen Alkylteilen, Alkoxycarbonylalkyl mit jeweils 1 oder 2 Kohlenstoffatomen im Alkoxy-und Alkylteil steht und

Ar für jeweils gegebenenfalls 1- bis 5-fach gleich oder verschieden substituiertes Phenyl oder Napththyl steht, wobei als Substituenten infrage kommen: Fluor, Chlor, Brom, Nitro, Cyano, Carboxy, jeweils geradkettiges oder verzweigtes Alkyl, Alkoxy oder Alkylthio mit jeweils 1 bis 4 Kohlenstoffatomen, geradkettiges oder verzweigtes Cyanalkyl mit 1 bis 4 Kohlenstoffatomen, geradkettiges oder verzweigtes Alkoxycarbonyl mit 1 bis 4 Kohlenstoffatomen im Alkoxyteil, geradkettiges oder verzweigtes Alkinyloxy mit 2 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und jeweils 1 bis 9 gleichen oder verschiedenen Halogenatomen, geradkettiges oder verzweigtes Alkylsulfonyl mit 1 bis 4 Kohlenstoffatomen, geradkettiges oder verzweigtes Halogenalkylsulfonyl mit 1 bis 4 Kohlenstoffatomen und 1 bis 9 gleichen oder verschiedenen Halogenatomen, Dialkylamino mit 1 bis 4 Kohlenstoffatomen in den jeweiligen geradkettigen oder verzweigten Alkylteilen, jeweils gegebenenfalls 1- oder 2-fach, gleich oder verschieden substituiertes Phenyl, Naphthyl, Phenoxy oder Naphthoxy, jeweils gegebenenfalls 1-oder 2-fach, gleich oder verschieden substituiertes Phenylalkyl oder Naphthylalkyl mit jeweils 1 bis 4 Kohlenstoffato men im geradkettigen oder verzweigten Alkylteil; Ar weiterhin für jeweils gegebenenfalls 1- oder 2-fach gleich oder verschieden substituiertes Furyl, Thienyl, Pyrrolyl oder Pyridyl, wobei als Substituenten jeweils die oben genannten Arylsubstituenten infrage kommen,

ausgenommen die Verbindungen 1-(3-Chlorphenyl)-3-methylthiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on.

**4.** Pyrazolin-5-on-Derivate der Formel (I) gemäß Anspruch 1, in welcher

$R^1$ für Methyl, Ethyl, n-Propyl, i-Propyl, n-Butyl, i-Butyl, s-Butyl, t-Butyl, 2-Phenylvinyl, 2-(3-Trifluormethylphenyl)-vinyl, Methoxymethyl, Methoxyethyl, Ethoxymethyl, Ethoxyethyl, für gegebenenfalls 1- bis 3-fach, gleich oder verschieden substituiertes Phenyl oder Naphthyl, jeweils gegebenenfalls 1- bis 3-fach im Phenylteil substituiertes Phenylmethyl oder Phenylethyl steht, wobei als Phenyl-und Naphthylsubstituenten jeweils die unter Ar aufgeführten Arylsubstituenten genannt seien; $R^1$ weiterhin für Furyl, Thienyl, Pyridyl, Furylmethyl, Thienylmethyl oder Pyridylmethyl steht,

$R^2$ für Wasserstoff, Methyl, Ethyl, n-Propyl, i-Propyl, n-Butyl, i-Butyl, s-Butyl, t-Butyl, Trifluormethyl, Trichlormethyl, 2,2,2-Trifluorethyl, 2,2,2-Trichlorethyl, Vinyl, Allyl, 1-Propenyl, Ethinyl, 1-Propinyl, 2-Propinyl, 2-Fluorvinyl, Methoxymethyl oder Methylthiomethyl steht und

Ar für jeweils gegebenenfalls 1- bis 3-fach gleich oder verschieden substituiertes Phenyl steht, wobei als Substituenten infrage kommen: Fluor, Chlor, Brom, Nitro, Cyano, Carboxy, Methyl,

Ethyl, Methoxy, Ethoxy, Methylthio, Ethylthio, Cyanmethyl, 1-Cyanethyl, 2-Cyanethyl, Methoxycarbonyl, Ethinyloxy, Fluormethyl, Difluormethyl, Trifluormethyl, Chlormethyl, Dichlormethyl, Trichlormethyl, 2-Fluorethyl, 2,2-Difluorethyl, 2,2,2-Trifluorethyl, 2-Chlorethyl, 2,2-Dichlorethyl, 2,2,2-Trichlorethyl, Fluormethoxy, Difluormethoxy, Trifluormethoxy, Chlormethoxy, Dichlormethoxy, Trichlormethoxy, Fluormethylthio, Difluormethylthio, Trifluormethylthio, Chlormethylthio, Dichlormethylthio, Trichlormethylthio, Methylsulfonyl, Ethylsulfonyl, Fluormethylsulfonyl, Difluormethylsulfonyl, Trifluormethylsulfonyl, Chlormethylsulfonyl, Dichlormethylsulfonyl, Trichlormethylsulfonyl, Dimethylamino, Diethylamino, Phenyl, Phenoxy, Phenylmethyl oder Phenylethyl steht.

**5.** Verfahren zur Herstellung von Pyrazolin-5-on-Derivaten der Formel (I)

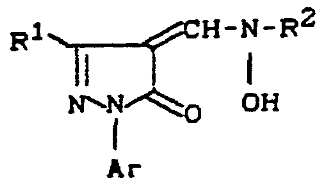

(I)

in welcher

$R^1$ für Alkyl, Cycloalkyl, Halogenalkyl, für jeweils gegebenenfalls substituiertes Alkenyl oder Alkinyl, wobei als Substituent gegebenenfalls substituiertes Phenyl ausgewählt ist, für Halogenalkenyl, Alkoxy, Alkoxyalkyl, Alkylthioalkyl, Alkylsulfonylalkyl, Alkylsulfinylalkyl, Alkoxycarbonylalkyl, für jeweils gegebenenfalls substituiertes Aryl, Aralkyl, Aryloxyalkyl oder Arylthioalkyl, für gegebenenfalls substituiertes Heterocyclyl, gegebenenfalls substituiertes Heterocyclylalkyl oder für die Gruppierungen -NH-CO-$R^3$ oder -CO-O-$R^4$ steht, worin

$R^3$ und $R^4$ jeweils unabhängig voneinander für Alkyl

$R^2$ für Wasserstoff, Alkyl, Cycloalkyl, Halogenalkyl Alkenyl, Alkinyl, Halogenalkenyl, Alkoxyalkyl, Alkylthioalkyl, Alkylsulfonylalkyl, Alkylsulfinylalkyl, Alkoxycarbonylalkyl steht und

Ar für gegebenenfalls substituiertes Aryl oder für gegebenenfalls substituiertes und/oder gegebenenfalls anelliertes Heterocyclyl steht,

ausgenommen die Verbindungen 1-(3-Chlorphenyl)-3-methylthiomethyl-4-(N-hydroxyaminomethyliden)-pyrazolin-5-on, 1-(4-Chlorphenyl)-3-methylthiomethyl-4-(N-hydroxyaminomethyliden)-pyrazolin-5-on, 1-(4-Trifluormethylphenyl)-3-methylthiomethyl-4-(N-hydroxyaminomethyliden)-pyrazolin-5-on und 1-(4-Nitrophenyl)-3-methylthiomethyl-4-(N-hydroxyaminomethyliden)-pyrazolin-5-on,

dadurch gekennzeichnet, daß man Dimethylaminomethylidenpyrazolin-5-on-Derivate der Formel (II)

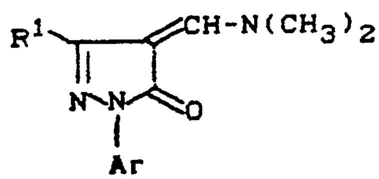

(II)

in welcher

$R^1$ und Ar die oben angegebenen Bedeutungen haben,

mit Hydroxylamin-hydrochlorid-Derivaten der Formel (III)

$$R^2\text{-NHOH} \quad x \quad HX \qquad (III)$$

in welcher

$R^2$ die oben angegebene Bedeutung hat und

HX für das Äquivalent einer Mineralsäure, wie z.B. Chlorwasserstoffsäure oder einer Carbonsäure, wie z.B. Oxalsäure steht,

gegebenenfalls in Gegenwart eines Verdünnungsmittels umsetzt.

6. Herbizide Mittel, gekennzeichnet durch einen Gehalt an mindestens einem Pyrazolin-5-on-Derivat der Formel (I) gemäß Anspruch 1 oder 5.

7. Verfahren zur Bekämpfung von unerwünschten Pflanzen, dadurch gekennzeichnet, daß man Pyrazolin-5-on-Derivate der Formel (I) gemäß Anspruch 1 oder 5 auf die Pflanzen und/oder ihren Lebensraum einwirken läßt.

8. Verwendung von Pyrazolin-5-on-Derivaten der Formel (I) gemäß Anspruch 1 oder 5 zur Bekämpfung von unerwünschten Pflanzen.

9. Verfahren zur Herstellung von herbiziden Mitteln, dadurch gekennzeichnet, daß man Pyrazolin-5-on-Derivate der Formel (I) gemäß Anspruch 1 oder 5 mit Streckmitteln und/oder oberflächenaktiven Substanzen vermischt.